# EUROPEAN PATENT SPECIFICATION

(11) **EP 2 908 856 B1**
(45) Date of publication and mention of the grant of the patent: **20.02.2019**
(21) Application number: 13779191.9
(22) Date of filing: 15.10.2013
(51) Int. Cl.: A61K 39/00, A61K 39/09, C07K 14/315

(54) **IMMUNOGENIC COMPOSITION**
IMMUNOGENE ZUSAMMENSETZUNG
COMPOSITION IMMUNOGÈNE

(30) Priority: 17.10.2012 GB 201218660; 17.10.2012 US 201261714942 P; 17.10.2012 US 201261714956 P; 14.03.2013 US 201313826696; 14.03.2013 US 201313826932; 14.03.2013 US 201313827203
(43) Date of publication of application: 26.08.2015
(73) Proprietor: GlaxoSmithKline Biologicals S.A., 1330 Rixensart (BE)
(72) Inventor: VERLANT, Vincent, B-1300 Wavre (BE)
(74) Representative: Baker, Suzanne J.
(86) International application number: PCT/EP2013/071477
(87) International publication number: WO 2014/060385

(56) References cited:
- WO-A1-2009/000826
- WO-A1-2010/071986
- WO-A1-2011/075823
- WO-A1-2012/075428
- WO-A1-2012/156391
- WO-A2-00/56360
- "A phase II, randomized, controlled, observer-blind study to assess the safety, reactogenicity and immunogenicity of GlaxoSmithKline (GSK) Biologicals' Streptococcus pneumoniae protein containing vaccine formulations when administered according to a 0-2-6 month schedule, in healthy children aged 12-2", International Clinical Trials Registry Platform, 19 March 2011 (2011-03-19), XP055097477, Retrieved from the Internet: URL:http://apps.who.int/trialsearch/trial. aspx?trialid=EUCTR2009-012701-19-CZ [retrieved on 2014-01-21]
- William Hausdorff: "NEED FOR AND DEVELOPMENT OF PROTEIN-BASED PNEUMOCOCCAL VACCINE", Burden of meningitis, advances from research, prevention of pneumococcal disease - cpnference 2011, 8 November 2011 (2011-11-08), XP055365053, Retrieved from the Internet: URL:http://www.meningitis.org/conference/d ay-one-abstracts2011#William_Hausdorff_Bio [retrieved on 2017-04-18]
- William Hausdorff ET AL: "Need for and development of a protein-based pneumococcal vaccine a protein-based pneumococcal vaccine", , 18 November 2011 (2011-11-18), XP055118369, Retrieved from the Internet: URL:http://www.meningitis.org/assets/x/539 80 [retrieved on 2014-05-16]
- Michael D Decker ET AL: "Combination Vaccines" In: "Vaccines, fourth edition", 1 January 2003 (2003-01-01), Elsevier, XP055354096, ISBN: 978-0-7216-9688-1 pages 825-861,
- R. Prymula ET AL: "Safety and immunogenicity of an investigational vaccine containing two common pneumococcal proteins given to czech toddlers", 8TH INTERNATIONAL SYMPOSIUM ON PNEUMOCOCCI AND PNEUMOCOCCAL DISEASES, IGUACU FALLS, BRAZILE, MARCH 11-15, 2012, no. Poster No 274, 11 March 2012 (2012-03-11), XP055111341,
- Leroux-Roels G ET AL: "Safety/reactogenicity results of a phase I clinical trial of an investigational pneumococcal protein-based vaccine in adults", 8th International Symposium on Pneumococci and Pneumococcal Diseases, Iguacu Falls, Brazile, March 11-15, 2012, no. Poster No 231, 11 March 2012 (2012-03-11), - 15 March 2012 (2012-03-15), page 543, XP055111237,

### SEQUENCE LISTING

<110> GlaxoSmithKline Biologicals S.A
<120> IMMUNOGENIC COMPOSITION
<130> VB65266
<160> 4
<170> FastSEQ for Windows Version 4.0
<210> 1
   <211> 818
   <212> PRT
   <213> Streptococcus pneumoniae
<400> 1
   Ser Tyr Glu Leu Gly Arg His Gln Ala Gly Gln Val Lys Lys Glu Ser
    1               5                  10                  15
   Asn Arg Val Ser Tyr Ile Asp Gly Asp Gln Ala Gly Gln Lys Ala Glu
               20                  25                  30
   Asn Leu Thr Pro Asp Glu Val Ser Lys Arg Glu Gly Ile Asn Ala Glu
           35                  40                  45
   Gln Ile Val Ile Lys Ile Thr Asp Gln Gly Tyr Val Thr Ser His Gly
       50                  55                  60
   Asp His Tyr His Tyr Tyr Asn Gly Lys Val Pro Tyr Asp Ala Ile Ile
   65                  70                  75                  80
   Ser Glu Glu Leu Leu Met Lys Asp Pro Asn Tyr Gln Leu Lys Asp Ser
                   85                  90                  95
   Asp Ile Val Asn Glu Ile Lys Gly Gly Tyr Val Ile Lys Val Asp Gly
               100                 105                 110
   Lys Tyr Tyr Val Tyr Leu Lys Asp Ala Ala His Ala Asp Asn Ile Arg
           115                 120                 125
   Thr Lys Glu Glu Ile Lys Arg Gln Lys Gln Glu His Ser His Asn His
       130                 135                 140
   Gly Gly Gly Ser Asn Asp Gln Ala Val Val Ala Ala Arg Ala Gln Gly
   145                 150                 155                 160
   Arg Tyr Thr Thr Asp Asp Gly Tyr Ile Phe Asn Ala Ser Asp Ile Ile
                   165                 170                 175
   Glu Asp Thr Gly Asp Ala Tyr Ile Val Pro His Gly Asp His Tyr His
               180                 185                 190
   Tyr Ile Pro Lys Asn Glu Leu Ser Ala Ser Glu Leu Ala Ala Ala Glu
           195                 200                 205
   Ala Tyr Trp Asn Gly Lys Gln Gly Ser Arg Pro Ser Ser Ser Ser Ser
       210                 215                 220
   Tyr Asn Ala Asn Pro Ala Gln Pro Arg Leu Ser Glu Asn His Asn Leu
   225                 230                 235                 240
   Thr Val Thr Pro Thr Tyr His Gln Asn Gln Gly Glu Asn Ile Ser Ser
                   245                 250                 255
   Leu Leu Arg Glu Leu Tyr Ala Lys Pro Leu Ser Glu Arg His Val Glu
               260                 265                 270
   Ser Asp Gly Leu Ile Phe Asp Pro Ala Gln Ile Thr Ser Arg Thr Ala
           275                 280                 285
   Arg Gly Val Ala Val Pro His Gly Asn His Tyr His Phe Ile Pro Tyr
       290                 295                 300
   Glu Gln Met Ser Glu Leu Glu Lys Arg Ile Ala Arg Ile Ile Pro Leu
   305                 310                 315                 320
   Arg Tyr Arg Ser Asn His Trp Val Pro Asp Ser Arg Pro Glu Gln Pro
                   325                 330                 335
   Ser Pro Gln Ser Thr Pro Glu Pro Ser Pro Ser Pro Gln Pro Ala Pro
                   340                 345                 350
   Asn Pro Gln Pro Ala Pro Ser Asn Pro Ile Asp Glu Lys Leu Val Lys
           355                 360                 365
   Glu Ala Val Arg Lys Val Gly Asp Gly Tyr Val Phe Glu Glu Asn Gly
       370                 375                 380
   Val Ser Arg Tyr Ile Pro Ala Lys Asp Leu Ser Ala Glu Thr Ala Ala
   385                 390                 395                 400
   Gly Ile Asp Ser Lys Leu Ala Lys Gln Glu Ser Leu Ser His Lys Leu
                   405                 410                 415
   Gly Ala Lys Lys Thr Asp Leu Pro Ser Ser Asp Arg Glu Phe Tyr Asn
               420                 425                 430
   Lys Ala Tyr Asp Leu Leu Ala Arg Ile His Gln Asp Leu Leu Asp Asn
           435                 440                 445
   Lys Gly Arg Gln Val Asp Phe Glu Ala Leu Asp Asn Leu Leu Glu Arg
       450                 455                 460
   Leu Lys Asp Val Pro Ser Asp Lys Val Lys Leu Val Asp Asp Ile Leu
   465                 470                 475                 480
   Ala Phe Leu Ala Pro Ile Arg His Pro Glu Arg Leu Gly Lys Pro Asn
                   485                 490                 495
   Ala Gln Ile Thr Tyr Thr Asp Asp Glu Ile Gln Val Ala Lys Leu Ala
               500                 505                 510
   Gly Lys Tyr Thr Thr Glu Asp Gly Tyr Ile Phe Asp Pro Arg Asp Ile
           515                 520                 525
   Thr Ser Asp Glu Gly Asp Ala Tyr Val Thr Pro His Met Thr His Ser
       530                 535                 540
   His Trp Ile Lys Lys Asp Ser Leu Ser Glu Ala Glu Arg Ala Ala Ala
   545                 550                 555                 560
   Gln Ala Tyr Ala Lys Glu Lys Gly Leu Thr Pro Pro Ser Thr Asp His
                   565                 570                 575
   Gln Asp Ser Gly Asn Thr Glu Ala Lys Gly Ala Glu Ala Ile Tyr Asn
               580                 585                 590
   Arg Val Lys Ala Ala Lys Lys Val Pro Leu Asp Arg Met Pro Tyr Asn
           595                 600                 605
   Leu Gln Tyr Thr Val Glu Val Lys Asn Gly Ser Leu Ile Ile Pro His
       610                 615                 620
   Tyr Asp His Tyr His Asn Ile Lys Phe Glu Trp Phe Asp Glu Gly Leu
   625                 630                 635                 640
   Tyr Glu Ala Pro Lys Gly Tyr Thr Leu Glu Asp Leu Leu Ala Thr Val
                   645                 650                 655
   Lys Tyr Tyr Val Glu His Pro Asn Glu Arg Pro His Ser Asp Asn Gly
               660                 665                 670
   Phe Gly Asn Ala Ser Asp His Val Arg Lys Asn Lys Val Asp Gln Asp
           675                 680                 685
   Ser Lys Pro Asp Glu Asp Lys Glu His Asp Glu Val Ser Glu Pro Thr
       690                 695                 700
   His Pro Glu Ser Asp Glu Lys Glu Asn His Ala Gly Leu Asn Pro Ser
   705                 710                 715                 720
   Ala Asp Asn Leu Tyr Lys Pro Ser Thr Asp Thr Glu Glu Thr Glu Glu
                   725                 730                 735
   Glu Ala Glu Asp Thr Thr Asp Glu Ala Glu Ile Pro Gln Val Glu Asn
               740                 745                 750
   Ser Val Ile Asn Ala Lys Ile Ala Asp Ala Glu Ala Leu Leu Glu Lys
           755                 760                 765
   Val Thr Asp Pro Ser Ile Arg Gln Asn Ala Met Glu Thr Leu Thr Gly
       770                 775                 780
   Leu Lys Ser Ser Leu Leu Leu Gly Thr Lys Asp Asn Asn Thr Ile Ser
   785                 790                 795                 800
   Ala Glu Val Asp Ser Leu Leu Ala Leu Leu Lys Glu Ser Gln Pro Ala
                   805                 810                 815
   Pro Ile
<210> 2
   <211> 6
   <212> PRT
   <213> Artificial Sequence
<220>
   <223> S. pneumoniae polyhistidine triad protein (Pht) family histidine triad motif
<221> VARIANT
   <222> (2)...(3)
   <223> X is any amino acid other than his
<221> VARIANT
   <222> (5)...(5)
   <223> X is any amino acid other than his
<400> 2
   His Xaa Xaa His Xaa His
    1               5
<210> 3
   <211> 4
   <212> PRT
   <213> Unknown
<220>
   <223> S. pneumoniae protein having a Type II Signal sequence Motif
<221> VARIANT
   <222> (2)...(3)
   <223> X is any amino acid
<400> 3
   Leu Xaa Xaa Cys
    1
<210> 4
   <211> 5
   <212> PRT
   <213> unknown
<220>
   <223> S. pneumoniae surface protein Cell Wall Anchored motif
<221> VARIANT
   <222> (3)...(3)
   <223> X is any amino acid
<400> 4
   Leu Pro Xaa Thr Gly
    1               5

## Description

### TECHNICAL FIELD

The present invention relates to improved immunogenic compositions and vaccines, and their use in medicine. In particular the invention relates to immunogenic compositions comprising 26-45µg of detoxified pneumolysin and PhtD per human dose for use in the treatment or prevention of disease caused by *Streptococcus pneumoniae.*

### BACKGROUND

*Streptococcus pneumoniae* (*S. pneumoniae*), also known as the pneumococcus, is a Gram-positive bacterium. *S. pneumoniae* is a major public health problem all over the world and is responsible for considerable morbidity and mortality, especially among infants, the elderly and immunocompromised persons. *S. pneumoniae* causes a wide range of important human pathologies including community-acquired pneumonia, acute sinusitis, otitis media, meningitis, bacteremia, septicemia, osteomyelitis, septic arthritis, endocarditis, peritonitis, pericarditis, cellulitis, and brain abscess. *S. pneumoniae* is estimated to be the causal agent in 3,000 cases of meningitis, 50, 000 cases of bacteremia, 500,000 cases of pneumonia, and 7,000,000 cases of otitis media anually in the United States alone (Reichler, M. R. et al., 1992, J. Infect. Dis. 166: 1346; Stool, S. E. and Field, M. J., 1989 Pediatr. Infect. Dis J. 8: S11). Mortality rates due to pneumococcal disease are especially high in children younger than 5 years of age from both developed and developing countries. The elderly, the immunocompromised and patients with other underlying conditions (diabetes, asthma) are also particularly susceptible to disease.

The major clinical syndromes caused by *S. pneumoniae* are widely recognized and discussed in all standard medical textbooks (Fedson D S, Muscher D M. In: Plotkin S A, Orenstein W A, editors. Vaccines. 4rth edition. Philadelphia WB Saunders Co, 2004a: 529-588). For instance, Invasive pneumococcal disease (IPD) is defined as any infection in which *S. pneumoniae* is isolated from the blood or another normally sterile site (Musher D M. Streptococcus pneumoniae. In Mandell G L, Bennett J E, Dolin R (eds). Principles and Practice of Infectious diseases (5th ed). New York, Churchill Livingstone, 2001, p 2128-2147).

Chronic obstructive pulmonary disease (COPD) is a chronic inflammatory disease of the lungs and a major cause of morbidity and mortality worldwide. Approximately one in 20 deaths in 2005 in the US had COPD as the underlying cause. (Drugs and Aging 26:985-999 (2009)). It is projected that in 2020 COPD will rise to the fifth leading cause of disability adjusted life years, chronic invalidating diseases, and to the third most important cause of mortality (Lancet 349:1498-1504 (1997)).

Although the advent of antimicrobial drugs has reduced the overall mortality from pneumococcal disease, the emergence of antibiotic resistant strains of *S. pneumoniae* is a serious and rapidly increasing problem. It is therefore important for effective vaccines against S. *pneumoniae* to be developed. Effective pneumococcal vaccines could have a major impact on the morbidity and mortality associated with *S. pneumoniae* disease.

The present invention relates to immunogenic compositions comprising 26-45µg of detoxified pneumolysin per human dose and 26-45 µg of PhtD (Poly Histidine Triad protein D) per human dose for use in the treatment or prevention of disease caused by *Streptococcus pneumoniae* infection. The inventors have surprisingly found that pneumolysin and PhtD can enhance antibody-mediated opsonic activty against a *Streptococcus pneumoniae* strain when the pneumolysin and/or PhtD are co-administered in combination with a *Streptococcus pneumoniae* saccharide derived from that strain, i.e that co-administering the *Streptococcus pneumoniae* saccharide with a higher level of *Streptococcus pneumoniae* protein leads to an increase in the antibody-mediated opsonic activity raised against the strain from which the saccharide is derived. The inventors have also found that this effect is higher when a composition comprising 26-45µg of pneumolysin and/or PhtD is administered than when a composition comprising lower doses of pneumolysin and/or PhtD is administered. Furthermore the inventors have found that this higher dose of pneumolysin and/or PhtD is not associated with statistically significantly increased reactivity which could be expected when higher doses of antigens are administered.

The following documents disclose immunogenic compositions comprising pneumolysin and/or PhtD;

"A phase II randomized, controlled, observer-blind study to assess the safety, reactogenicity and immunogenicity of a GSK Biologicals' Streptococcus pneumoniae protein containing vaccine formulations when administered according to a 0-2-6 month schedule, in healthy children aged 12-2" (International Clinical Trials Registry Platform, 19 March 2011, XP055097477), Prymula et a/2012 (8th International Symposium on Pneumococci and Pneumococcal Diseases, Iguacu Falls, Brazile, 11th March 2012, Poster No: 274, XP055111341), Leroux-Roels G et a/2012 (8th International Symposium on Pneumococci and Pneumococcal Diseases, Iguacu Falls, Brazile, 15th March 2012, Poster No: 231, XP055111237) and William Hausdorff et al "Need for and development of protein-based pneumococcal vaccine", Burden of meningitis, advances from research, prevention of pneumococcal disease - conference 2011, 8th November 2011 (XP055365053) and 18th November 2011 (XP055118369)

Although a positive interaction between Pneumococcal saccharides and proteins has been demonstrated previously (for example WO02/22167) WO02/22167 refers to results obtained by simultaneously stimulating the cell mediated branch of the immune system and the humoral branch of the immune system. The effect in WO02/22167 would not enhance the antibody-mediated opsonic activity against a strain of *Streptococcus pneumoniae,* and would not be reflected in enhanced antibody-mediated opsonic activity measured using an opsonophagocytosis (OPA) assay. This is because the opsonic activity measured using an OPA assay (such as that described in example 2) is measured in the absence of components of the cell mediated immune system.

### BRIEF SUMMARY

The inventors have surprisingly found that an immunogenic composition comprising 26-45µg of pneumolysin and/or PhtD (Poly Histidine Triad Protein D) is not significantly more reactogenic than a composition with a lower dose of pneumolysin and/or Phtd and that the higher (26-45µg) dose can enhance antibody mediated opsonic activity against a *Streptococcus pneumoniae* strain when the pneumolysin and/or PhtD are co-administered in combination with a *Streptococcus pneumoniae* saccharide from that strain.

Accordingly in a first aspect there is provided an immunogenic composition comprising 26µg-45µg (for example 26µg-40µg, 28µg-35µg or around 30µg) of PhtD per human dose and 26µg-45µg (for example 26µg-40µg, 28µg-35µg or around 30µg) of detoxified pneumolysin (dPly) per human dose, for use in the treatment or prevention of disease caused by *Streptococcus pneumoniae* infection wherein the immunogenic composition further comprises *Streptococcus pneumoniae* capsular saccharide conjugates comprising a conjugated serotype 4 saccharide, a conjugated serotype 6B saccharide, a conjugated serot ype 9V saccharide, a conjugated serotype 14 saccharide, a conjugated serotype 18C saccharide, a conjugated serotype 19F saccharide and a conjugated serotype 23F saccharide.

In a second aspect there is provided a vaccine comprising an immunogenic composition comprising 26µg-45µg (for example 26µg-40µg, 28µg-35µg or around 30µg) of PhtD per human dose and 26µg-45µg (for example 26µg-40µg, 28µg-35µg or around 30µg) of detoxified pneumolysin (dPly) per human dose, for use in the treatment or prevention of disease caused by *Streptococcus pneumoniae* infection wherein the immunogenic composition further comprises *Streptococcus pneumoniae* capsular saccharide conjugates comprising a conjugated serotype 4 saccharide, a conjugated serotype 6B saccharide, a conjugated serotype 9V saccharide, a conjugated serotype 14 saccharide, a conjugated serotype 18C saccharide, a conjugated serotype 19F saccharide and a conjugated serotype 23F saccharide and a pharmaceutically acceptable excipient.

### BRIEF DESCRIPTION OF THE DRAWINGS

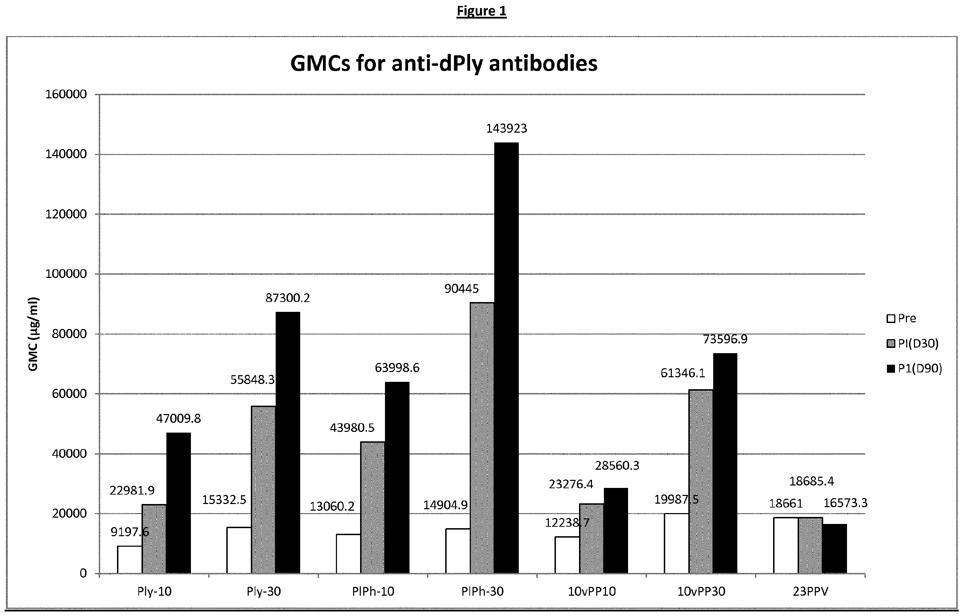

**Figure 1****:** Bar chart showing anti-dPly antibody titres, according to an ELISA assay, in human adults after immunisation with 10µg detoxified Pneumolysin (Ply-10), 30µg detoxified pneumolysin (Ply-30), 10µg of detoxified pneumolysin and 10µg of PhtD (PIPh-10), 30µg of detoxified pneumolysin and 30µg of PhtD (PIPh-3), a vaccine comprising 10 pneumococcal conjugates combined with 10µg detoxified pneumolysin and 10µg of PhtD (10vPP10), a vaccine comprising 10 pneumococcal conjugates combined with 30µg detoxified pneumolysin and 30µg of PhtD (10vPP30), and a vaccine comprising 23 unconjugated pneumococcal saccharides. The white bars represent immunogenicity prior to immunisation, the grey bars represent immunogenicity after 1 dose of vaccine and the black bars represent immunogenicity after 2 doses of vaccine.
**Figure 2****:** Similar bar chart to Figure 2 but depicting the anti-PhtD titres rather than anti-Ply titres.

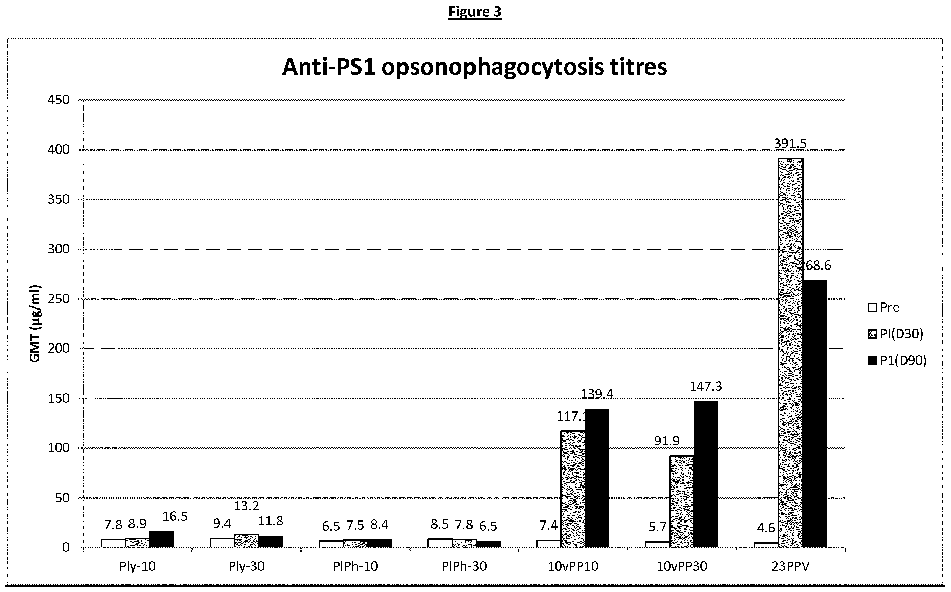

**Figure 3****:** Bar chart showing anti-PS1 opsonic antibody titres, according to an opsonophagocytosis assay, in human adults after immunisation with 10µg detoxified Pneumolysin (Ply-10), 30µg detoxified pneumolysin (Ply-30), 10µg of detoxified pneumolysin and 10µg of PhtD (PIPh-10), 30µg of detoxified pneumolysin and 30µg of PhtD (PIPh-3), a vaccine comprising 10 pneumococcal conjugates combined with 10µg detoxified pneumolysin and 10µg of PhtD (10vPP10), a vaccine comprising 10 pneumococcal conjugates combined with 30µg detoxified pneumolysin and 30µg of PhtD (10vPP30), and a vaccine comprising 23 unconjugated pneumococcal saccharides. The white bars represent immunogenicity prior to immunisation, the grey bars represent immunogenicity after 1 dose of vaccine and the black bars represent immunogenicity after 2 doses of vaccine.
**Figure 4****:** Similar bar chart to Figure 3 but depicting the anti-PS4 titres rather than the anti-PS1 titers.
**Figure 5****:** Similar bar chart to Figure 3 but depicting the anti-PS5 titres rather than the anti-PS1 titers.
**Figure 6****:** Similar bar chart to Figure 3 but depicting the anti-PS6B titres rather than the anti-PS1 titers.
**Figure 7****:** Similar bar chart to Figure 3 but depicting the anti-PS7F titres rather than the anti-PS1 titers.
**Figure 8****:** Similar bar chart to Figure 3 but depicting the anti-PS9V titres rather than the anti-PS1 titers.
**Figure 9****:** Similar bar chart to Figure 3 but depicting the anti-PS14 titres rather than the anti-PS1 titers.
**Figure 10****:** Similar bar chart to Figure 3 but depicting the anti-PS18C titres rather than the anti-PS1 titers.
**Figure 11****:** Similar bar chart to Figure 3 but depicting the anti-PS19F titres rather than the anti-PS1 titers.
**Figure 12****:** Similar bar chart to Figure 3 but depicting the anti-PS23F titres rather than the anti-PS1 titers.
**Figure 13****:** Sequence listing
**Figure 14****:** Graph comparing the immunogenicity of a composition comprising 12 saccharide conjugates, PhtD, dPly and PE-PilA (12V + prot) with a composition comprising 12 saccharides conjugates (12V) and a composition comprising 10 saccharide conjugates (10V) as measured after injection of mice using an anti-saccharide ELISA assay. GMC= geometric mean concentration . IC = confidence intervals.
**Figure 15****:** Graph comparing the immunogenicity of a composition comprising 12 saccharide conjugates, PhtD, dPly and PE-PilA (12V + prot) with a composition comprising 12 saccharides conjugates (12V) and a composition comprising 10 saccharide conjugates (10V) as measured after injection of mice using an opsonophagocytosis assay. GMT = geometric means titer.
**Figure 16****:** Graph comparing the immunogenicity of a composition comprising 12 saccharide conjugates, PhtD, dPly and PE-PilA (12V + prot) with a composition comprising PhtD, dPly and PE-PilA alone (prot) as measured after injection of mice using an anti-protein ELISA assay. GMC= geometric mean concentration . IC = confidence intervals.
**Figure 17****:** Graph comparing the immunogenicity of a composition comprising 12 saccharide conjugates, PhtD, dPly and PE-PilA (12V + prot) with a composition comprising 12 saccharides conjugates (12V) and a composition comprising 10 saccharide conjugates (10V) as measured after injection of guinea pigs using an opsonophagocytosis assay. GMT = geometric means titer.
**Figure 18****:** Graph comparing the immunogenic of a composition comprising 12 saccharide conjugates, PhtD, dPly and PE-PilA (12V + prot) with a composition comprising 12 saccharides conjugates (12V) and a composition comprising 10 saccharide conjugates (10V) as measured after injection of guinea pigs using an anti-saccharide ELISA. GMC= geometric mean concentration . IC = confidence intervals.

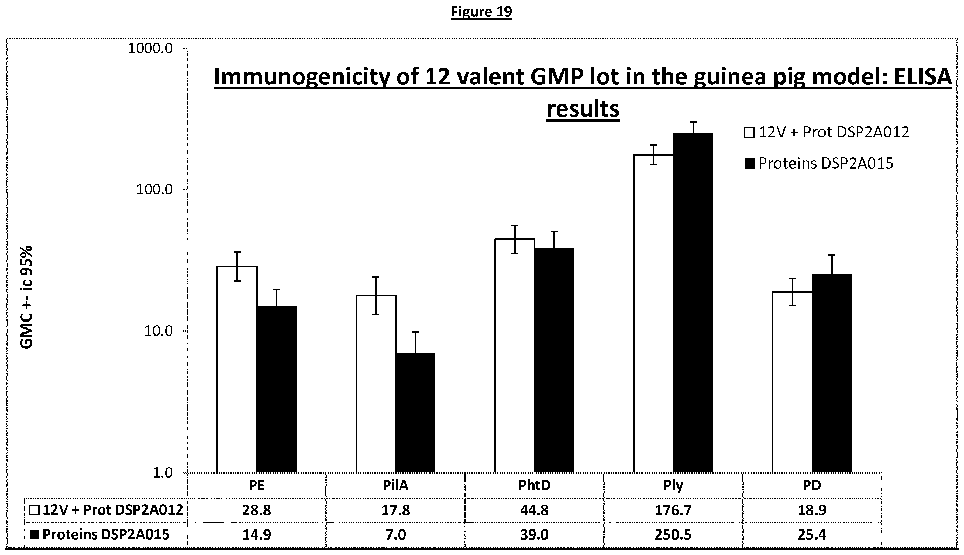

**Figure 19****:** Graph comparing the immunogenic of a composition comprising 12 saccharide conjugates, PhtD, dPly and PE-PilA (12V + prot) with a composition comprising PhtD, dPly and PE-PilA alone (prot) as measured after injection of guinea pigs using an anti-protein ELISA. GMC= geometric mean concentration . ic = confidence intervals.

### DETAILED DESCRIPTION

In a first aspect, the present invention relates to an immunogenic composition comprising 26µg-45µg (for example 26µg-40µg, 27µg-39µg, 27µg-37µg, 28µg-35µg, 29µg-33µg or around 30µg) of pneumolysin, per human dose and 26µg-45µg (26µg-40µg, 27µg-39µg, 27µg-37µg, 28µg-35µg, 29µg-33µg or around 30µg) of PhtD, per human dose for use in the treatment or prevention of disease caused by *Streptococcus pneumoniae* infection. In one embodiment the pneumolysin has been chemically detoxified. In a further embodiment the pneumolysin has been genetically detoxified.

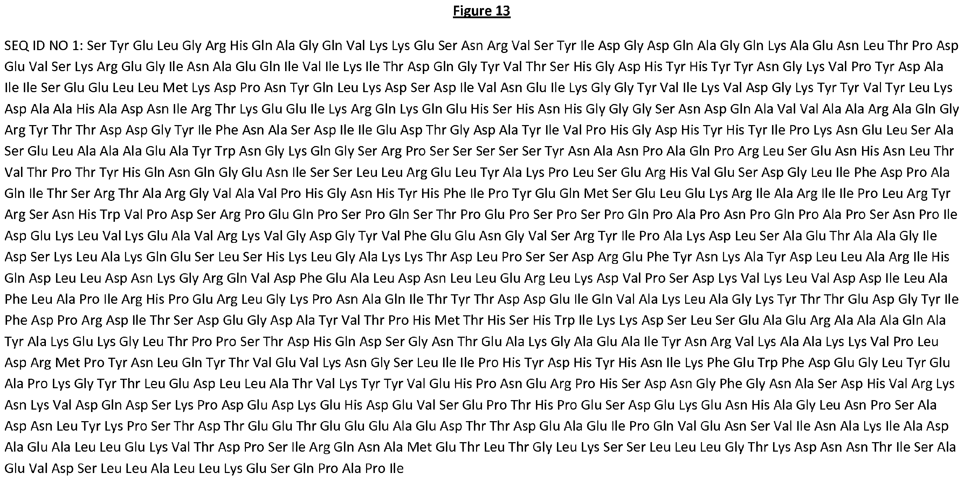

In one embodiment the PhtD comprises an amino acid sequence at least 85%, 90%, 95%, 98%, 99% or 100% identical to the sequence at amino acids 21-838 of SEQ ID NO:4 of WO00/37105 (herein incorporated by reference) (amino acids 21-828 of SEQ ID NO:4 of WO00/37105 have the sequence of SEQ ID NO: 1 in Figure 13 of the present application).

### Pneumolysin

By pneumolysin, or "Ply", it is meant: native or wild-type pneumolysin from pneumococcus, recombinant pneumolysin, and fragments and/or variants thereof. In an embodiment, pneumolysin is native or wild-type pneumolysin from pneumococcus or recombinant pneumolysin.

Ply is a 53 kDa pore-forming toxin and as such has lytic effects on many mammalian cell types, and at sublytic concentrations Ply has numerous other effects, including complement activation in the absence of antipneumolysin antibodies and induction of pro-inflammatory mediators (Hirst et al., 2000; Mitchell & Andrew, 1997; Paton et al., 1984; Zysk et al., 2001). Complement activation is independent of the haemolytic activity of the toxin (Berry et al., 1995). Pneumolysin is now known to interact directly with Toll-like receptor 4 (TLR-4) and signal via myeloid differentiation marker 88 to induce production of tumour necrosis factor alpha and interleukin 6 (Malley et al., 2003; Trzcinski et al., 2008). Pneumolysin is produced by all known clinical isolates of *S. pneumoniae* regardless of serotype and genotype. Expression and cloning of wild-type or native pneumolysin is known in the art. See, for example, Walker et al. (Infect Immun, 55:1184-1189 (1987)), Mitchell et al. (Biochim Biophys Acta, 1007:67-72 (1989) and Mitchell et al (NAR, 18:4010 (1990)). WO2010/071986 (herein incorporated by reference) describes wild-type Ply, e.g. SEQ IDs 2-42 (for example SEQ IDs 34, 35, 36, 37, 41). In one aspect, pneumolysin is Seq ID NO:34 of WO2010/071986. In another aspect, pneumolysin is Seq ID NO:35 of WO2010/071986. In another aspect, pneumolysin is Seq ID NO:36 of WO2010/071986. In another aspect, pneumolysin is Seq ID NO:37 of WO2010/071986. In another aspect, pneumolysin is Seq ID NO:41 of WO2010/071986. Furthermore, EP1601689B1 describes methods for purifying bacterial cytolysins such as pneumococcal pneumolysin by chromatography in the presence of detergent and high salt.

In an embodiment, the term 'pneumolysin' includes fragments and/or variants of pneumolysin, having differences in nucleic acid or amino acid sequences as compared to a wild type sequence. Where fragments of pneumolysin are used, these fragments will be at least about 15, at least about 20, at least about 40, or at least about 60 contiguous amino acid residues in length. In an embodiment of the invention, immunogenic fragments of pneumolysin comprise at least about 15, at least about 20, at least about 40, or at least about 60 contiguous amino acid residues of the full length sequence, wherein said polypeptide is capable of eliciting an immune response specific for said amino acid sequence. Pneumolysin is known to consist of four major structural domains (Rossjohn et al. Cell. 1997 May 30; 89(5):685-92). These domains may be modified by removing and/or modifying one or more of these domains. In an embodiment, the or each fragment contains exactly or at least 1, 2 or 3 domains. In another embodiment, the or each fragment contains exactly or at least 2 or 3 domains. In another embodiment, the or each fragment contains at least 3 domains. The or each fragment may be more than 50, 60, 70, 80, 90, 95, 98, 99 or 100% identical to a wild type pneumolysin sequence.

In accordance with the present invention, a variant of pneumolysin includes sequences in which one or more amino acids are substituted and/or deleted and/or inserted compared to the wild type sequence. Amino acid substitution may be conservative or non-conservative. In one aspect, amino acid substitution is conservative. Substitutions, deletions, insertions or any combination thereof may be combined in a single variant so long as the variant is an immunogenic polypeptide. Variants of pneumolysin typically include any pneumolysin or any fragment of pneumolysin which shares at least 80, 90, 94, 95, 98, or 99% amino acid sequence identity with a wild-type pneumolysin sequence, e.g. SEQ IDs 2-42 from WO2010/071986 (for example SEQ IDs 34, 35, 36, 37, 41). In an embodiment, variants of pneumolysin typically include any pneumolysin or any fragment of pneumolysin which shares at least 80, 90, 94, 95, 98, or 99% amino acid sequence identity with SEQ ID 36 from WO2010/071986. In an embodiment, the present invention includes fragments and/or variants in which several, 5 to 10, 1 to 5, 1 to 3, 1 to 2, 1, up to 10, up to 20, up to 30 or up to 50 amino acids are substituted, deleted, or added in any combination. In another embodiment, the present invention includes fragments and/or variants which comprise a B-cell or T-cell epitope. Such epitopes may be predicted using a combination of 2D-structure prediction, e.g. using the PSIPRED program (from David Jones, Brunel Bioinformatics Group, Dept. Biological Sciences, Brunel University, Uxbridge UB8 3PH, UK) and antigenic index calculated on the basis of the method described by Jameson and Wolf (CABIOS 4:181-186 [1988]). Variants of pneumolysin are described for example in WO04/43376, WO05/108580, WO05/076696, WO10/071986, WO10/109325 (all of which are herein incorporated by reference) (SEQ IDs 44, 45 and 46) and WO10/140119 (herein incorporated by reference) (SEQ IDs 50 and 51). In an embodiment, the immunogenic composition of the invention comprises a variant of pneumolysin, for example, those described in WO05/108580, WO05/076696, WO10/071986.

The term "fragment" as used in this specification is a moiety that is capable of eliciting a humoral and/or cellular immune response in a host animal. Fragments of a protein can be produced using techniques known in the art, e.g. recombinantly, by proteolytic digestion, or by chemical synthesis. Internal or terminal fragments of a polypeptide can be generated by removing one or more nucleotides from one end (for a terminal fragment) or both ends (for an internal fragment) of a nucleic acid which encodes the polypeptide. Typically, fragments comprise at least 10, 20, 30, 40 or 50 contiguous amino acids of the full length sequence. Fragments may be readily modified by adding or removing 1, 2, 3, 4, 5, 6, 7, 8, 9, 10, 20, 30, 40 or 50 amino acids from either or both of the N and C termini.

The term "conservative amino acid substitution" as used in this specification involves substitution of a native amino acid residue with a non-native residue such that there is little or no effect on the size, polarity, charge, hydrophobicity, or hydrophilicity of the amino acid residue at that position, and without resulting in decreased immunogenicity. For example, these may be substitutions within the following groups: valine, glycine; glycine, alanine; valine, isoleucine, leucine; aspartic acid, glutamic acid; asparagine, glutamine; serine, threonine; lysine, arginine; and phenylalanine, tyrosine. Conservative amino acid modifications to the sequence of a polypeptide (and the corresponding modifications to the encoding nucleotides) may produce polypeptides having functional and chemical characteristics similar to those of a parental polypeptide.

The term "deletion" as used in this specification is the removal of one or more amino acid residues from the protein sequence. Typically, no more than about from 1 to 6 residues (e.g. 1 to 4 residues) are deleted at any one site within the protein molecule.

The term "insertion" as used in this specification is the addition of one or more non-native amino acid residues in the protein sequence. Typically, no more than about from 1 to 6 residues (e.g. 1 to 4 residues) are inserted at any one site within the protein molecule.

In an embodiment of the invention, pneumolysin and its fragments and/or variants thereof, have an amino acid sequence sharing at least 80, 85, 90, 95, 98, 99 or 100% identity with the wild type sequence for pneumolysin, e.g. SEQ IDs 34, 35, 36, 37, 41 from WO2010/071986. In another embodiment of the invention, pneumolysin and its fragments and/or variants thereof, comprise at least about 15, at least about 20, at least about 40, or at least about 60 contiguous amino acid residues of the wild type sequence for pneumolysin.

Pneumolysin is administered after being detoxified (i.e. rendered non-toxic to a human when provided at a dosage suitable for protection). As used herein, it is understood that the term "dPly" refers to detoxified pneumolysin suitable for medical use (i.e. non toxic). Pneumolysin may be detoxified chemically and/or genetically. Detoxification of pneumolysin can be conducted by chemical means, e.g. using a crosslinking agent, such as formaldehyde, glutaraldehyde and a cross-linking reagent containing an N-hydroxysuccinomido ester and/or a maleimide group (e.g. GMBS) or a combination of these. In one embodiment the pneumolysin is detoxified by exposure to formaldehyde. Such methods are well known in the art for various toxins; see for example EP1601689B1, WO04/081515, WO2006/032499. The pneumolysin used in chemical detoxification may be a native or recombinant protein or a protein that has been genetically engineered to reduce its toxicity (see below). Fusion proteins of pneumolysin or fragments and/or variants of pneumolysin may also be detoxified by chemical means. Therefore, in an embodiment, immunogenic compositions of the invention may comprise pneumolysin which has been chemically detoxified, e.g. by a formaldehyde treatment.

Pneumolysin can also be genetically detoxified. Thus, the invention encompasses pneumococcal proteins which may be, for example, mutated proteins. The term "mutated" is used herein to mean a molecule which has undergone deletion, addition or substitution of one or more amino acids (e.g. 1, 2, 3, 4, 5, 6, 7, 8, 9, 10, 11 or 12 amino acids), for example by using well known techniques for site directed mutagenesis or any other conventional method. In one embodiment, the molecule has undergone deletion or substitution of 1-15, suitably 10-15 amino acids. The mutated sequences may remove undesirable activities such as membrane permeation, cell lysis, and cytolytic activity against human erythrocytes and other cells, in order to reduce the toxicity, whilst retaining the ability to induce anti-pneumolysin protective and/or neutralizing antibodies following administration to a human. Fusion proteins of pneumolysin or fragments and/or variants of pneumolysin may also be detoxified by genetic means. Any of these modifications may be introduced using standard molecular biology and biochemical techniques. For example, as described above, a mutant pneumolysin protein may be altered so that it is biologically inactive whilst still maintaining its immunogenic epitopes, see, for example, WO90/06951, Berry et al. (Infect Immun, 67:981-985 (1999)) and WO99/03884. For example, a pneumolysin protein may be detoxified by three amino acid substitutions comprising T₆₅ to C, G₂₉₃ to C and C₂₄₈ to A. Another example of a genetically detoxified pneumolysin that can be used in the present invention is SEQ ID 9 from WO2011/075823 (herein incorporated by reference). Thus, in a further embodiment, immunogenic compositions of the invention may comprise pneumolysin which has been genetically detoxified.

A combination of techniques may be used to detoxify pneumolysin. For example, immunogenic compositions of the invention may comprise pneumolysin which has been chemically and genetically detoxified.

### PhtD

The term "PhtD" (Poly Histidine Triad D) as used herein includes the full length protein with the signal sequence attached or the mature full length protein with the signal peptide (for example 20 amino acids at N-terminus) removed, and fragments, variants and/or fusion proteins thereof, e.g. SEQ ID NO: 4 of WO00/37105. PhtD is also referred to "Sp036D". In one aspect, PhtD is the full length protein with the signal sequence attached e.g. SEQ ID NO: 4 of WO00/37105. In another aspect, PhtD is a sequence comprising the mature full length protein with the signal peptide (for example 20 amino acids at N-terminus) removed, e.g. amino acids 21-838 of SEQ ID NO: 4 of WO00/37105 (or SEQ ID NO:1 of the present application). Suitably, the PhtD sequence comprises an N-terminal methionine. The present invention also includes PhtD polypeptides which are immunogenic fragments of PhtD, variants of PhtD and/or fusion proteins of PhtD. For example, as described in WO00/37105, WO00/39299, US6699703 and WO09/12588.

Where fragments of PhtD proteins are used (separately or as part of a fusion protein), these fragments will be at least about 15, at least about 20, at least about 40, or at least about 60 contiguous amino acid residues in length, e.g from a PhtD amino acid sequence in WO00/37105 or WO00/39299, such as SEQ ID NO: 4 of WO00/37105. In an embodiment of the invention, immunogenic fragments of PhtD protein comprise at least about 15, at least about 20, at least about 40, or at least about 60 contiguous amino acid residues of the sequence shown in SEQ ID NO: 4 of WO00/37105, wherein said polypeptide is capable of eliciting an immune response specific for said amino acid sequence. In an embodiment, the immunogenic composition of the invention comprises a fragment of PhtD, for example described in WO09/12601, WO01/98334 and WO09/12588. Where fragments of PhtD proteins are used (separately or as part of a fusion protein), each fragment optionally contains one or more histidine triad motif(s) of such polypeptides. A histidine triad motif is the portion of polypeptide that has the sequence HxxHxH (SEQ ID NO:2) where H is histidine and x is an amino acid other than histidine. In an embodiment of the present invention, the or each fragment contains exactly or at least 2, 3, 4 or 5 histidine triad motifs (optionally, with native PhtD sequence between the 2 or more triads, or intra-triad sequence) where the fragment is more than 50, 60, 70, 80, 85, 90, 95, 98, 99 or 100% identical to a native pneumococcal intra-triad PhtD sequence (e.g. the intra-triad sequence shown in SEQ ID NO: 4 of WO00/37105). Fragments of PhtD proteins optionally contain one or more coiled coil regions of such polypeptides. A coiled coil region is a region predicted by "Coils" algorithm Lupus, A et al (1991) Science 252; 1162-1164. In an embodiment of the present invention, the or each fragment contains exactly or at least 2, 3 or 4 coiled coil regions. In an embodiment of the present invention, the or each fragment contains exactly or at least 2, 3 or 4 coiled coil regions where the fragment is more than 50, 60, 70, 80, 90, 95, 96, 98, 99 or 100% identical to a native pneumococcal PhtD sequence (e.g. the sequence shown in SEQ ID NO: 4 of WO00/37105). In another embodiment of the present invention, the or each fragment includes one or more histidine triad motif as well as at least 1, 2, 3 or 4 coiled coil regions.

In the case where the PhtD polypeptide is a variant, the variation is generally in a portion thereof other than the histidine triad residues and the coiled-coil region, although variations in one or more of these regions may be made. In accordance with the present invention, a polypeptide variant includes sequences in which one or more amino acids are substituted and/or deleted and/or inserted compared to the wild type sequence. Amino acid substitution may be conservative or non-conservative. In one aspect, amino acid substitution is conservative. Substitutions, deletions, insertions or any combination thereof may be combined in a single variant so long as the variant is an immunogenic polypeptide. Variants of PhtD typically include any fragment or variation of PhtD which shares at least 80, 90, 95, 96, 98, or 99% amino acid sequence identity with a wild-type PhtD sequence, e.g. SEQ ID NO: 4 of WO00/37105. In an embodiment, the present invention includes fragments and/or variants in which several, 5 to 10, 1 to 5, 1 to 3, 1 to 2,1, up to 10, up to 20, up to 30 or up to 50 amino acids are substituted, deleted, or added in any combination. In another embodiment, the present invention includes fragments and/or variants which comprise a B-cell or T-cell epitope. Such epitopes may be predicted using a combination of 2D-structure prediction, e.g. using the PSIPRED program (from David Jones, Brunel Bioinformatics Group, Dept. Biological Sciences, Brunel University, Uxbridge UB8 3PH, UK) and antigenic index calculated on the basis of the method described by Jameson and Wolf (CABIOS 4:181-186 [1988]). Variants can be produced by conventional molecular biology techniques. Variants as used herein may also include naturally occurring PhtD alleles from alternate *Streptococcus* strains that exhibit polymorphisms at one or more sites within the homologous PhtD gene.

In an embodiment of the invention, PhtD and its fragments, variants and/or fusion proteins thereof comprise an amino acid sequence sharing at least 80, 85, 90, 95, 96, 97, 98, 99 or 100% identity with amino acid sequence 21 to 838 of SEQ ID NO:4 of WO00/37105. In another embodiment of the invention, PhtD and its fragments, variants and/or fusion proteins thereof have an amino acid sequence sharing at least 80, 85, 90, 95, 96, 97, 98, 99 or 100% identity with amino acid sequence 21 to 838 of SEQ ID NO:4 of WO00/37105. Suitably, PhtD and its fragments, variants and/or fusion proteins thereof comprise an amino acid sequence having an N-terminal methionine. In another embodiment of the invention, PhtD and its fragments, variants and/or fusion proteins thereof comprise at least about 15, at least about 20, at least about 40, or at least about 60 or at least about 100, or at least about 200, or at least about 400 or at least about 800 contiguous amino acid residues of the sequence shown in SEQ ID NO: 4 of WO00/37105.

In an embodiment of the invention, PhtD and its fragments, variants and/or fusion proteins thereof comprise an amino acid sequence sharing at least 80, 85, 90, 95, 96, 97, 98, 99 or 100% identity with amino acid sequence SEQ ID NO:73 of WO00/39299 (herein incorporated by reference). In another embodiment of the invention, PhtD and its fragments, variants and/or fusion proteins thereof have an amino acid sequence sharing at least 80, 85, 90, 95, 96, 97, 98, 99 or 100% identity with amino acid sequence SEQ ID NO:73 of WO00/39299. In another embodiment of the invention, PhtD and its fragments, variants and/or fusion proteins thereof comprise at least about 15, at least about 20, at least about 40, or at least about 60, or at least about 100, or at least about 200, or at least about 400 or at least about 800 contiguous amino acid residues of the sequence shown in SEQ ID NO: 73 of WO00/39299. In another embodiment of the invention, the PhtD sequence is SEQ ID NO. 1 or 5 from WO2011/075823.

The present invention also includes PhtD proteins which differ from naturally occurring *S. pneumoniae* polypeptides in ways that do not involve the amino acid sequence. Non-sequence modifications include changes in acetylation, methylation, phosphorylation, carboxylation, or glycosylation. Also within the invention are those with modifications which increase peptide stability; such analogs may contain, for example, one or more non-peptide bonds (which replace the peptide bonds) in the peptide sequence. Also within the invention are analogs that include residues other than naturally occurring L-amino acids, e.g. D-amino acids or non-naturally occurring or synthetic amino acids, e.g. β or γ amino acids, and cyclic analogs.

### Dosage

The term "human dose" as used herein means a dose which is in a volume suitable for human use. Generally the final dose volume (vaccine composition volume) administered to a human patient may be between 0.25 to 1.5 ml, 0.2 to 1.0 ml, or 0.4 to 0.6 ml. In an embodiment, a human dose is 0.5 ml. In a further embodiment, a human dose is higher than 0.5 ml, for example 0.6, 0.7, 0.8, 0.9 or 1 ml. In a further embodiment, a human dose is between 1 ml and 1.5 ml. In another embodiment, in particular when the immunogenic composition is for the paediatric population, a human dose may be less than 0.5 ml such as between 0.25 and 0.5 ml.

### Further unconjugated or conjugated S. pneumoniae proteins

The immunogenic compositions of the invention may comprise at least one further unconjugated or conjugated *Streptococcus pneumoniae* protein. For the purposes of the present application the terms *S.pneumoniae* and *Streptococcus pneumoniae* are considered to be interchangeable and equivalent to one another. In one embodiment the at least one further unconjugated or conjugated *Streptococcus pneumoniae* protein is selected from the group consisting of Poly Histidine Triad family (PhtX), Choline Binding Protein Family (CbpX), CbpX truncates, LytX (autolytic enzyme) family, LytX truncates, CbpX truncate-LytX truncate chimeric proteins, PcpA (pneumococcal choline binding protein A), PspA (Pneumococcal Surface Protein A), PsaA (pneumococcal surface adhesion protein A, Sp128 (*Streptococcus pneumoniae* 128), Sp101 (*Streptococcus pneumoniae* 101), Sp130 (*Streptococcus pneumoniae* 130), SP125 (*Streptococcus pneumoniae* 125) and SP133 (*Streptococcus pneumoniae* 133). In a further embodiment the at least one further unconjugated or conjugated *Streptococcus pneumoniae* protein is selected from the group consisting of Choline Binding Protein Family (CbpX), CbpX truncates, LytX family, LytX truncates, CbpX truncate-LytX truncate chimeric proteins, PcpA, PspA, PsaA, Sp128, Sp101, Sp130, SP125 and SP133.

The Pht (Poly Histidine Triad) family comprises proteins PhtA (Poly Histidine Triad family A), PhtB (Poly Histidine Triad family B), PhtD (Poly Histidine Triad family B), and PhtE(Poly Histidine Triad family E). The Poly Histidine Triad family is also known as the Pneumococal Histidine Triad family and thus the terms 'Poly Histidine Triad' and 'Pneumococcal Histidine Triad' are considered to be interchangeable and equivalent to one another. The family is characterized by a lipidation sequence, two domains separated by a proline-rich region and several histidine triads, possibly involved in metal or nucleoside binding or enzymatic activity, (3-5) coiled-coil regions, a conserved N-terminus and a heterogeneous C terminus. It is present in all strains of pneumococci tested. Homologous proteins have also been found in other Streptococci and Neisseria. In one embodiment of the invention, the Pht protein of the invention is PhtD. It is understood, however, that the terms Pht A, B, D, and E refer to proteins having sequences disclosed in the citations below as well as naturally-occurring (and man-made) variants thereof that have a sequence homology that is at least 90% identical to the referenced proteins. Optionally it is at least 95% identical or at least 97% identical.

With regards to the PhtX proteins, PhtA is disclosed in WO 98/18930, and is also referred to Sp36. As noted above, it is a protein from the Poly Histidine triad family and has the type II signal motif of LXXC (SEQ ID NO:3). PhtD is disclosed in WO 00/37105, and is also referred to Sp036D. As noted above, it also is a protein from the Poly Histidine triad family and has the type II LXXC signal motif. PhtB is disclosed in WO 00/37105, and is also referred to Sp036B. Another member of the PhtB family is the C3-Degrading Polypeptide, as disclosed in WO 00/17370. This protein also is from the Poly Histidine triad family and has the type II LXXC signal motif. For example, an immunologically functional equivalent is the protein Sp42 disclosed in WO 98/18930. A PhtB truncate (approximately 79kD) is disclosed in WO99/15675 which is also considered a member of the PhtX family. PhtE is disclosed in WO00/39299 and is referred to as BVH-3. Where any Pht protein is referred to herein, it is meant that immunogenic fragments or fusions thereof of the Pht protein can be used. For example, a reference to PhtX includes immunogenic fragments or fusions thereof from any Pht protein. A reference to PhtD or PhtB is also a reference to PhtDE or PhtBE fusions as found, for example, in WO0198334.

Concerning the Choline Binding Protein family (CbpX), members of that family were originally identified as pneumococcal proteins that could be purified by choline-affinity chromatography. All of the choline-binding proteins are non-covalently bound to phosphorylcholine moieties of cell wall teichoic acid and membrane-associated lipoteichoic acid. Structurally, they have several regions in common over the entire family, although the exact nature of the proteins (amino acid sequence, length, etc.) can vary. In general, choline binding proteins comprise an N terminal region (N), conserved repeat regions (R1 and/or R2), a proline rich region (P) and a conserved choline binding region (C), made up of multiple repeats, that comprises approximately one half of the protein. As used in this application, the term "Choline Binding Protein family (CbpX)" is selected from the group consisting of Choline Binding Proteins as identified in WO97/41151, PbcA, SpsA, PspC, CbpA, CbpD, and CbpG. CbpA is disclosed in WO97/41151. CbpD and CbpG are disclosed in WO00/29434. PspC is disclosed in WO97/09994. PbcA is disclosed in WO98/21337.SpsA is a Choline binding protein disclosed in WO 98/39450. Optionally the Choline Binding Proteins are selected from the group consisting of CbpA, PbcA, SpsA and PspC.

An embodiment of the invention comprises CbpX truncates wherein "CbpX" is defined above and "truncates" refers to CbpX proteins lacking 50% or more of the Choline binding region (C). Optionally such proteins lack the entire choline binding region. Optionally, the such protein truncates lack (i) the choline binding region and (ii) a portion of the N-terminal half of the protein as well, yet retain at least one repeat region (R1 or R2). Optionally, the truncate has 2 repeat regions (R1 and R2). Examples of such embodiments are NR1xR2 and R1xR2 as illustrated in WO99/51266 or WO99/51188, however, other choline binding proteins lacking a similar choline binding region are also contemplated within the scope of this invention.

The LytX family is membrane associated proteins associated with cell lysis. The N-terminal domain comprises choline binding domain(s), however the LytX family does not have all the features found in the CbpA family noted above and thus for the present invention, the LytX family is considered distinct from the CbpX family. In contrast with the CbpX family, the C-terminal domain contains the catalytic domain of the LytX protein family. The family comprises LytA, B and C. With regards to the LytX family, LytA is disclosed in Ronda et al., Eur J Biochem, 164:621-624 (1987). LytB is disclosed in WO 98/18930, and is also referred to as Sp46. LytC is also disclosed in WO 98/18930, and is also referred to as Sp91. An embodiment of the invention comprises LytC.

Another embodiment comprises LytX truncates wherein "LytX" is defined above and "truncates" refers to LytX proteins lacking 50% or more of the Choline binding region. Optionally such proteins lack the entire choline binding region. Yet another embodiment of this invention comprises CbpX truncate-LytX truncate chimeric proteins (or fusions). Optionally this comprises NR1xR2 (or R1xR2) of CbpX and the C-terminal portion (Cterm, i.e., lacking the choline binding domains) of LytX (e.g., LytCCterm or Sp91 Cterm). Optionally CbpX is selected from the group consisting of CbpA, PbcA, SpsA and PspC. Optionally, it is CbpA. Optionally, LytX is LytC (also referred to as Sp91). Another embodiment of the present invention is a PspA or PsaA truncate lacking the choline binding domain (C) and expressed as a fusion protein with LytX. Optionally, LytX is LytC.

With regards to PsaA and PspA, both are know in the art. For example, PsaA and transmembrane deletion variants thereof have been described by Berry & Paton, Infect Immun 1996 Dec;64(12):5255-62. PspA and transmembrane deletion variants thereof have been disclosed in, for example, US 5804193, WO 92/14488, and WO 99/53940.

With regards to PcpA this protein is known in the art, for example PcpA has been described in WO2011/075823. The term 'PcpA' refers to a protein comprising at least 80%, 85%, 90%, 95%, 98%, 99% or 100% identical to SEQ ID NO:2 or 7 from WO2011/075823 (herein incorporated by reference) or fragments of at least 100, 150, 200, 250 or more consecutive amino acids of SEQ ID NO:2 or 7 from WO2011/075823.

Sp128 and Sp130 are disclosed in WO00/76540. Sp125 is an example of a pneumococcal surface protein with the Cell Wall Anchored motif of LPXTG (SEQ ID NO:4)(where X is any amino acid). Any protein within this class of pneumococcal surface protein with this motif has been found to be useful within the context of this invention, and is therefore considered a further protein of the invention. Sp125 itself is disclosed in WO 98/18930, and is also known as ZmpB - a zinc metalloproteinase. Sp101 is disclosed in WO 98/06734 (where it has the reference # y85993). It is characterized by a Type I signal sequence. Sp133 is disclosed in WO 98/06734 (where it has the reference # y85992). It is also characterized by a Type I signal sequence.

Any of these further *Streptococcus pneumoniae* proteins may be present is an unconjugated or conjugated form. One or more *S.pneumoniae* proteins is optionally conjugated to an *S.pneumoniae* saccharide (described in the section entitled *Streptococcus pneumoniae* saccharide conjugates below). Optionally one or more *Streptococcus pneumoniae* proteins is conjugated to a saccharide from a different bacterium.

The term 'conjugated to' in this context means that the protein is covalently bonded to a saccharide, in this situation the protein is acting as a carrier protein.

In an embodiment the at least one further unconjugated or conjugated *S.pneumoniae* protein comprises a Poly Histidine family (PhtX) protein selected from the group consisting of PhtB, PhtE, PhtA, and a PhtBD or PhtDE fusion protein.

In one embodiment the composition comprises 1µg-100µg, 1µg-75µg, 1µg-50µg, 1µg-25µg, 1µg-20µg, 5µg-15µg, 25µg-40µg, 28µg-35µg, around 10µg or around 30µg of the at least one further unconjugated or conjugated *S.pneumoniae* protein, per human dose per protein.

### Streptococcus pneumoniae capsular saccharide conjugates

In one embodiment the immunogenic compositions of the invention further comprises 7 or more (e.g. 7, 8, 9, 10, 11, 12, 13, 14, 15, 16, 17, 18, 19, 20, 21, 22, or 23) *Streptococcus pneumoniae* capsular saccharide conjugates. The term capsular saccharide includes capsular polysaccharides and oligosaccharides derivable from the capsular polysaccharide. An oligosaccharide contains at least 4 sugar residues. The terms conjugate and conjugated relate to a capsular saccharide covalently bonded to a carrier protein.

Optionally the total number of saccharide serotypes is less than or equal to 23, optionally the immunogenic composition comprising 10-23 serotypes, 10-16, serotypes 10-15 serotypes, 10-14 serotypes, 10-13 serotypes or 10-12 serotypes.

In one embodiment the 7 or more *Streptococcus pneumoniae* capsular saccharide conjugates of the invention will comprise saccharides derived from serotypes selected from the following serotypes 1, 2, 3, 4, 5, 6A, 6B, 7F, 8, 9N, 9V, 10A, 11A, 12F, 14, 15B, 17F, 18C, 19A, 19F, 20, 22F, 23F and 33F, although it is appreciated that one or two other serotypes could be substituted depending on the age of the recipient receiving the immunogenic composition and the geographical location where the vaccine will be administered. For example, a 7 valent immunogenic composition may comprise saccharides from serotypes 4, 6B, 9V, 14, 18C, 19F and 23F. A 10 valent immunogenic composition may comprise saccharides derived from the same 7 serotypes and further comprise saccharides from serotypes 1, 5 and 7F. A 12 valent immunogenic composition may comprise saccharides derived from the same 10 serotypes and further comprises saccharides derived from serotypes 6A and 19A. A 13 valent immunogenic composition may comprise the same 12 serotypes and further comprise serotype 3. A 15 valent immunogenic composition may comprise saccharides derived from the same 13 serotypes and further comprise saccharides derived from serotypes 22F and 33F.

Further saccharide antigens, for example 23 valent (such as serotypes 1, 2, 3, 4, 5, 6B, 7F, 8, 9N, 9V, 10A, 11A, 12F, 14, 15B, 17F, 18C, 19A, 19F, 20, 22F, 23F and 33F), are also contemplated by the invention.

The term "carrier protein" is intended to cover both small peptides and large polypeptides (>10 kDa). The carrier protein may be any peptide or protein. It may comprise one or more T-helper epitopes. The carrier protein may be tetanus toxoid (TT), tetanus toxoid fragment C, non-toxic mutants of tetanus toxin [note all such variants of TT are considered to be the same type of carrier protein for the purposes of this invention], polypeptides comprising tetanus toxin T-cell epitopes such as N19 (WO2006/067632), diphtheria toxoid (DT), CRM197 (cross reacting material 197), other non-toxic mutants of diphtheria toxin [such as CRM176, CRM 197, CRM228, CRM 45 (Uchida et al J. Biol. Chem. 218; 3838-3844, 1973); CRM 9, CRM 45, CRM102, CRM 103 and CRM107 (where CRM stands for cross reacting material) and other mutations described by Nicholls and Youle in Genetically Engineered Toxins, Ed: Frankel, Maecel Dekker Inc, 1992; deletion or mutation of Glu-148 to Asp, Gln or Ser and/or Ala 158 to Gly and other mutations disclosed in US 4709017 or US 4950740; mutation of at least one or more residues Lys 516, Lys 526, Phe 530 and/or Lys 534 and other mutations disclosed in US 5917017 or US 6455673; or fragment disclosed in US 5843711] (note all such variants of DT are considered to be the same type of carrier protein for the purposes of this invention), pneumococcal pneumolysin (Kuo et al (1995) Infect Immun 63; 2706-13), OMPC (outer membrane protein C) (meningococcal outer membrane protein - usually extracted from N. meningitidis serogroup B - EP0372501), synthetic peptides (EP0378881, EP0427347), heat shock proteins (WO 93/17712, WO 94/03208), pertussis proteins (WO 98/58668, EP0471177), cytokines, lymphokines, growth factors or hormones (WO 91/01146), artificial proteins comprising multiple human CD4+ T cell epitopes from various pathogen derived antigens (Falugi et al (2001) Eur J Immunol 31; 3816-3824) such as N19 protein (Baraldoi et al (2004) Infect Immun 72; 4884-7) pneumococcal surface protein PspA (pneumococcal surface protein A) (WO 02/091998), iron uptake proteins (WO 01/72337), toxin A or B of C. difficile (WO 00/61761), *H. influenzae* Protein D (EP594610 and WO 00/56360), pneumococcal PhtA (poly histidine triad protein A) (WO 98/18930, also referred to Sp36), pneumococcal PhtD (poly histidine triad protein D (as described in the section on PhtD above), pneumococcal PhtB (poly histidine triad protein B) (disclosed in WO 00/37105, and is also referred to Sp036B), or PhtE (poly histidine triad protein E) (disclosed in WO00/30299 and is referred to as BVH-3).

In one embodiment the 7 or more *Streptococcus pneumoniae* capsular saccharide conjugates are conjugated to a carrier protein independently selected from the group consisting of tetanus toxoid (TT), fragment C of TT, diphtheria toxoid, CRM197 (cross reacting material 197), detoxified pneumolysin, protein D (from *H.influenzae*), PhtD, PhtDE and N19. In a further embodiment the 7 or more *Streptococcus pneumoniae* capsular saccharide conjugates are all independently conjugated to CRM197.

In one embodiment the immunogenic composition comprises at least one *Streptococcus pneumoniae* capsular saccharide conjugated to protein D. In one embodiment a minority of conjugated *Streptococcus pneumoniae* saccharides are conjugated to protein D. In a further embodiment the immunogenic composition comprises between 1-20, 1-18, 1-16, 1-14, 1-12, 1-10, 1-9, 1-8, 1-7, 1-6, 1-5, 1-4, or 1-2 *Streptococcus pneumoniae* capsular saccharide conjugated to protein D. In one embodiment the immunogenic composition comprises at least one *Streptococcus pneumoniae* capsular saccharides conjugated to diphtheria toxoid. In a further embodiment the immunogenic composition comprises a 19F saccharide conjugate wherein the 19F saccharide is conjugated to diphtheria toxoid. In one embodiment the immunogenic composition comprises at least one *Streptococcus pneumoniae* capsular saccharides conjugated to tetanus toxoid. In a further embodiment the immunogenic composition comprises an 18C saccharide conjugate wherein the 18C saccharide is conjugated to tetanus toxoid.

In one embodiment the immunogenic composition comprises a serotype 1 saccharide conjugated to protein D or CRM197. In one embodiment the immunogenic composition comprises a serotype 4 saccharide conjugated to protein D or CRM197. In one embodiment the immunogenic composition comprises a serotype 5 saccharide conjugated to protein D or CRM197. In one embodiment the immunogenic composition comprises a serotype 6B saccharide conjugated to protein D or CRM197. In one embodiment the immunogenic composition comprises a serotype 7F saccharide conjugated to protein D or CRM197. In one embodiment the immunogenic composition comprises a serotype 9V saccharide conjugated to protein D or CRM197. In one embodiment the immunogenic composition comprises a serotype 14 saccharide conjugated to protein D or CRM197. In one embodiment the immunogenic composition comprises a serotype 18C saccharide conjugated to tetanus toxoid or CRM197. In one embodiment the immunogenic composition comprises a 19F saccharide conjugated to diphtheria toxoid or CRM197. In one embodiment the immunogenic composition comprises a 23F saccharide conjugated to protein D or CRM197. In one embodiment the immunogenic composition comprises a 19A saccharide conjugated to CRM197. In one embodiment the immunogenic composition comprises a 6A saccharide conjugated to CRM197.

In one embodiment the 7 or more *Streptococcus pneumoniae* conjugates comprises a serotype 1 saccharide conjugated to protein D, a serotype 4 saccharide conjugated to protein D, a serotype 5 saccharide conjugated to protein D, a serotype 6B saccharide conjugated to protein D, a serotype 7F saccharide conjugated to protein D, a serotype 9V saccharide conjugated to protein D, a serotype 14 saccharide conjugated to protein D, a serotype 23F saccharide conjugated to protein D, a serotype 18C saccharide conjugated to tetanus toxoid and a 19F saccharide conjugated to diphtheria toxoid.

Optionally the ratio of carrier protein to *S. pneumoniae* saccharide is between 1:5 and 5:1; 1:2 and 2.5:1; 1:1 and 2:1 (w/w). In an embodiment, the majority of the conjugates, for example 6, 7, 8, 9 or more of the conjugates have a ratio of carrier protein to saccharide that is greater than 1:1, for example 1.1:1, 1.2:1, 1.3:1, 1.4:1, 1.5:1 or 1.6:1.

The term "saccharide" throughout this specification may indicate polysaccharide or oligosaccharide and includes both. Polysaccharides are isolated from bacteria and may be sized to some degree by known methods (see for example EP497524 and EP497525) and optionally by microfluidisation. Polysaccharides can be sized in order to reduce viscosity in polysaccharide samples and/or to improve filterability for conjugated products. Oligosaccharides have a low number of repeat units (typically 5-30 repeat units) and are typically hydrolysed polysaccharides.

Capsular polysaccharides of *Streptococcus pneumoniae* comprise repeating oligosaccharide units which may contain up to 8 sugar residues. For a review of the oligosaccharide units for the key Streptococcus pneumoniae serotypes see JONES, Christopher. Vaccines based on the cell surface carbohydrates of pathogenic bacteria. An. Acad. Bras. Ciênc., June 2005, vol.77, no.2, p.293-324. ISSN 0001-3765. In one embodiment, a capsular saccharide antigen may be a full length polysaccharide, however in others it may be one oligosaccharide unit, or a shorter than native length saccharide chain of repeating oligosaccharide units. In one embodiment, all of the saccharides present in the vaccine are polysaccharides. Full length polysaccharides may be "sized" i.e. their size may be reduced by various methods such as acid hydrolysis treatment, hydrogen peroxide treatment, sizing by emulsiflex® followed by a hydrogen peroxide treatment to generate oligosaccharide fragments or microfluidization.

### Conjugation

The saccharide conjugates present in the immunogenic compositions of the invention may be conjugated to a carrier protein using any conjugation technique.

In an embodiment, the *Streptococcus pneumoniae* saccharide is conjugated to the carrier protein via a linker, for instance a bifunctional linker. The linker is optionally heterobifunctional or homobifunctional, having for example a reactive amino group and a reactive carboxylic acid group, 2 reactive amino groups or two reactive carboxylic acid groups. The linker has for example between 4 and 20, 4 and 12, 5 and 10 carbon atoms. A possible linker is ADH. Other linkers include B-propionamido (WO 00/10599), nitrophenyl-ethylamine (Gever et al (1979) Med. Microbiol. Immunol. 165; 171-288), haloalkyl halides (US4057685), glycosidic linkages (US4673574, US4808700), hexane diamine and 6-aminocaproic acid (US4459286). In an embodiment, ADH is used as a linker for conjugating saccharide from serotype 18C.

The saccharide conjugates present in the immunogenic compositions of the invention may be prepared by any known coupling technique. The conjugation method may rely on activation of the saccharide with 1-cyano-4-dimethylamino pyridinium tetrafluoroborate (CDAP) to form a cyanate ester. The activated saccharide may thus be coupled directly or via a spacer (linker) group to an amino group on the carrier protein. For example, the spacer could be cystamine or cysteamine to give a thiolated polysaccharide which could be coupled to the carrier via a thioether linkage obtained after reaction with a maleimide-activated carrier protein (for example using GMBS) or a haloacetylated carrier protein (for example using iodoacetimide [e.g. ethyl iodoacetimide HCl] or N-succinimidyl bromoacetate or SIAB, or SIA, or SBAP). Optionally, the cyanate ester (optionally made by CDAP chemistry) is coupled with hexane diamine or ADH and the amino-derivatised saccharide is conjugated to the carrier protein using carbodiimide (e.g. EDAC or EDC) chemistry via a carboxyl group on the protein carrier. Such conjugates are described in PCT published application WO 93/15760 Uniformed Services University and WO 95/08348 and WO 96/29094.

Other suitable techniques use carbodiimides, carbiinides, hydrazides, active esters, norborane, p-nitrobenzoic acid, N-hydroxysuccinimide, S-NHS, EDC, TSTU. Many are described in WO 98/42721. Conjugation may involve a carbonyl linker which may be formed by reaction of a free hydroxyl group of the saccharide with CDI (Bethell et al J. Biol. Chem. 1979, 254; 2572-4, Hearn et al J. Chromatogr. 1981. 218; 509-18) followed by reaction of with a protein to form a carbamate linkage. This may involve reduction of the anomeric terminus to a primary hydroxyl group, optional protection/deprotection of the primary hydroxyl group' reaction of the primary hydroxyl group with CDI to form a CDI carbamate intermediate and coupling the CDI carbamate intermediate with an amino group on a protein.

The conjugates can also be prepared by direct reductive amination methods as described in US 4365170 (Jennings) and US 4673574 (Anderson). Other methods are described in EP-0-161-188, EP-208375 and EP-0-477508.

A further method involves the coupling of a cyanogen bromide (or CDAP) activated saccharide derivatised with adipic acid dihydrazide (ADH) to the protein carrier by Carbodiimide condensation (Chu C. et al Infect. Immunity, 1983 245 256), for example using EDAC (1-ethyl-2-(3-dimethylaminopropyl) carbodiimide).

In an embodiment, a hydroxyl group (optionally an activated hydroxyl group for example a hydroxyl group activated to make a cyanate ester [e.g. using CDAP]) on a saccharide is linked to an amino or carboxylic group on a protein either directly or indirectly (through a linker). Where a linker is present, a hydroxyl group on a saccharide is optionally linked to an amino group on a linker, for example by using CDAP conjugation. A further amino group in the linker for example ADH) may be conjugated to a carboxylic acid group on a protein, for example by using carbodiimide chemistry, for example by using EDAC. In an embodiment, the pneumococcal capsular saccharide(s) is conjugated to the linker first before the linker is conjugated to the carrier protein. Alternatively the linker may be conjugated to the carrier before conjugation to the saccharide.

A combination of techniques may also be used, with some saccharide-protein conjugates being prepared by CDAP, and some by reductive amination.

In general the following types of chemical groups on a protein carrier can be used for coupling / conjugation:
A) Carboxyl (for instance via aspartic acid or glutamic acid). In one embodiment this group is linked to amino groups on saccharides directly or to an amino group on a linker with carbodiimide chemistry e.g. with EDAC.
B) Amino group (for instance via lysine). In one embodiment this group is linked to carboxyl groups on saccharides directly or to a carboxyl group on a linker with carbodiimide chemistry e.g. with EDAC. In another embodiment this group is linked to hydroxyl groups activated with CDAP or CNBr on saccharides directly or to such groups on a linker; to saccharides or linkers having an aldehyde group; to saccharides or linkers having a succinimide ester group.
C) Sulphydryl (for instance via cysteine). In one embodiment this group is linked to a bromo or chloro acetylated saccharide or linker with maleimide chemistry. In one embodiment this group is activated/modified with bis diazobenzidine.
D) Hydroxyl group (for instance via tyrosine). In one embodiment this group is activated/modified with bis diazobenzidine.
E) Imidazolyl group (for instance via histidine). In one embodiment this group is activated/modified with bis diazobenzidine.
F) Guanidyl group (for instance via arginine).
G) Indolyl group (for instance via tryptophan).

On a saccharide, in general the following groups can be used for a coupling: OH, COOH or NH2. Aldehyde groups can be generated after different treatments known in the art such as: periodate, acid hydrolysis, hydrogen peroxide, etc.
Direct coupling approaches:
   Saccharide-OH + CNBr or CDAP -----> cyanate ester + NH2-Prot -----> conjugate
   Saccharide-aldehyde + NH2-Prot -----> Schiff base + NaCNBH3 -----> conjugate
   Saccharide-COOH + NH2-Prot + EDAC -----> conjugate
   Saccharide-NH2 + COOH-Prot + EDAC -----> conjugate
Indirect coupling via spacer (linker) approaches:
   Saccharide-OH + CNBr or CDAP -----> cyanate ester + NH2----NH2 -----> saccharideNH2 + COOH-Prot + EDAC -----> conjugate
   Saccharide-OH + CNBr or CDAP -----> cyanate ester + NH2----SH -----> saccharide-SH + SH-Prot (native Protein with an exposed cysteine or obtained after modification of amino groups of the protein by SPDP for instance) -----> saccharide-S-S-Prot
   Saccharide-OH + CNBr or CDAP -----> cyanate ester + NH2----SH -----> saccharide-SH + maleimide-Prot (modification of amino groups) -----> conjugate
   Saccharide-OH + CNBr or CDAP -----> cyanate ester + NH2-----SH -----> Saccharide-SH + haloacetylated-Prot ----> Conjugate
   Saccharide-COOH + EDAC + NH2-----NH2 ---> saccharide-----NH2 + EDAC + COOH-Prot -----> conjugate
   Saccharide-COOH + EDAC+ NH2----SH -----> saccharide----SH + SH-Prot (native Protein with an exposed cysteine or obtained after modification of amino groups of the protein by SPDP for instance) -----> saccharide-S-S-Prot
   Saccharide-COOH + EDAC+ NH2----SH -----> saccharide----SH + maleimide-Prot (modification of amino groups) ----> conjugate
   Saccharide-COOH + EDAC + NH2----SH ---> Saccharide-SH + haloacetylated-Prot ----> Conjugate
   Saccharide-Aldehyde + NH2-----NH2 ----> saccharide---NH2 + EDAC + COOH-Prot ----> conjugate

Note: instead of EDAC above, any suitable carbodiimide may be used.

In summary, the types of protein carrier chemical group that may be generally used for coupling with a saccharide are amino groups (for instance on lysine residues), COOH groups (for instance on aspartic and glutamic acid residues) and SH groups (if accessible) (for instance on cysteine residues.

In one embodiment at least 1, 2, 3, 4, 5, 6, 7, 8, 9, 10, 11, 12, 13, 14, 15, 16, 17, 18, 19, 20, 21, 22, or 23 *S.pneumoniae* saccharides are conjugated to a carrier protein through reductive amination. In one embodiment less than 23, 22, 21, 20, 19, 18, 17, 16, 15, 14, 13, 12, 11, 10, 9, 8, 7, 6, 5, 4, 3, 2 or 1 *S.pneumoniae* saccharides are conjugated to a carrier protein through reductive amination. In one embodiment between 1 and 23, 2 and 22, 3 and 21, 4 and 20, 5 and 19, 6 and 18, 7 and 17, 8 and 16, 9 and 15, 10 and 14, 11 and 13, 1 and 23, and 22, 1 and 21, 1 and 20, 1 and 19, 1 and 18, 1 and 17, 1 and 16, 1 and 15, 1 and 14, 1 and 13, 1 and 12, 1 and 11, 1 and 10, 1 and 9, 1 and 8, 1 and 7, 1 and 6, 1 and 5, 1 and 4, 1 and 3, or 1 and 2 *S.pneumoniae* saccharide are conjugated to a carrier protein through reductive amination. In a further embodiment all of the *S.pneumoniae* capsular saccharides are conjugated to a carrier protein through reductive amination.

In one embodiment at least 1, 2, 3, 4, 5, 6, 7, 8, 9, 10, 11, 12, 13, 14, 15, 16, 17, 18, 19, 20, 21, 22, or 23 *S.pneumoniae* saccharides are conjugated to a carrier protein through CDAP chemistry. In one embodiment less than 23, 22, 21, 20, 19, 18, 17, 16, 15, 14, 13, 12, 11, 10, 9, 8, 7, 6, 5, 4, 3, 2 or 1 *S.pneumoniae* saccharides are conjugated to a carrier protein through CDAP chemistry. In one embodiment between 1 and 23, 2 and 22, 3 and 21, 4 and 20, 5 and 19, 6 and 18, 7 and 17, 8 and 16, 9 and 15, 10 and 14, 11 and 13, 1 and 23, and 22, 1 and 21, 10 and 23, 10 and 22, 10 and 21, 10 and 20, 10 and 19, 10 and 18, 10 and 17, 10 and 16, 10 and 15, 10 and 14, 10 and 13, 10 and 12, 10 and 11, 1 and 20, 1 and 19, 1 and 18, 1 and 17, 1 and 16, 1 and 15, 1 and 14, 1 and 13, 1 and 12, 1 and 11, 1 and 10, 1 and 9, 1 and 8, 1 and 7, 1 and 6, 1 and 5, 1 and 4, 1 and 3, or 1 and 2 *S.pneumoniae* saccharide are conjugated to a carrier protein through CDAP chemistry. In a further embodiment all of the *S.pneumoniae* capsular saccharides are conjugated to a carrier protein through CDAP chemistry.

In one embodiment the immunogenic composition of the invention comprises at least 1, 2, 3, 4, 5, 6, 7, 8, 9, 10, 11, 12, 13, 14, 15, 16, 17, 18, 19, 20, 21 or 22 saccharides conjugated to a carrier protein through reductive amination and comprises at least 1, 2, 3, 4, 5, 6, 7, 8, 9, 10, 11, 12, 13, 14, 15, 16, 17, 18, 19, 20, 21, or 22 saccharides conjugated to a carrier protein through a chemistry other than reductive amination for example CDAP chemistry.

In an embodiment capsular saccharides from at least one of the serotypes selected from the group consisting of serotypes 1, 3, 19A and 19F are conjugated through a chemistry other than reductive amination and at least one of the serotypes selected from the group consisting of serotypes 4, 5, 6A, 6B, 6C, 7F, 9V, 14, 18C and 23F are conjugated through reductive amination. In an embodiment, the immunogenic composition of the invention comprises *S. pneumoniae* capsular saccharide(s) from serotype 1 or 3 or 19A or 19F; 1 and 3; 1 and 19A; 1 and 19F; 3 and 19A; 3 and 19F; 19A and 19F; 1, 3 and 19A; 1, 3 and 19F, 1, 19A and 19F; 3, 19A and 19F or 1, 3, 19A and 19F conjugated to a protein carrier through a chemistry other than reductive animation. In an embodiment, 19F is conjugated to a carrier protein through a chemistry other than reductive amination. In an embodiment, the immunogenic composition of the invention comprises *S. pneumoniae* capsular saccharide from serotype 1 or 3 or 19A or 19F; 1 and 3; 1 and 19A; 1 and 19F; 3 and 19A; 3 and 19F; 19A and 19F; 1, 3 and 19A; 1, 3 and 19F, 1, 19A and 19F; 3, 19A and 19F or 1, 3, 19A and 19F conjugated to a protein carrier through cyanylation chemistry such as CDAP chemistry. In an .embodiment, 19F is conjugated to a carrier protein by CDAP chemistry. In an embodiment of the invention, the following *S. pneumoniae* capsular saccharide or group thereof is conjugated to a carrier protein by reductive amination; serotype 4, 5, 6A, 6B, 7F, 9V, 14, 18C or 23F, 4 and 5, 4 and 6A, 4 and 6B, 4 and 7F, 4 and 9V, 4 and 14, 4 and 18C, 4 and 23F, 5 and 6A, 5 and 6B, 5 and 7F, 5 and 9V, 5 and 14, 5 and 18C, 5 and 23F, 6A and 6B, 6A and 7F, 6A and 9V, 6A and 14, 6A and 18C, 6A and 23F, 6B and 7F, 6B and 9V, 6B and 14, 6B and 18C, 6B and 23F, 7F and 9V, 7F and 14, 7F and 18C, 7F and 23F, 9V and 14, 9V and 18C, 9V and 23F, 14 and 18C, 14 and 23F or 18C and 23F. In an embodiment, 23F is conjugated to a carrier protein by reductive amination chemistry.

### Doses of saccharide conjugates

In general, the immunogenic composition of the invention may comprise a dose of between 0.1 and 20µg, 1 and 5µg, 1 and 10µg or 1 and 3µg of saccharide per conjugate.

In an embodiment, the immunogenic composition of the invention contains conjugates comprising each *S. pneumoniae* capsular saccharide at a dose of between 0.1-20µg; 0.5-10µg; 0,5- 5µg or 1-3µg of saccharide. In an embodiment, capsular saccharides may be present at different dosages, for example some capsular saccharides may be present at a dose of exactly 1µg or some capsular saccharides may be present at a dose of exactly 3µg. In an embodiment, saccharides from serotypes 3, 18C and 19F (or 4, 18C and 19F) are present at a higher dose than other saccharides. In one aspect of this embodiment, serotypes 3, 18C and 19F (or 4, 18C and 19F) are present at a dose of around or exactly 3 µg whilst other saccharides in the immunogenic composition are present at a dose of around or exactly 1µg. In one embodiment serotypes 1, 5, 6B, 7F, 9V, 14 and 23F are present at a dose of around or exactly 1µg.

### Adjuvants

In one embodiment the composition does not comprise an adjuvant comprising QS21, Monophosphoryl lipid A (MPL), phospholipid and sterol, presented in the form of a liposome. Compositions comprising adjuvant comprising QS21, Monophosphoryl lipid A (MPL), phospholiopid and sterol presented in the form of a liposome are described in PCT application number WO2012/156391. In one embodiment the immunogenic composition does not comprise an adjuvant comprising QS 21. In a further embodiment the immunogenic composition does not comprise an adjuvant comprising Monophosphoryl lipid A (MPL). In one embodiment the immunogenic composition does not comprise either QS21 or MPL. QS21 is an adjuvant derived from the tree Quillaja saponaria.

In one embodiment the immunogenic composition further comprises an adjuvant.

Suitable adjuvants include, but are not limited to, aluminium salts (aluminium phosphate or aluminium hydroxide), monophosphoryl lipid A (for example 3D-MPL), saponins (for example QS21), oil in water emulsions, blebs or outer membrane vesicle preparations from Gram negative bacterial strains (such as those taught by WO02/09746), lipid A or derivatives thereof, alkyl glucosamide phosphates or combinations of two or more of these adjuvants. In one embodiment the adjuvant is aluminium phosphate. In a further embodiment the adjuvant comprises 100-750, 150-600, 200-500, 250-450, 300-400, or around 350µg aluminium as aluminium phosphate per human dose. In a further embodiment the adjuvant is aluminium hydroxide. In one embodiment the adjuvant does not comprise an oil in water adjuvant.

### Vaccines

The present invention provides a vaccine comprising an immunogenic composition comprising 26µg-45µg (for example 26µg-40µg, 28µg-35µg or around 30µg) of PhtD per human dose and 26µg-45µg (for example 26µg-40µg, 28µg-35µg or around 30µg) of detoxified pneumolysin (dPly) per human dose, for use in the treatment or prevention of disease caused by *Streptococcus pneumoniae* infection wherein the immunogenic composition further comprises *Streptococcus pneumoniae* capsular saccharide conjugates comprising a conjugated serotype 4 saccharide, a conjugated serotype 6B saccharide, a conjugated serotype 9V saccharide, a conjugated serotype 14 saccharide, a conjugated serotype 18C saccharide, a conjugated serotype 19F saccharide and a conjugated serotype 23F saccharide and a pharmaceutically acceptable excipient. Embodiments herein relating to "immunogenic compositions" of the invention are also applicable to embodiments relating to "vaccines" of the invention, and vice versa. In an embodiment, the vaccine comprises the immunogenic composition of the invention and a pharmaceutically acceptable excipient.

The vaccines of the disclosure may be administered by any suitable delivery route, such as intradermal, mucosal e.g. intranasal, oral, intramuscular or subcutaneous. Other delivery routes are well known in the art. Vaccine preparation is generally described in Vaccine Design ("The subunit and adjuvant approach" (eds Powell M.F. & Newman M.J.) (1995) Plenum Press New York).

In one aspect, the immunogenic composition of the disclosure is administered by the intramuscular delivery route. Intramuscular administration may be to the thigh or the upper arm. Injection is typically via a needle (e.g. a hypodermic needle), but needle-free injection may alternatively be used. A typical intramuscular dose is 0.5 ml.

Intradermal administration of the vaccine forms an embodiment of the present disclosure. Human skin comprises an outer "horny" cuticle, called the stratum corneum, which overlays the epidermis. Underneath this epidermis is a layer called the dermis, which in turn overlays the subcutaneous tissue. The conventional technique of intradermal injection, the "mantoux procedure", comprises steps of cleaning the skin, and then stretching with one hand, and with the bevel of a narrow gauge needle (26 to 31 gauge) facing upwards the needle is inserted at an angle of between 10 to 15°. Once the bevel of the needle is inserted, the barrel of the needle is lowered and further advanced whilst providing a slight pressure to elevate it under the skin. The liquid is then injected very slowly thereby forming a bleb or bump on the skin surface, followed by slow withdrawal of the needle.

More recently, devices that are specifically designed to administer liquid agents into or across the skin have been described, for example the devices described in WO99/34850 and EP1092444, also the jet injection devices described for example in WO01/13977, US5,480,381, US5,599,302, US5,334,144, US5,993,412, US5,649,912, US5,569,189, US5,704,911, US5,383,851, US5,893,397, US5,466,220, US5,339,163, US5,312,335, US5,503,627, US5,064,413, US5,520, 639, US4,596,556, US4,790,824, US4,941,880, US4,940,460, WO97/37705 and WO97/13537. Alternative methods of intradermal administration of the vaccine preparations may include conventional syringes and needles, or devices designed for ballistic delivery of solid vaccines (WO99/27961), or transdermal patches (WO97/48440, WO98/28037), or applied to the surface of the skin (transdermal or transcutaneous delivery WO98/20734, WO98/28037).

When the vaccines of the present disclosure are to be administered to the skin, or more specifically into the dermis, the vaccine is in a low liquid volume, particularly a volume of between about 0.05 ml and 0.2 ml.

Another suitable administration route is the subcutaneous route. Any suitable device may be used for subcutaneous delivery, for example classical needle. In one aspect of the disclosure, a needle-free jet injector service is used, such as that published in WO01/05453, WO01/05452, WO01/05451 , WO01/32243, WO01/41840, WO01/41839, WO01/47585, WO01/56637, WO01/58512, WO01/64269, WO01/78810, WO01/91835, WO01/97884, WO02/09796, WO02/34317. In another aspect of the disclosure, the device is pre-filled with the liquid vaccine formulation.
Alternatively the vaccine of the disclosure is administered intranasally. Typically, the vaccine is administered locally to the nasopharyngeal area, e.g. without being inhaled into the lungs. It is desirable to use an intranasal delivery device which delivers the vaccine formulation to the nasopharyngeal area, without or substantially without it entering the lungs. Preferred devices for intranasal administration of the vaccines according to the invention are spray devices. Suitable commercially available nasal spray devices include Accuspray™ (Becton Dickinson).

In an embodiment of the disclosure, spray devices for intranasal use are devices for which the performance of the device is not dependent upon the pressure applied by the user. These devices are known as pressure threshold devices. Liquid is released from the nozzle only when a threshold pressure is applied. These devices make it easier to achieve a spray with a regular droplet size. Pressure threshold devices suitable for use with the present invention are known in the art and are described for example in WO91/13281 and EP311 863 and EP516636, incorporated herein by reference. Such devices are commercially available from Pfeiffer GmbH and are also described in Bommer, R. Pharmaceutical Technology Europe, Sept 1999.

In another embodiment of the disclosure, intranasal devices produce droplets (measured using water as the liquid) in the range 1 to 200 µm, e.g. 10 to 120 µm. Below 10 µm there is a risk of inhalation, therefore it is desirable to have no more than about 5% of droplets below 10 µm. Droplets above 120 µm do not spread as well as smaller droplets, so it is desirable to have no more than about 5% of droplets exceeding 120 µm.

Bi-dose delivery is another embodiment of an intranasal delivery system for use with the vaccines according to the disclosure. Bi-dose devices contain two sub-doses of a single vaccine dose, one sub-dose for administration to each nostril. Generally, the two sub-doses are present in a single chamber and the construction of the device allows the efficient delivery of a single sub-dose at a time. Alternatively, a monodose device may be used for administering the vaccines according to the disclosure.

A further aspect of the invention is a method of making a vaccine of the invention comprising the steps of mixing the unconjugated *S. pneumoniae* protein with the adjuvant composition.

Although the vaccine of the disclosure may be administered as a single dose, components thereof may also be co-administered together at the same time or at different times (for instance pneumococcal saccharide conjugates could be administered separately, at the same time or 1 to 2 weeks after the administration of the any bacterial protein component of the vaccine for optimal coordination of the immune responses with respect to each other). Following an initial vaccination, subjects may receive one or several booster immunisation adequately spaced.

In one aspect of the disclosure there is provided a method of immunizing a human host against diseases caused by *streptococcus pneumoniae* infection comprising administering to the host an immunoprotective dose of the immunogenic composition or vaccine of the disclosure. In a further aspect of the invention is provided an immunogenic composition or vaccine of the invention for use in the treatment or prevention of disease caused by *Streptococcus pneumoniae* infection. In a further aspect of the disclosure there is provided of use of the immunogenic composition or vaccine of the invention in the manufacture of a medicament for the treatment or prevention of diseases caused by *Streptococcus pneumoniae* infection.

In one embodiment the host is an adult human, an adult human is a human greater than 18 years of age. In a further embodiment the host is an elderly human suitably an elderly person aged 60, 62, 65, 67, or 70 years and over. In a further embodiment the host is a younger adult (i.e. between 18 and 64 years of age), optionally a younger high-risk adult such as a younger adult working in a heath institution or a young adult with a risk factor such as cardiovascular and pulmonary disease, or diabetes. In a further embodiment the host is an infant human such as a child more than 6 months old, especially children 6 to 23 months of age. Optionally the host is a toddler such as a child between 9 months and 3 years of age.

In a further embodiment of the invention the disease caused by *Streptococcus pneumoniae* infection are selected from the group consisting of pneumonia, invasive pneumococcal disease (IPD), exacerbations of chronic obstructive pulmonary disease (COPD), otitis media, recurrent otitis media, meningitis, bacteraemia and conjunctivitis.

### General

Around" or "approximately" are defined as within 10% more or less of the given figure for the purposes of the invention.

The terms "comprising", "comprise" and "comprises" herein are intended by the inventors to be optionally substitutable with the terms "consisting of", "consist of" and "consists of", respectively, in every instance.

Embodiments herein relating to "vaccine compositions" of the invention are also applicable to embodiments relating to "immunogenic compositions" of the invention, and vice versa.

References to 'pneumococcal' and *'S.pneumoniae'* can be considered to be references to *Streptococcus pneumoniae.*

In order that this invention may be better understood, the following examples are set forth. These examples are for purposes of illustration only, and are not to be construed as limiting the scope of the invention in any manner.

### EXAMPLES

### Example 1 - production of vaccines for clinical trial study

6 Vaccines were designed:
**dPly-10-AIPO₄:** A vaccine comprising 10µg of dPly adjuvanted with Aluminium phosphate.
**dPly-30-AIPO₄:** A vaccine comprising 30µg of dPly adjuvanted with Aluminium phosphate.
**dPly/PhtD-10-AIPO₄:** A vaccine comprising 10µg of dPly and 10µg of PhtD adjuvanted with Aluminium phosphate.
**dPly/PhtD-30-AIPO₄:** A vaccine comprising 30µg of dPly and 30µg of PhtD adjuvanted with Aluminium phosphate.
**10PCV/dPly/PhtD -10-AIPO₄:** A vaccine comprising the following antigens adjuvanted to Aluminium phosphate:
   1µg capsular saccharide from serotype 1 conjugated to protein D (1-PD)
   3µg capsular saccharide from serotype 4 conjugated to protein D (4-PD)
   1µg capsular saccharide from serotype 5 conjugated to protein D (5-PD)
   1µg capsular saccharide from serotype 6B conjugated to protein D (6B-PD)
   1µg capsular saccharide from serotype 7F conjugated to protein D (7F-PD)
   1µg capsular saccharide from serotype 9V conjugated to protein D (9V-PD)
   1µg capsular saccharide from serotype 14 conjugated to protein D (14-PD)
   1µg capsular saccharide from serotype 23F conjugated to protein D (23F-PD)
   3µg capsular saccharide from serotype 18C conjugated to tetanus toxoid (18C-TT)
   1µg capsular saccharide from serotype 19F conjugated to Diphtheria Toxin (19F-DT)
   10µg dPly
   10µg PhtD
**10PCV/dPly/PhtD -30-AIPO₄:** A vaccine comprising the following antigens adjuvanted to Aluminium phosphate:
   1µg capsular saccharide from serotype 1 conjugated to protein D (1-PD)
   3µg capsular saccharide from serotype 4 conjugated to protein D (4-PD)
   1µg capsular saccharide from serotype 5 conjugated to protein D (5-PD)
   1µg capsular saccharide from serotype 6B conjugated to protein D (6B-PD)
   1µg capsular saccharide from serotype 7F conjugated to protein D (7F-PD)
   1µg capsular saccharide from serotype 9V conjugated to protein D (9V-PD)
   1µg capsular saccharide from serotype 14 conjugated to protein D (14-PD)
   1µg capsular saccharide from serotype 23F conjugated to protein D (23F-PD)
   3µg capsular saccharide from serotype 18C conjugated to tetanus toxoid (18C-TT)
   1µg capsular saccharide from serotype 19F conjugated to Diphtheria Toxin (19F-DT)
   30µg dPly
   30µg PhtD
**10PCV:** A vaccine comprising the following antigens adjuvanted to Aluminium phosphate:
   1µg capsular saccharide from serotype 1 conjugated to protein D (1-PD)
   3µg capsular saccharide from serotype 4 conjugated to protein D (4-PD)
   1µg capsular saccharide from serotype 5 conjugated to protein D (5-PD)
   1µg capsular saccharide from serotype 6B conjugated to protein D (6B-PD)
   1µg capsular saccharide from serotype 7F conjugated to protein D (7F-PD)
   1µg capsular saccharide from serotype 9V conjugated to protein D (9V-PD)
   1µg capsular saccharide from serotype 14 conjugated to protein D (14-PD)
   1µg capsular saccharide from serotype 23F conjugated to protein D (23F-PD)
   3µg capsular saccharide from serotype 18C conjugated to tetanus toxoid (18C-TT)
   1µg capsular saccharide from serotype 19F conjugated to Diphtheria Toxin (19F-DT)
   30µg dPly
   30µg PhtD

### Preparation of Antigens

**Preparation of dPly:** Pneumococcal pneumolysin was prepared and detoxified as described in WO2004/081515 and WO2006/32499 using formaldehyde detoxification.

### Expression and purification of PhtD:

**EXPRESSION OF PhtD:** The PhtD protein is a member of the pneumococcal histidine-triad (Pht) protein family characterized by the presence of histidine-triads. PhtD is a 838 aa- molecule and carries 5 histidine triads (see Medlmmune WO00/37105 SEQ ID NO: 4 for amino acid sequence and SEQ ID NO: 5 for DNA sequence). PhtD also contains a proline-rich region in the middle (amino acid position 348-380). PhtD has a 20 aa-N-terminal signal sequence. Preparation and purification of PhtD is described in WO2007/071710 (see for example Example 1b).

### Expression of protein D

Protein D was expressed as described in WO2007/071710

### Preparation of conjugates

It is well known in the art how to make purified pneumococcal polysaccharides. For the purposes of these examples the polysaccharides were made essentially as described in EP072513 or by closely-related methods. Before conjugation the polysaccharides may be sized by microfluidisation as described below.

The activation and coupling conditions are specific for each polysaccharide. These are given in Table 1. Sized polysaccharide (except for 6B and 23F) was dissolved in NaCl 2M, NaCl 0.2M or in water for injection (WFI). The optimal polysaccharide concentration was evaluated for all the serotypes. All serotypes except serotype 18C were conjugated directly to the carrier protein as detailed below.

From a 100 mg/ml stock solution in acetonitrile or acetonitrile/water (50%/50%) solution, CDAP (1-cyano-4-dimethylamino pyridinium tetrafluoroborate) (CDAP/PS ratio 0.5-1.5 mg/mg PS) was added to the polysaccharide solution. 1.5 minute later, 0.2M-0.3M NaOH was added to obtain the specific activation pH. The activation of the polysaccharide was performed at this pH for 3 minutes at 25 °C. Purified protein (protein D, CRM197 or DT) (the quantity depends on the initial PS/carrier protein ratio) was added to the activated polysaccharide and the coupling reaction was performed at the specific pH for up to 2 hour (depending upon serotype) under pH regulation. In order to quench un-reacted cyanate ester groups, a 2M glycine solution was then added to the mixture. The pH was adjusted to the quenching pH (pH 9.0). The solution was stirred for 30 minutes at 25 °C and then incubated overnight at 2-8 °C with continuous slow stirring.

### Preparation of 18C:

18C was linked to the carrier protein via a linker - Adipic acid dihydrazide (ADH) Polysaccharide serotype 18C was microfluidized before conjugation.

### Derivatization of tetanus toxoid with EDAC (1-ethyl-3-(3-dimethylaminopropyl)carbodiimide)

For derivatization of the tetanus toxoid, purified TT was diluted at 25 mg/ml in 0.2M NaCl and the ADH spacer was added in order to reach a final concentration of 0.2M. When the dissolution of the spacer was complete, the pH was adjusted to 6.2. EDAC (1-ethyl-3-(3-dimethylaminopropyl) carbodiimide) was then added to reach a final concentration of 0.02M and the mixture was stirred for 1 hour under pH regulation. The reaction of condensation was stopped by increasing pH up to 9.0 for at least 30 minutes at 25°C.

Derivatized TT was then diafiltrated (10 kDa CO membrane) in order to remove residual ADH and EDAC reagent.

TT_{AH} (tetanus toxoid conjugated to an ADH linker) bulk was finally sterile filtered until coupling step and stored at -70°C.

### Chemical coupling of TT_{AH} to PS 18C

Details of the conjugation parameters can be found in Table 1.

2 grams of microfluidized PS were diluted at the defined concentration in water and adjusted to 2M NaCl by NaCl powder addition.

CDAP solution (100 mg/ml freshly prepared in 50/50 v/v acetonitrile/WFI) was added to reach the appropriate CDAP/PS ratio.

The pH was raised up to the activation pH 9.0 by the addition of 0.3M NaOH and was stabilised at this pH until addition of TT_{AH}.

After 3 minutes, derivatized TT_{AH} (20 mg/ml in 0.2 M NaCl) was added to reach a ratio TT_{AH} /PS of 2; the pH was regulated to the coupling pH 9.0. The solution was left one hour under pH regulation.

For quenching, a 2M glycine solution, was added to the mixture PS/TT_{AH}/CDAP.

The pH was adjusted to the quenching pH (pH 9.0).

The solution was stirred for 30 min at 25 °C, and then overnight at 2-8°C with continuous slow stirring.

### Specific activation/coupling/quenching conditions of S. pneumoniae capsular saccharide-Protein D/TT/DT/PhtD/Ply conjugates

Where **"µfluid"** appears in a row header, it indicates that the saccharide was sized by microfluidisation before conjugation. Sizes of saccharides following microfluidisation are given in table 2.

**Table 1 Specific activation/coupling/guenching conditions of S. pneumoniae capsular saccharide-Protein D/TT/DT/CRM197 conjugates**

| **Serotype** | **1 µfluid** | **4 µfluid** | **5 µfluid** | **6A µfluid** | **6B** | **7F µfluid** |
|---|---|---|---|---|---|---|
| **PS conc.(mg/ml)** | 2.27 | 2.37 | 7.1 | 10 | 5.0 | 5.0 |
| **PS dissolution** | WFI | WFI | WFI | NaCl 2M | NaCl 2M | NaCl 2M |
| **Carrier conc.(mg/ml)** | 10.0 PD | 10.0 PD | 5.0 PD | 10 CRM197 | 5.0 PD | 10.0 PD |
| **Initial PROT/PS Ratio (w/w)** | 1.65/1 | 1.60/1 | 1/1 | 1.5/1 | 1.1/1 | 1.2/1 |
| **CDAP conc. (mg/mg PS)** | 0.55 | 0.55 | 0.79 | 1.0 | 0.83 | 0.75 |
| **pHₐ=pH_{c}=pH_{q}** | 9.0/9.0/9.0 | 9.5/9.5/9.0 | 9.0/9.0/9.0 | 9.5/9.5/9.0 | 9.5/9.5/9.0 | 9.5/9.5/9.0 |

| **Serotype** | **9V µfluid** | **14 µfluid** | **18C µfluid** | **19A µfluid** | **19F µfluid** | **23F** |
|---|---|---|---|---|---|---|
| **PS conc.(mg/ml)** | 5.0 | 5.0 | 4.5 | 15.0 | 9.0 | 2.38 |
| **PS dissolution** | NaCl 2M | NaCl 2M | NaCl 2M | NaCl 2M | NaCl 2M | NaCl 2M |
| **Carrier protein conc.(mg/ml)** | 10.0 | 10.0 | 20.0 (TT) | 15.0 (CRM19 7) | 20.0 (DT) | 5.0 |
| **Initial carrier protein/PS Ratio (w/w)** | 1.2/1 | 1.2/1 | 2/1 | 1.5/1 | 1.5/1 | 1/1 |
| **CDAP conc. (mg/mg PS)** | 0.50 | 0.75 | 0.75 | 1.5 | 1.5 | 0.79 |
| **pHₐ=pH_{c}=pH_{q}** | 9.5/9.5/9.0 | 9.5/9.5/9.0 | 9.0/9.0/9.0 | 9.0/9.0/9.0 | 9.0/9.0/9.0 | 9.5/9.5/9.0 |

| | | | | | | |
|---|---|---|---|---|---|---|
| Note: pHa,c,q corresponds to the pH for activation, coupling and quenching, respectively | | | | | | |

### Purification of the conjugates:

The conjugates were purified by gel filtration using a Sephacryl S400HR gel filtration column equilibrated with 0.15M NaCl (except S500HR was used as buffer for 18C and 20mM acetate containing 1.15MNaCl pH6.2 was used for 19A) to remove small molecules (including DMAP) and unconjugated saccharide and protein. Based on the different molecular sizes of the reaction components, PS-PD, PS-TT, PS-CRM197 or PS-DT conjugates are eluted first, followed by free PS, then by free protein carrier and finally DMAP and other salts (NaCl, glycine).

Fractions containing conjugates are detected by UV_{280 nm}. Fractions are pooled according to their Kd, sterile filtered (0.22µm) and stored at +2-8°C. The PS/Protein ratios in the conjugate preparations were determined.

### Example 2 - Clinical trial to study efficacy of vaccines comprising high and low doses of dPly and PhtD carried out in adults

A clinical trial was carried out using seven parallel groups;
**1**. **dPly-10-AIPO₄** group ('Ply-10' in result tables and figures): subjects receiving the dPly-10-ALPO₄ vaccine as described in example 1 (formulated with 167µg AIPO₄, 150mM NaCl and 0.78mM PO₄ buffer).
**2. dPly-30-AIPO₄** group ('Ply-30' in result tables and figures): subjects receiving the dPly-30-ALPO₄ vaccine as described in example 1 (formulated with 500 AIPO₄, 150nM NaCl and 0.78mM PO₄ buffer).
**3. dPly/PhtD-10-AIPO₄** group ('PIPh-10' in result tables and figures): subjects receiving the **dPly/PhtD-10-AIPO₄** vaccine as described in example 1 (formulated with 500 µg AIPO₄, 150mM NaCl and 0.72mM PO₄ buffer).
**4. dPly/PhtD-30-AIPO₄** group ('PIPh-30' in result tables and figures): subjects receiving the dPly/PhtD-30-AIPO₄ vaccine as described in example 1 (formulated with 500 µg AIPO₄, 150mM NaCl and 0.86mM PO₄ buffer).
**5. 10PCV/dPly/PhtD -10-AIP0₄group** ('10vPP10' in result tables and figures): subjects receiving the 10PCV/dPly/PhtD -10-AIPO₄ vaccine as described in example 1 (formulated with 500 µg AIPO₄, 150mM NaCl and 0.72mM PO₄ buffer).
**6**. **10PCV/dPly/PhtD** -**30-AIPO₄** group ('10vPP30' in result tables and figures): subjects receiving the 10PCV/dPly/PhtD -30-AIPO₄ vaccine as described in example 1 (formulated with 500 µg AIPO₄, 150mM NaCl and 0.86 mM PO₄ buffer).
**7. 23PPV Comparator** group ('23PPV' in result tables and figures): subjects receiving one dose of a commercially available licensed 23-valent c Polysacharide Vaccine (23PPV) named Pneumovax 23™ at Day 0 (Month 0) followed by placebo at Day 60 (Month 2).

Subjects in all groups received 2 doses of the respective study vaccines, one at Day 0 (Month 0) and one at Day 60 (Month 2); the 23PPV Comparator group received the licensed 23PPV vaccine at Day 0 (Month 0) and a placebo at Day 60 (Month 2). Vaccines were administered as an intramuscular injection in the non dominant deltoid. Subjects were healthy males or females between, and including, 18 and 40 years old at the time of the first vaccination.

### Safety/reactogenicity methods

Incidence of solicited and/or unsolicited local and general AEs (adverse events) during the 31-day post-vaccination follow-up period was calculated with 95% Cl (confidence interval), after each vaccine dose and overall, according to the type of symptom, the intensity and relationship to vaccination. Incidence of each local and each general solicited symptom reported during the 7-day post-vaccination follow-up period was calculated with 95% Cl, after each vaccine dose and overall, according to the type of symptom, the intensity and relationship to vaccination. The percentages of subjects/doses with an unsolicited symptom reported within the 31-day post-vaccination follow-up period were summarized according to the Medical Dictionary for Regulatory Activities (MedDRA), with 95% Cl (confidence interval) according to the intensity and relationship to vaccination. Prevalence of concomitant antipyretic/medication during the 7-day post-vaccination follow-up period and during the 31-day post-vaccination follow-up period was computed with 95% Cl, after each vaccine dose and overall. Serious adverse events (SAEs) and withdrawals due to adverse event(s) reported during the entire study period were described in detail. For each haematology and biochemistry parameter, the number and percentage of subjects with levels below, within and above normal ranges were tabulated per study group, at each assessed time point. The grading and the change in grading from baseline (baseline: day 0 assessment) was summarized per study group, at each assessed time point. For each urine parameter, the grading and the change in grading from baseline (baseline: day 0 assessment) was summarized per study group, at each assessed time point. pH quantified in urinalysis over time was displayed per study group.

### Laboratory assays and time points for immunogenicity evaluation

For all subjects, an approximately 20 mL sample of whole venous blood was collected for serology at Visit 1 [Pre], Visit 4 [(PI(D30) (post dose I at day 30)] and Visit 8 [(PII(D90) (post dose II at day 90)]. After blood centrifugation and serum separation, samples were stored at - 20°C until collection by the sponsor. The aliquots of serum (approximately 10 mL) were sent to GSK Biologicals for the serological tests described in the following paragraphs.
Opsonophagocytic activity for antibodies against each of the 10 pneumococcal serotypes (antibodies to 1, 4, 5, 6B, 7F, 9V, 14, 18C, 19F, 23F) was measured by a killing-assay using a HL 60 cell line. The results were presented as the dilution of serum (opsonic titre) able to sustain 50% killing of live pneumococci under the assay conditions. The cut-off of the assay was an opsonic titre of 8.
Anti-dPly and anti-PhtD antibodies were quantified using an ELISA (enzyme linked immunosorbent assay).

A sample ELISA protocol is described below:
Microtiter plates are coated with purified serotype specific pneumococcal PS (polysaccharide) and mixed with methylated human serum albumin (0.5 to 7.5 µg/ml, depending on the PS). Serum samples are diluted in adsorption buffer (phosphate-buffered saline [PBS], 10% fetal calf serum [FCS], 0.1% polyvinyl alcohol) containing 10 µg/ml of the CWPS (capsular wall polysaccharide) (SSI) and 2 µg/ml of PS of serotype 22F (ATCC) and are incubated overnight at 4°C. The 89-SF reference serum sample (FDA) (89-SF is the number of the reference serum calibrated by the World Health Organisation) is diluted in the same adsorption buffer but with 10 µg/ml of CWPS only, and the mixture is incubated overnight at 4°C. An internal reference serum sample, calibrated against standard serum sample 89-SF, is included in every plate. Unlike the working standard serum sample, in line with the WHO recommendations, the 89-SF reference serum sample is preadsorbed only with CWPS and not with 22F because the values for the anti-PS concentrations of the 89-SF reference serum sample are assigned without the use of 22F PS. Bound antibodies are detected by using alkaline phosphatase-conjugated anti-human IgG.

A sample OPA (opsonophagocytosis assay) is described below:
The OPA assay is performed as follows: serum samples are heated for 40 min at 56 °C to inactivate any remaining endogenous complement. Twenty-five microlitres aliquots of each 1:2 diluted serum sample is two-fold serially diluted in 25 µl OPA buffer (Hank's Balanced salt solution or HBSS -14.4% inactivated FBS (fetal bovine serum)) per well of a 96-well round bottom microtitre plate (Nalge Nunc International, Rochester, NY, USA). Two positive control samples with known opsonophagocytic titre for the specific pneumococcal serotype are run on each assay plate to determine the assay validity. In one column no serum sample is added to estimate the average CFU (colony forming units) which corresponds to 0% killing in the absence of opsonophagocytic antibodies. Subsequently, 25 µl of a mixture of activated HL-60 cells (1×107 cells/ml), freshly thawed pneumococcal working seed and freshly thawed baby rabbit complement in an e.g. 4/2/2.82 ratio (volume/volume/volume (v/v/v); the ratio may vary depending on the lot of complement) are added to the diluted sera to yield a final volume of 50µl. As a result, final serial dilutions of serum samples are 1:8-1:1024. The assay plate is incubated for 2 h at 37 °C with orbital shaking (210 rpm) to promote the phagocytic process. The reaction is stopped by laying the microplate on ice for at least 1 min. A 20µl aliquot of each well of the plate is then transferred into the corresponding well of a 96-well flat bottom microplate (Nalge Nunc International) and 100µl of Todd-Hewitt Broth-0.9% agar is added to each well. After overnight incubation at 37 °C and 5% CO₂, pneumococcal colonies appearing in the agar are counted using an automated image analysis system (KS 400, Zeiss, Oberkochen,

Germany). Eight wells without serum sample are used as bacterial controls to determine the number of pneumococci per well. The mean number of CFU of the control wells is determined and used for the calculation of the killing activity for each serum sample. The OPA titre for the serum samples is determined by the reciprocal dilution of serum able to facilitate 50% killing of the pneumococci. The opsonophagocytic titre is calculated by using a 4-parameter curve fit analysis. Each sample titre was adjusted through an adjustment factor based on the average ratio of actually measured titre/known titre of two positive control samples included in each assay. The cut-off OPA titre for all serotypes is a reciprocal serum dilution of 8. Serum samples with OPA titres <8 are assigned a titre of 4 for the purpose of data analysis.

A sample anti-PhtD and anti-Ply ELISA is described below
Plates are coated overnight at 4°C with 100 µl per well of 2 µg/ml of PhtD (1021 µg/ml) in PBS, 3 µg/ml of Ply (3675µg/ml) in TR034 (Na 50mM ph 9.5).

The plates are then washed three times with TR 112 (Na2K 39.65 mM - NaCl 1.73 M - Polysorbate 20 0.275% 5P/V° pH 6.6-6.8).

After the first wash, the plates are saturated for 1 hour at RT with 200µl/wells of diluant buffer (PBS without Ca and Mg -BSA 1% for PhtD) or BSA 5% for Ply - Polysorbate 20 0.1% - ProClin 300™ 0.2% pH 7.0)with shaking.

After a second wash (same buffer) 100µl of sera are added to microwells at the dilution 1/100, 1/1000 and 1/10000 in the diluant buffer.

The plates are incubated for 1 hour at room temperature with shaking. After 5 washes, the plates are incubated with a goat anti -human IgG Fc antibody conjugated to peroxydase , diluted 1/20000 for PhtD and 1/12500 for Ply (100 µl per well) at room temperature for 1 hour with shaking. Plates are washed as above and 100µl of the TMB substrate conjugate is added to each well for 10 min in darkness.

The reaction is stopped by addition of H2SO4 (0.18M) 100 µl and the absorbance is read at 450nm and 650 nm.

### Immunogenicity

Immunogenicity analysis was performed on the according to protocol (ATP) cohort for immunogenicity. No second analysis on the Total vaccinated cohort was performed as less than 5% of subjects were excluded from the ATP cohort for immunogenicity. Sera were analysed for the presence of antibodies against dPly, PhtD, and *Streptococcus pneumoniae* capsular saccharide serotypes 1, 4, 5, 6B, 7F, 9V, 14, 18C, 19F and 23F.

The seropositivity rates and GMCs for anti-Ply antibodies are described in table 1 below, these data are illustrated in Figure 1:

| **Table 1: Seropositivity rates and GMCs for anti-Ply antibodies (ATP cohort for immunogenicity)** | | | | | | | | | | |
|---|---|---|---|---|---|---|---|---|---|---|
| | | | | **≥ 599 LU/mL** | | | | **GMC** | | |
| | | | | | | **95% CI** | | | **95% CI** | |
| **Antibody** | **Group** | **Timing** | **N** | **n** | **%** | **LL** | **UL** | **value** | **LL** | **UL** |
| Anti-dPly | Ply-10 | PRE | 10 | 10 | 100 | 69.2 | 100 | 9197.6 | 5993.2 | 14115.4 |
| | | PI(D30) | 10 | 10 | 100 | 69.2 | 100 | 22981.9 | 15630.8 | 33790.3 |
| | | PII(D90) | 10 | 10 | 100 | 69.2 | 100 | 47009.8 | 30960.2 | 71379.4 |
| | Ply-30 | PRE | 24 | 24 | 100 | 85.8 | 100 | 15332.5 | 10750.7 | 21866.9 |
| | | PI(D30) | 24 | 24 | 100 | 85.8 | 100 | 55848.3 | 37181.4 | 83886.8 |
| | | PII(D90) | 21 | 21 | 100 | 83.9 | 100 | 87300.2 | 58201.8 | 130947 |
| | PIPh-10 | PRE | 24 | 24 | 100 | 85.8 | 100 | 13060.2 | 9621.1 | 17728.5 |
| | | PI(D30) | 24 | 24 | 100 | 85.8 | 100 | 43980.5 | 31936 | 60567.7 |
| | | PII(D90) | 23 | 23 | 100 | 85.2 | 100 | 63998.6 | 48405.7 | 84614.4 |
| | PIPh-30 | PRE | 23 | 23 | 100 | 85.2 | 100 | 14904.9 | 11128.3 | 19963 |
| | | PI(D30) | 24 | 24 | 100 | 85.8 | 100 | 90445 | 62893.9 | 130065 |
| | | PII(D90) | 24 | 24 | 100 | 85.8 | 100 | 143923 | 106150 | 195138 |
| | 10vPP10 | PRE | 24 | 24 | 100 | 85.8 | 100 | 12238.7 | 8648.1 | 17319.9 |
| | | PI(D30) | 24 | 24 | 100 | 85.8 | 100 | 23276.4 | 16926.3 | 32008.9 |
| | | PII(D90) | 24 | 24 | 100 | 85.8 | 100 | 28560.3 | 21331.4 | 38239.1 |
| | 10vPP30 | PRE | 24 | 24 | 100 | 85.8 | 100 | 19987.5 | 15149.1 | 26371.2 |
| | | PI(D30) | 24 | 24 | 100 | 85.8 | 100 | 61346.1 | 46049.1 | 81724.5 |
| | | PII(D90) | 21 | 21 | 100 | 83.9 | 100 | 73596.9 | 52250.3 | 103665 |
| | 23PPV | PRE | 24 | 24 | 100 | 85.8 | 100 | 18661 | 12726.5 | 27363 |
| | | PI(D30) | 24 | 24 | 100 | 85.8 | 100 | 18685.4 | 12612.1 | 27683.4 |
| | | PII(D90) | 23 | 23 | 100 | 85.2 | 100 | 16573.3 | 11007.1 | 24954.2 |

| | | | | | | | | | | |
|---|---|---|---|---|---|---|---|---|---|---|
| A seropositive subject was defined as a subject with antibody concentration ≥ 0.05 µg/mL. GMC = geometric mean antibody concentration calculated on all subjects N = number of subjects with available results n/% = number/percentage of subjects with concentration within the specified range 95% Cl = 95% confidence interval; LL = lower limit, UL = upper limit PRE = pre-vaccination blood sample PI(D30) = blood sample taken post dose I at day 30 PII (D90)= blood sample taken post dose II at day 90 | | | | | | | | | | |

An increase in the anti-dPly antibodies GMCs was observed following each vaccination in all groups, except for the 23PPV group that did not receive dPly. The anti-dPly antibody GMCs tended to be higher in the PIPh-10 and PIPh-30 groups compared to the Ply-10 and Ply-30 groups respectively this is unexpected as one might expect antigen interference between the PhtD and dPly. The highest anti-dPly antibody GMCs were observed for the formulation containing 30µg of dPly and PhtD proteins (i.e PIPh-30 and 10vPP30 groups).

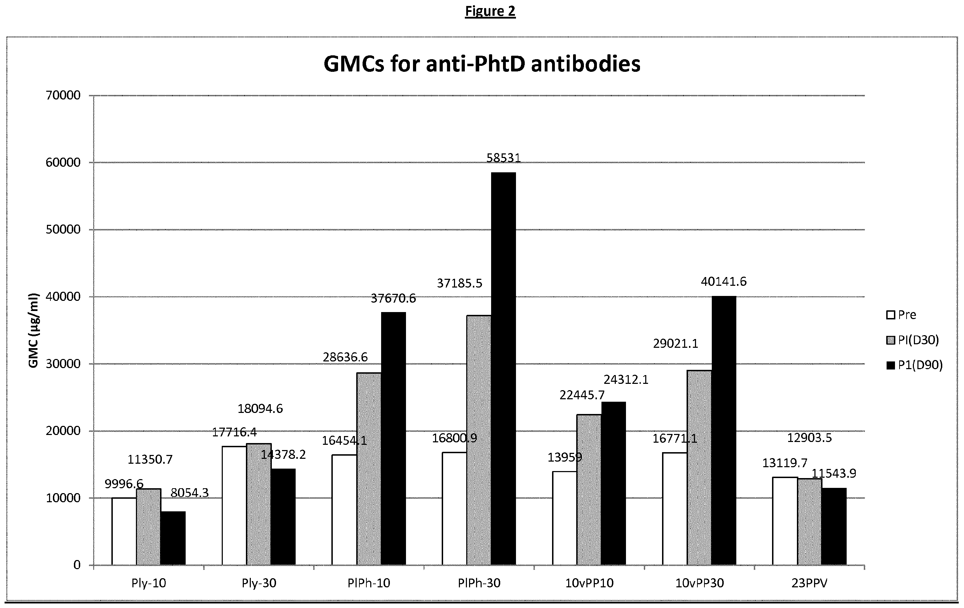

The seropositivity rates and GMCs for anti-PhtD antibodies (ATP cohort of immunogenicity) are described in table 2 below and illustrated in Figure 2:

| **Table 2: Seropositivity rates and GMCs for anti-PhtD antibodies (ATP cohort for immunogenicity)** | | | | | | | | | | |
|---|---|---|---|---|---|---|---|---|---|---|
| | | | | **≥391 LU/mL** | | | | **GMC** | | |
| | | | | | | **95% CI** | | | **95% CI** | |
| **Antibody** | **Group** | **Timing** | **N** | **n** | **%** | **LL** | **UL** | **value** | **LL** | **UL** |
| Anti-PhtD | Ply-10 | PRE | 10 | 10 | 100 | 69.2 | 100 | 9996.6 | 6608.7 | 15121.2 |
| | | PI(D30) | 10 | 10 | 100 | 69.2 | 100 | 11350.7 | 6928.5 | 18595.5 |
| | | PII(D90) | 10 | 10 | 100 | 69.2 | 100 | 8054.3 | 5005.4 | 12960.5 |
| | Ply-30 | PRE | 24 | 24 | 100 | 85.8 | 100 | 17716.4 | 13160.7 | 23849.1 |
| | | PI(D30) | 24 | 24 | 100 | 85.8 | 100 | 18094.6 | 13270.3 | 24672.7 |
| | | PII(D90) | 21 | 21 | 100 | 83.9 | 100 | 14378.2 | 10119.6 | 20428.9 |
| | PIPh-10 | PRE | 24 | 24 | 100 | 85.8 | 100 | 16454.1 | 11491.1 | 23560.6 |
| | | PI(D30) | 24 | 24 | 100 | 85.8 | 100 | 28636.6 | 20777.7 | 39468.1 |
| | | PII(D90) | 23 | 23 | 100 | 85.2 | 100 | 37670.6 | 29311.2 | 48414 |
| | PIPh-30 | PRE | 23 | 23 | 100 | 85.2 | 100 | 16800.9 | 13270.5 | 21270.5 |
| | | PI(D30) | 24 | 24 | 100 | 85.8 | 100 | 37185.5 | 28847.4 | 47933.7 |
| | | PII(D90) | 24 | 24 | 100 | 85.8 | 100 | 58531 | 49363.7 | 69400.8 |
| | 10vPP10 | PRE | 24 | 24 | 100 | 85.8 | 100 | 13959 | 10649.8 | 18296.6 |
| | | PI(D30) | 24 | 24 | 100 | 85.8 | 100 | 22445.7 | 17793.5 | 28314.3 |
| | | PII(D90) | 24 | 24 | 100 | 85.8 | 100 | 24312.1 | 18800.7 | 31439.3 |
| | 10vPP30 | PRE | 24 | 24 | 100 | 85.8 | 100 | 16771.1 | 11893.3 | 23649.5 |
| | | PI(D30) | 24 | 24 | 100 | 85.8 | 100 | 29021.1 | 20873.4 | 40349 |
| | | PII(D90) | 21 | 21 | 100 | 83.9 | 100 | 40141.6 | 29095.1 | 55382.1 |
| | 23PPV | PRE | 24 | 24 | 100 | 85.8 | 100 | 13119.7 | 10035.1 | 17152.5 |
| | | PI(D30) | 24 | 24 | 100 | 85.8 | 100 | 12903.5 | 9567.7 | 17402.4 |
| | | PII(D90) | 23 | 23 | 100 | 85.2 | 100 | 11543.9 | 8457.8 | 15756.1 |

An increase in the anti-PhtD antibody GMCs was observed with each dose except for the Ply-10, Ply-30 and 23PPV groups that did not receive PhtD. Anti-PhtD antibody GMCs post-dose 1 and post dose-2 tended to be lower in the 10vPP10 and 10vPP30 groups compared to the PIPh-10 and PIPh-30 groups, respectively. The highest anti-PhtD antibody GMCs were observed for the formulations containing 30µg of dPly and PhtD proteins (i.e PIPh-30 and 10vPP30 groups).

Seropositivity rates and GMTs for serotype 1 antibodies are described in Table 3 below, these data are illustrated in Figure 3:

| **Table 3: Seropositivity rates and GMTs for anti-PS1 antibodies (ATP cohort for immunogenicity)** | | | | | | | | | | |
|---|---|---|---|---|---|---|---|---|---|---|
| | | | | | | **95% CI** | | | **95% CI** | |
| **Antibody** | **Group** | **Timing** | **N** | **n** | **%** | **LL** | **UL** | **value** | **LL** | **UL** |
| OPSONO-1 | Ply-10 | PRE | 10 | 3 | 30 | 6.7 | 65.2 | 7.8 | 3.6 | 16.8 |
| | | PI(D30) | 10 | 5 | 50 | 18.7 | 81.3 | 8.9 | 4.8 | 16.5 |
| | | PII(D90) | 10 | 6 | 60 | 26.2 | 87.8 | 16.5 | 5.8 | 46.7 |
| | Ply-30 | PRE | 21 | 6 | 28.6 | 11.3 | 52.2 | 9.4 | 4.8 | 18.3 |
| | | PI(D30) | 22 | 13 | 59.1 | 36.4 | 79.3 | 13.2 | 7.7 | 22.7 |
| | | PII(D90) | 21 | 11 | 52.4 | 29.8 | 74.3 | 11.8 | 6.9 | 20 |
| | PIPh-10 | PRE | 23 | 3 | 13 | 2.8 | 33.6 | 6.5 | 3.5 | 12 |
| | | PI(D30) | 23 | 5 | 21.7 | 7.5 | 43.7 | 7.5 | 4 | 14.3 |
| | | PII(D90) | 22 | 6 | 27.3 | 10.7 | 50.2 | 8.4 | 4.4 | 16.4 |
| | PIPh-30 | PRE | 20 | 8 | 40 | 19.1 | 63.9 | 8.5 | 5.3 | 13.4 |
| | | PI(D30) | 24 | 9 | 37.5 | 18.8 | 59.4 | 7.8 | 5.4 | 11.3 |
| | | PII(D90) | 24 | 6 | 25 | 9.8 | 46.7 | 6.5 | 4.5 | 9.5 |
| | 10vPP10 | PRE | 21 | 5 | 23.8 | 8.2 | 47.2 | 7.4 | 4.2 | 13 |
| | | PI(D30) | 24 | 23 | 95.8 | 78.9 | 99.9 | 117.1 | 67.7 | 202.5 |
| | | PII(D90) | 24 | 23 | 95.8 | 78.9 | 99.9 | 139.4 | 83.8 | 231.9 |
| | 10vPP30 | PRE | 22 | 4 | 18.2 | 5.2 | 40.3 | 5.7 | 4 | 8 |
| | | PI(D30) | 23 | 22 | 95.7 | 78.1 | 99.9 | 91.9 | 56 | 150.9 |
| | | PII(D90) | 21 | 21 | 100 | 83.9 | 100 | 147.3 | 98.7 | 219.8 |
| | 23PPV | PRE | 21 | 2 | 9.5 | 1.2 | 30.4 | 4.6 | 3.8 | 5.7 |
| | | PI(D30) | 24 | 24 | 100 | 85.8 | 100 | 391.5 | 193.7 | 791.4 |
| | | PII(D90) | 23 | 22 | 95.7 | 78.1 | 99.9 | 268.6 | 129 | 559.2 |

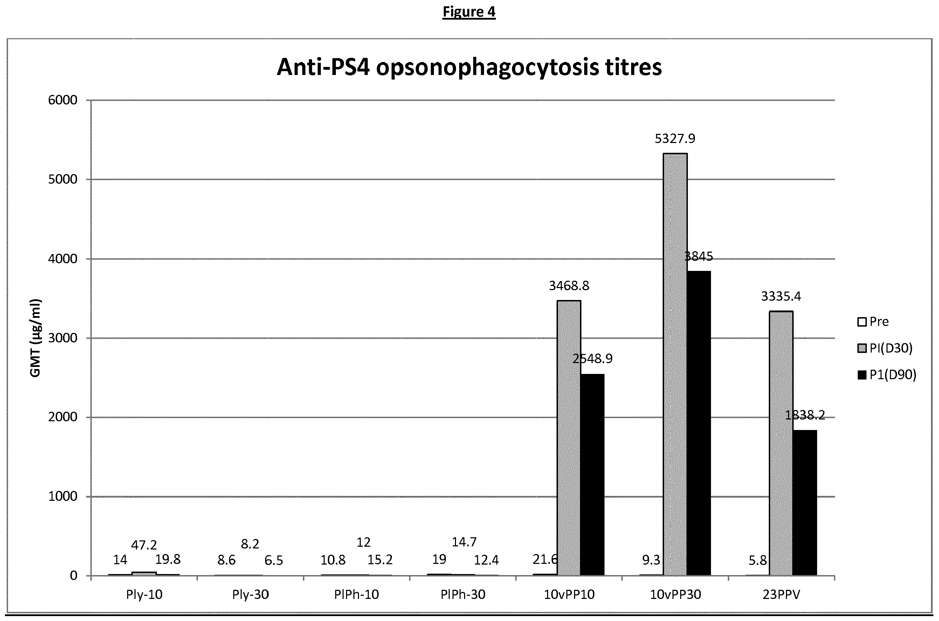

Seropositivity rates and OPA (opsonophagocytosis) GMTs for serotype 4 antibodies are described in Table 4 below, these data are illustrated in Figure 4:

| **Table 4: Seropositivity rates and GMTs for anti-PS4 antibodies (ATP cohort for immunogenicity)** | | | | | | | | | | |
|---|---|---|---|---|---|---|---|---|---|---|
| | | | | | | **95% CI** | | | **95% CI** | |
| **Antibody** | **Group** | **Timing** | **N** | **n** | **%** | **LL** | **UL** | **value** | **LL** | **UL** |
| OPSONO-4 | Ply-10 | PRE | 3 | 1 | 33.3 | 0.8 | 90.6 | 14 | 0.1 | 3085.3 |
| | | PI(D30) | 7 | 4 | 57.1 | 18.4 | 90.1 | 47.2 | 5.2 | 428.2 |
| | | PII(D90) | 8 | 3 | 37.5 | 8.5 | 75.5 | 19.8 | 3.1 | 126.1 |
| | Ply-30 | PRE | 17 | 3 | 17.6 | 3.8 | 43.4 | 8.6 | 3.6 | 20.5 |
| | | PI(D30) | 18 | 3 | 16.7 | 3.6 | 41.4 | 8.2 | 3.6 | 19 |
| | | PII(D90) | 21 | 2 | 9.5 | 1.2 | 30.4 | 6.5 | 3.2 | 13 |
| | PlPh-10 | PRE | 18 | 4 | 22.2 | 6.4 | 47.6 | 10.8 | 4.1 | 28.4 |
| | | PI(D30) | 21 | 5 | 23.8 | 8.2 | 47.2 | 12 | 4.8 | 30.3 |
| | | PII(D90) | 22 | 6 | 27.3 | 10.7 | 50.2 | 15.2 | 5.6 | 41.6 |
| | PlPh-30 | PRE | 15 | 5 | 33.3 | 11.8 | 61.6 | 19 | 5.4 | 67.6 |
| | | PI(D30) | 20 | 5 | 25 | 8.7 | 49.1 | 14.7 | 5 | 43.4 |
| | | PII(D90) | 22 | 5 | 22.7 | 7.8 | 45.4 | 12.4 | 4.8 | 32.2 |
| | 10vPP10 | PRE | 15 | 6 | 40 | 16.3 | 67.7 | 21.6 | 6.2 | 75.5 |
| | | PI(D30) | 24 | 24 | 100 | 85.8 | 100 | 3468.8 | 2091.4 | 5753.3 |
| | | PII(D90) | 23 | 23 | 100 | 85.2 | 100 | 2548.9 | 1735.9 | 3742.7 |
| | 10vPP30 | PRE | 17 | 3 | 17.6 | 3.8 | 43.4 | 9.3 | 3.5 | 24.8 |
| | | PI(D30) | 24 | 24 | 100 | 85.8 | 100 | 5327.9 | 3480.7 | 8155.3 |
| | | PII(D90) | 21 | 21 | 100 | 83.9 | 100 | 3845 | 2573.6 | 5744.6 |
| | 23PPV | PRE | 16 | 1 | 6.3 | 0.2 | 30.2 | 5.8 | 2.6 | 12.7 |
| | | PI(D30) | 24 | 24 | 100 | 85.8 | 100 | 3335.4 | 1902.8 | 5846.6 |
| | | PII(D90) | 23 | 22 | 95.7 | 78.1 | 99.9 | 1838.2 | 855.4 | 3950.4 |

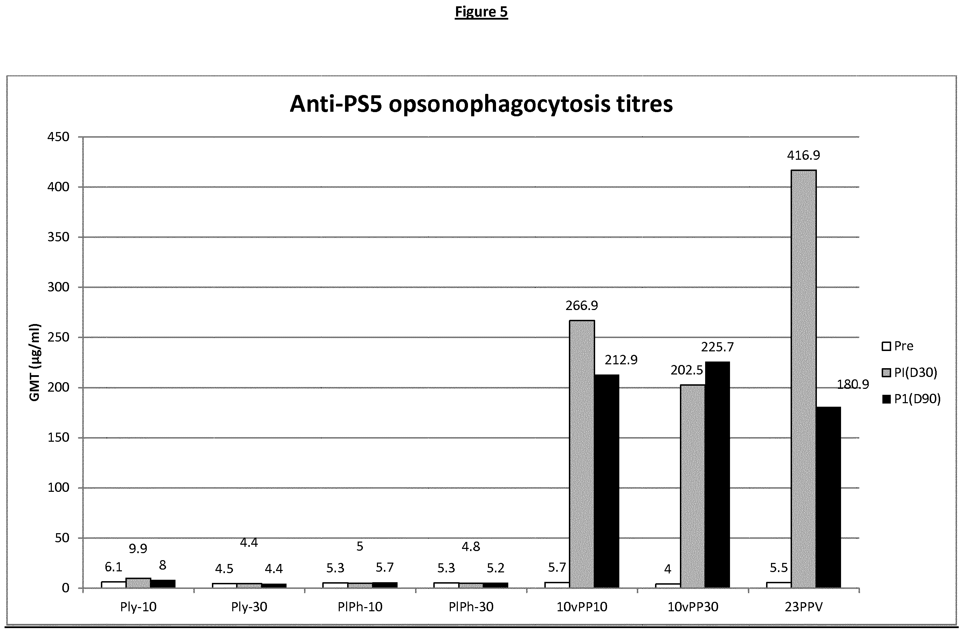

Seropositivity rates and OPA (opsonophagocytosis) GMTs for serotype 5 antibodies are described in Table 5 below, these data are illustrated in Figure 5:

| **Table 5: Seropositivity rates and GMTs for anti-PS5 antibodies (ATP cohort for immunogenicity)** | | | | | | | | | | |
|---|---|---|---|---|---|---|---|---|---|---|
| | | | | **≥ 8** | | | | **GMT** | | |
| | | | | | | **95% CI** | | | **95% CI** | |
| **Antibody** | **Group** | **Timing** | **N** | **n** | **%** | **LL** | **UL** | **value** | **LL** | **UL** |
| OPSONO-5 | Ply-10 | PRE | 7 | 1 | 14.3 | 0.4 | 57.9 | 6.1 | 2.2 | 17.1 |
| | | PI(D30) | 10 | 4 | 40 | 12.2 | 73.8 | 9.9 | 4 | 24.2 |
| | | PII(D90) | 10 | 4 | 40 | 12.2 | 73.8 | 8 | 3.8 | 16.9 |
| | Ply-30 | PRE | 22 | 2 | 9.1 | 1.1 | 29.2 | 4.5 | 3.8 | 5.4 |
| | | PI(D30) | 21 | 1 | 4.8 | 0.1 | 23.8 | 4.4 | 3.6 | 5.4 |
| | | PII(D90) | 21 | 2 | 9.5 | 1.2 | 30.4 | 4.4 | 3.8 | 5 |
| | PlPh-10 | PRE | 23 | 3 | 13 | 2.8 | 33.6 | 5.3 | 3.7 | 7.5 |
| | | PI(D30) | 23 | 2 | 8.7 | 1.1 | 28 | 5 | 3.6 | 7.1 |
| | | PII(D90) | 23 | 4 | 17.4 | 5 | 38.8 | 5.7 | 4 | 8.2 |
| | PlPh-30 | PRE | 23 | 5 | 21.7 | 7.5 | 43.7 | 5.3 | 4.2 | 6.7 |
| | | PI(D30) | 23 | 3 | 13 | 2.8 | 33.6 | 4.8 | 3.9 | 6 |
| | | PII(D90) | 24 | 5 | 20.8 | 7.1 | 42.2 | 5.2 | 4.1 | 6.5 |
| | 10vPP10 | PRE | 24 | 4 | 16.7 | 4.7 | 37.4 | 5.7 | 4 | 8.1 |
| | | PI(D30) | 23 | 22 | 95.7 | 78.1 | 99.9 | 266.9 | 125 | 569.8 |
| | | PII(D90) | 24 | 23 | 95.8 | 78.9 | 99.9 | 212.9 | 111.2 | 407.6 |
| | 10vPP30 | PRE | 23 | 0 | 0 | 0 | 14.8 | 4 | 4 | 4 |
| | | PI(D30) | 24 | 23 | 95.8 | 78.9 | 99.9 | 202.5 | 116.2 | 352.8 |
| | | PII(D90) | 21 | 21 | 100 | 83.9 | 100 | 225.7 | 139.7 | 364.8 |
| | 23PPV | PRE | 23 | 2 | 8.7 | 1.1 | 28 | 5.5 | 3.5 | 8.7 |
| | | PI(D30) | 24 | 24 | 100 | 85.8 | 100 | 416.9 | 185.6 | 936.8 |
| | | PII(D90) | 23 | 21 | 91.3 | 72 | 98.9 | 180.9 | 70.3 | 465.7 |

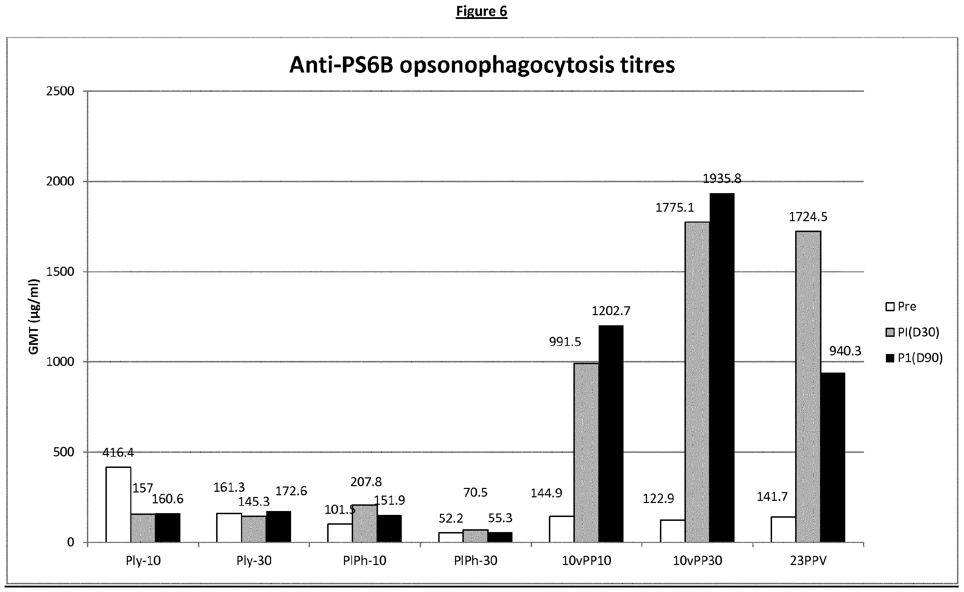

Seropositivity rates and OPA (opsonophagocytosis) GMTs for serotype 6B antibodies are described in Table 6 below, these data are illustrated in Figure 6:

| **Table 6: Seropositivity rates and GMTs for anti-PS6B antibodies (ATP cohort for immunogenicity)** | | | | | | | | | | |
|---|---|---|---|---|---|---|---|---|---|---|
| | | | | **≥ 8** | | | | **GMT** | | |
| | | | | | | **95% CI** | | | **95% CI** | |
| **Antibody** | **Group** | **Timing** | **N** | **n** | **%** | **LL** | **UL** | **value** | **LL** | **UL** |
| OPSONO-6B | Ply-10 | PRE | 8 | 7 | 87.5 | 47.3 | 99.7 | 416.4 | 75.4 | 2300.9 |
| | | PI(D30) | 10 | 7 | 70 | 34.8 | 93.3 | 157 | 23.6 | 1043.7 |
| | | PII(D90) | 10 | 7 | 70 | 34.8 | 93.3 | 160.6 | 24.7 | 1043 |
| | Ply-30 | PRE | 14 | 10 | 71.4 | 41.9 | 91.6 | 161.3 | 37.7 | 689.9 |
| | | PI(D30) | 22 | 16 | 72.7 | 49.8 | 89.3 | 145.3 | 50 | 422.3 |
| | | PII(D90) | 21 | 15 | 71.4 | 47.8 | 88.7 | 172.6 | 52.4 | 568.6 |
| | PlPh-10 | PRE | 11 | 7 | 63.6 | 30.8 | 89.1 | 101.5 | 16.9 | 608 |
| | | PI(D30) | 21 | 16 | 76.2 | 52.8 | 91.8 | 207.8 | 69.8 | 618.9 |
| | | PII(D90) | 23 | 16 | 69.6 | 47.1 | 86.8 | 151.9 | 50.1 | 460.8 |
| | PlPh-30 | PRE | 10 | 5 | 50 | 18.7 | 81.3 | 52.2 | 7.3 | 375 |
| | | PI(D30) | 22 | 12 | 54.5 | 32.2 | 75.6 | 70.5 | 21 | 236.1 |
| | | PII(D90) | 23 | 11 | 47.8 | 26.8 | 69.4 | 55.3 | 16.1 | 190.3 |
| | 10vPP10 | PRE | 13 | 10 | 76.9 | 46.2 | 95 | 144.9 | 37.6 | 558.8 |
| | | PI(D30) | 24 | 24 | 100 | 85.8 | 100 | 991.5 | 583.3 | 1685.2 |
| | | PII(D90) | 24 | 24 | 100 | 85.8 | 100 | 1202.7 | 792.4 | 1825.3 |
| | 10vPP30 | PRE | 12 | 8 | 66.7 | 34.9 | 90.1 | 122.9 | 23.3 | 648.5 |
| | | PI(D30) | 24 | 24 | 100 | 85.8 | 100 | 1775.1 | 961.8 | 3276.3 |
| | | PII(D90) | 21 | 21 | 100 | 83.9 | 100 | 1935.8 | 1193.7 | 3139.2 |
| | 23PPV | PRE | 10 | 7 | 70 | 34.8 | 93.3 | 141.7 | 22.4 | 897.5 |
| | | PI(D30) | 23 | 22 | 95.7 | 78.1 | 99.9 | 1724.5 | 837.6 | 3550.7 |
| | | PII(D90) | 23 | 21 | 91.3 | 72 | 98.9 | 940.3 | 399.2 | 2214.7 |

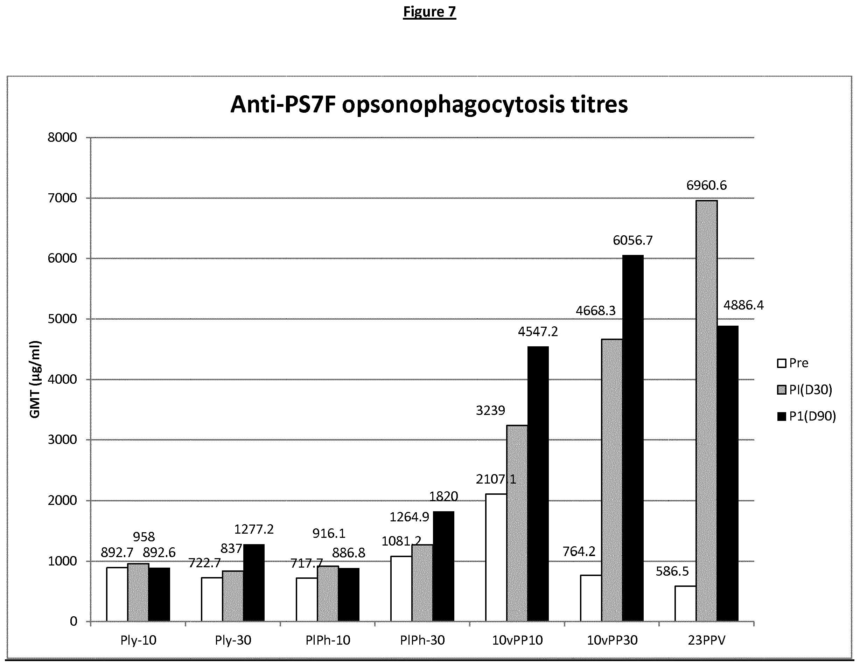

Seropositivity rates and OPA (opsonophagocytosis) GMTs for serotype 7F antibodies are described in Table 7 below, these data are illustrated in Figure 7:

| **Table7: Seropositivity rates and GMTs for anti-PS7Fantibodies (ATP cohort for immunogenicity)** | | | | | | | | | | |
|---|---|---|---|---|---|---|---|---|---|---|
| | | | | ≥ **8** | | | | **GMT** | | |
| | | | | | | **95% CI** | | | **95% CI** | |
| **Antibody** | **Group** | **Timing** | **N** | **n** | **%** | **LL** | **UL** | **value** | **LL** | **UL** |
| OPSONO-7F | Ply-10 | PRE | 8 | 8 | 100 | 63.1 | 100 | 892.7 | 407.2 | 1957.3 |
| | | PI(D30) | 9 | 9 | 100 | 66.4 | 100 | 958 | 453.6 | 2023.3 |
| | | PII(D90) | 10 | 10 | 100 | 69.2 | 100 | 892.6 | 443.1 | 1798 |
| | Ply-30 | PRE | 17 | 15 | 88.2 | 63.6 | 98.5 | 722.7 | 229.1 | 2279.9 |
| | | PI(D30) | 24 | 22 | 91.7 | 73 | 99 | 837 | 380 | 1843.6 |
| | | PII(D90) | 20 | 19 | 95 | 75.1 | 99.9 | 1277.2 | 567.4 | 2875.2 |
| | PlPh-10 | PRE | 14 | 12 | 85.7 | 57.2 | 98.2 | 717.7 | 179.5 | 2869.1 |
| | | PI(D30) | 24 | 23 | 95.8 | 78.9 | 99.9 | 916.1 | 465.2 | 1804.2 |
| | | PII(D90) | 23 | 21 | 91.3 | 72 | 98.9 | 886.8 | 376.7 | 2087.3 |
| | PlPh-30 | PRE | 17 | 16 | 94.1 | 71.3 | 99.9 | 1081.2 | 468 | 2497.9 |
| | | PI(D30) | 24 | 24 | 100 | 85.8 | 100 | 1264.9 | 895.7 | 1786.3 |
| | | PII(D90) | 24 | 24 | 100 | 85.8 | 100 | 1820 | 1218.9 | 2717.5 |
| | 10vPP10 | PRE | 11 | 11 | 100 | 71.5 | 100 | 2107.1 | 964.6 | 4602.7 |
| | | PI(D30) | 24 | 24 | 100 | 85.8 | 100 | 3239 | 2209.1 | 4749.1 |
| | | PII(D90) | 24 | 24 | 100 | 85.8 | 100 | 4547.2 | 3105.4 | 6658.4 |
| | 10vPP30 | PRE | 12 | 11 | 91.7 | 61.5 | 99.8 | 764.2 | 193.3 | 3022 |
| | | PI(D30) | 24 | 24 | 100 | 85.8 | 100 | 4668.3 | 2811.9 | 7750.4 |
| | | PII(D90) | 21 | 21 | 100 | 83.9 | 100 | 6056.7 | 4211.8 | 8709.6 |
| | 23PPV | PRE | 14 | 12 | 85.7 | 57.2 | 98.2 | 586.5 | 156.8 | 2194.4 |
| | | PI(D30) | 22 | 22 | 100 | 84.6 | 100 | 6960.6 | 4531.6 | 10691.5 |
| | | PII(D90) | 23 | 23 | 100 | 85.2 | 100 | 4886.4 | 2956.8 | 8075.1 |

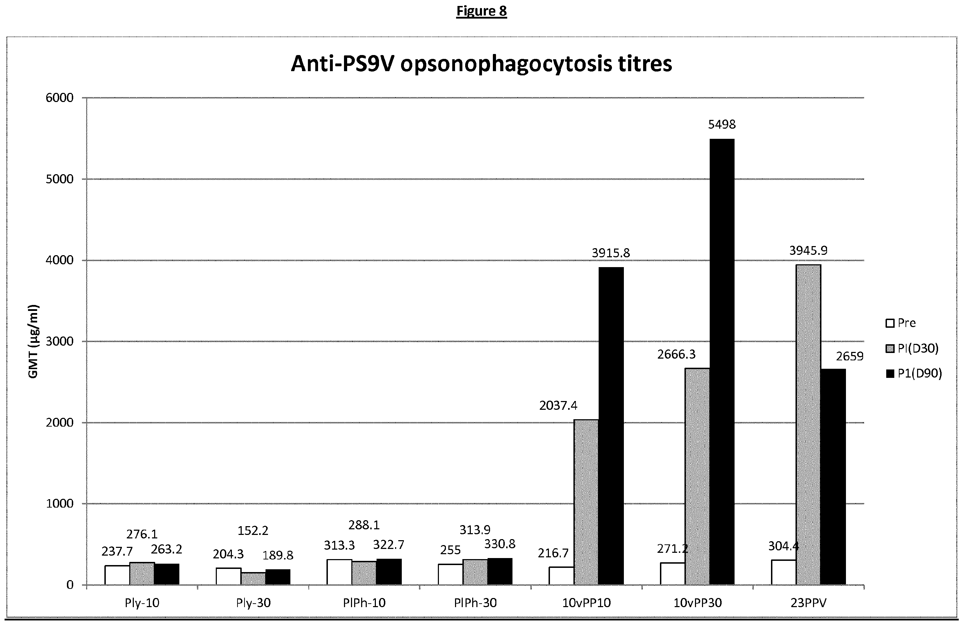

Seropositivity rates and OPA GMTs for serotype 9V antibodies are described in Table 8 below, these data are illustrated in Figure 8:

| **Table 8: Seropositivity rates and GMTs for anti-PS9V antibodies (ATP cohort for immunogenicity)** | | | | | | | | | | |
|---|---|---|---|---|---|---|---|---|---|---|
| | | | | **≥ 8** | | | | **GMT** | | |
| | | | | | | **95% CI** | | | **95% CI** | |
| **Antibody** | **Group** | **Timing** | **N** | **n** | **%** | **LL** | **UL** | **value** | **LL** | **UL** |
| OPSONO-9V | Ply-10 | PRE | 9 | 9 | 100 | 66.4 | 100 | 237.7 | 128.4 | 439.9 |
| | | PI(D30) | 10 | 10 | 100 | 69.2 | 100 | 276.1 | 149.9 | 508.5 |
| | | PII(D90) | 10 | 10 | 100 | 69.2 | 100 | 263.2 | 157.7 | 439.4 |
| | Ply-30 | PRE | 19 | 17 | 89.5 | 66.9 | 98.7 | 204.3 | 87.4 | 477.8 |
| | | PI(D30) | 22 | 18 | 81.8 | 59.7 | 94.8 | 152.2 | 61.8 | 375.2 |
| | | PII(D90) | 21 | 17 | 81 | 58.1 | 94.6 | 189.8 | 70.7 | 510 |
| | PlPh-10 | PRE | 22 | 21 | 95.5 | 77.2 | 99.9 | 313.3 | 163.8 | 599.3 |
| | | PI(D30) | 24 | 23 | 95.8 | 78.9 | 99.9 | 288.1 | 156.1 | 531.5 |
| | | PII(D90) | 22 | 21 | 95.5 | 77.2 | 99.9 | 322.7 | 168.8 | 616.8 |
| | PlPh-30 | PRE | 21 | 21 | 100 | 83.9 | 100 | 255 | 161 | 403.9 |
| | | PI(D30) | 22 | 21 | 95.5 | 77.2 | 99.9 | 313.9 | 164.5 | 598.8 |
| | | PII(D90) | 23 | 22 | 95.7 | 78.1 | 99.9 | 330.8 | 173.8 | 629.6 |
| | 10vPP10 | PRE | 24 | 22 | 91.7 | 73 | 99 | 216.7 | 103.5 | 453.8 |
| | | PI(D30) | 24 | 24 | 100 | 85.8 | 100 | 2037.4 | 1208.5 | 3434.7 |
| | | PII(D90) | 24 | 24 | 100 | 85.8 | 100 | 3915.8 | 2467.4 | 6214.3 |
| | 10vPP30 | PRE | 22 | 22 | 100 | 84.6 | 100 | 271.2 | 177.8 | 413.6 |
| | | PI(D30) | 24 | 24 | 100 | 85.8 | 100 | 2666.3 | 1586.8 | 4480.2 |
| | | PII(D90) | 21 | 21 | 100 | 83.9 | 100 | 5498 | 3334.3 | 9065.8 |
| | 23PPV | PRE | 20 | 19 | 95 | 75.1 | 99.9 | 304.4 | 148.5 | 624 |
| | | PI(D30) | 24 | 24 | 100 | 85.8 | 100 | 3945.9 | 2390.3 | 6514.1 |
| | | PII(D90) | 23 | 23 | 100 | 85.2 | 100 | 2659 | 1463.6 | 4830.9 |

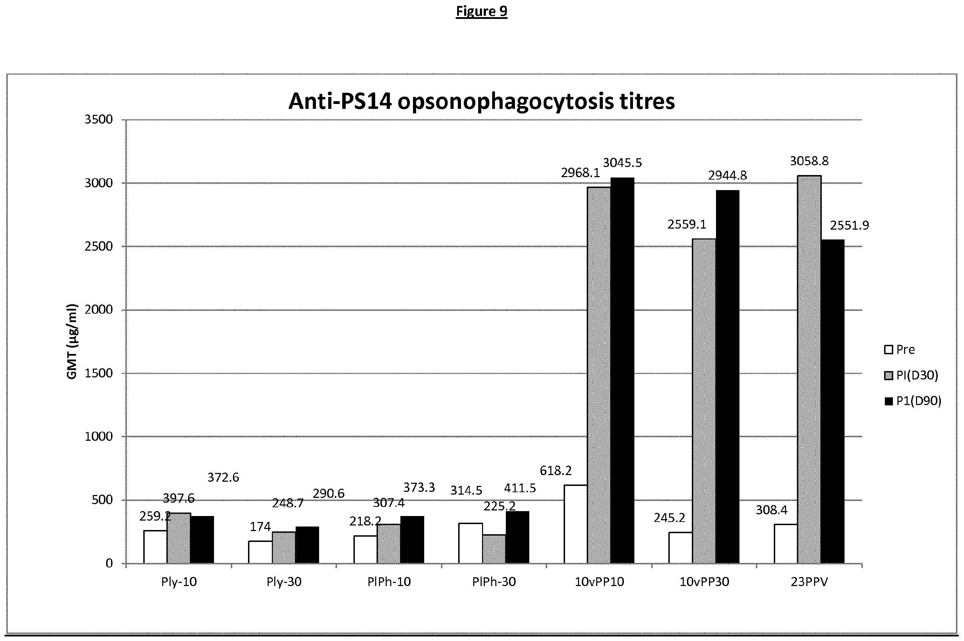

Seropositivity rates and OPA GMTs for serotype 14 antibodies are described in Table 9 below, these data are illustrated in Figure 9:

| **Table 9: Seropositivity rates and GMTs for anti-PS14 antibodies (ATP cohort for immunogenicity)** | | | | | | | | | | |
|---|---|---|---|---|---|---|---|---|---|---|
| | | | | **≥ 8** | | | | **GMT** | | |
| | | | | | | **95% CI** | | | **95% CI** | |
| **Antibody** | **Group** | **Timing** | **N** | **n** | **%** | **LL** | **UL** | **value** | **LL** | **UL** |
| OPSONO-14 | Ply-10 | PRE | 9 | 8 | 88.9 | 51.8 | 99.7 | 259.2 | 67.5 | 994.5 |
| | | PI(D30) | 9 | 8 | 88.9 | 51.8 | 99.7 | 397.6 | 97.5 | 1621.7 |
| | | PII(D90) | 10 | 9 | 90 | 55.5 | 99.7 | 372.6 | 110.6 | 1255.5 |
| | Ply-30 | PRE | 18 | 16 | 88.9 | 65.3 | 98.6 | 174 | 76.4 | 396.3 |
| | | PI(D30) | 22 | 20 | 90.9 | 70.8 | 98.9 | 248.7 | 122.3 | 505.7 |
| | | PII(D90) | 21 | 19 | 90.5 | 69.6 | 98.8 | 290.6 | 134.4 | 628.3 |
| | PlPh-10 | PRE | 20 | 17 | 85 | 62.1 | 96.8 | 218.2 | 85.7 | 555.5 |
| | | PI(D30) | 21 | 19 | 90.5 | 69.6 | 98.8 | 307.4 | 134.8 | 701 |
| | | PII(D90) | 21 | 19 | 90.5 | 69.6 | 98.8 | 373.3 | 167.3 | 832.9 |
| | PlPh-30 | PRE | 14 | 13 | 92.9 | 66.1 | 99.8 | 314.5 | 126.7 | 780.2 |
| | | PI(D30) | 20 | 18 | 90 | 68.3 | 98.8 | 225.2 | 98.1 | 517.2 |
| | | PII(D90) | 21 | 20 | 95.2 | 76.2 | 99.9 | 411.5 | 215.1 | 787.5 |
| | 10vPP10 | PRE | 17 | 17 | 100 | 80.5 | 100 | 618.2 | 364.9 | 1047.4 |
| | | PI(D30) | 24 | 24 | 100 | 85.8 | 100 | 2968.1 | 1713.8 | 5140.3 |
| | | PII(D90) | 24 | 24 | 100 | 85.8 | 100 | 3045.5 | 2007.2 | 4620.9 |
| | 10vPP30 | PRE | 16 | 15 | 93.8 | 69.8 | 99.8 | 245.2 | 116.4 | 516.5 |
| | | PI(D30) | 24 | 24 | 100 | 85.8 | 100 | 2559.1 | 1651 | 3966.7 |
| | | PII(D90) | 21 | 21 | 100 | 83.9 | 100 | 2944.8 | 1854 | 4677.5 |
| | 23PPV | PRE | 20 | 19 | 95 | 75.1 | 99.9 | 308.4 | 164.8 | 576.8 |
| | | PI(D30) | 24 | 24 | 100 | 85.8 | 100 | 3058.8 | 1829.4 | 5114.2 |
| | | PII(D90) | 22 | 22 | 100 | 84.6 | 100 | 2551.9 | 1518.7 | 4287.9 |

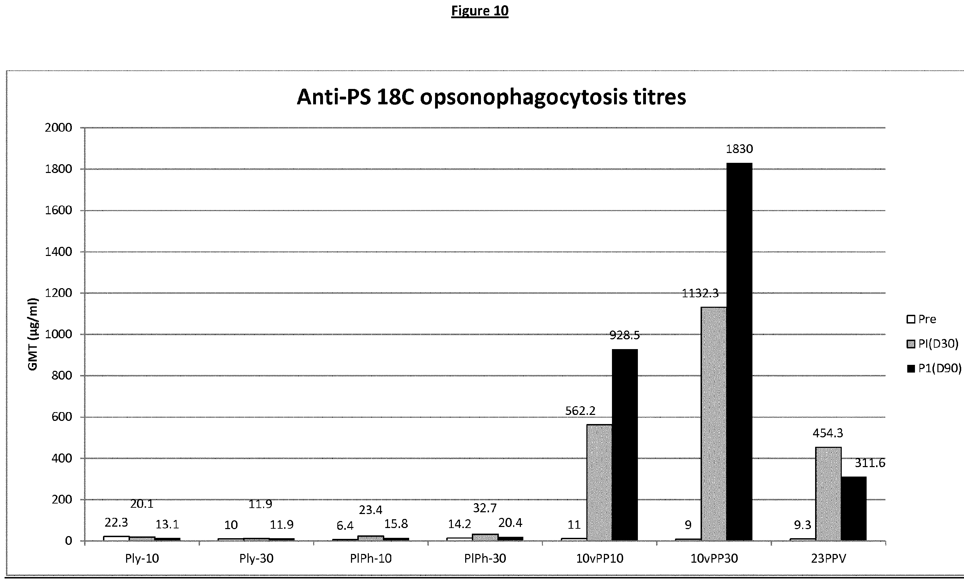

Seropositivity rates and OPA GMTs for serotype 18C antibodies are described in Table 10 below, these data are illustrated in Figure 10:

| **Table 10: Seropositivity rates and GMTs for anti-PS18C antibodies (ATP cohort for immunogenicity)** | | | | | | | | | | |
|---|---|---|---|---|---|---|---|---|---|---|
| | | | | **≥ 8** | | | | **GMT** | | |
| | | | | | | **95% CI** | | | **95% CI** | |
| **Antibody** | **Group** | **Timing** | **N** | **n** | **%** | **LL** | **UL** | **value** | **LL** | **UL** |
| OPSONO-18C | Ply-10 | PRE | 10 | 4 | 40 | 12.2 | 73.8 | 22.3 | 4.5 | 111.2 |
| | | PI(D30) | 9 | 3 | 33.3 | 7.5 | 70.1 | 20.1 | 3.1 | 130.7 |
| | | PII(D90) | 10 | 3 | 30 | 6.7 | 65.2 | 13.1 | 3.2 | 53.8 |
| | Ply-30 | PRE | 18 | 4 | 22.2 | 6.4 | 47.6 | 10 | 3.9 | 25.7 |
| | | PI(D30) | 21 | 7 | 33.3 | 14.6 | 57 | 11.9 | 5.2 | 27.2 |
| | | PII(D90) | 17 | 6 | 35.3 | 14.2 | 61.7 | 11.9 | 4.8 | 29.6 |
| | PlPh-10 | PRE | 15 | 3 | 20 | 4.3 | 48.1 | 6.4 | 3.3 | 12.7 |
| | | PI(D30) | 20 | 9 | 45 | 23.1 | 68.5 | 23.4 | 8.6 | 64 |
| | | PII(D90) | 22 | 8 | 36.4 | 17.2 | 59.3 | 15.8 | 6.3 | 39.9 |
| | PlPh-30 | PRE | 15 | 7 | 46.7 | 21.3 | 73.4 | 14.2 | 5.9 | 34.2 |
| | | PI(D30) | 24 | 14 | 58.3 | 36.6 | 77.9 | 32.7 | 14.2 | 75.4 |
| | | PII(D90) | 20 | 9 | 45 | 23.1 | 68.5 | 20.4 | 7.9 | 52.7 |
| | 10vPP10 | PRE | 18 | 6 | 33.3 | 13.3 | 59 | 11 | 5.2 | 23.3 |
| | | PI(D30) | 24 | 23 | 95.8 | 78.9 | 99.9 | 562.2 | 286 | 1105 |
| | | PII(D90) | 24 | 24 | 100 | 85.8 | 100 | 928.5 | 613.2 | 1405.8 |
| | 10vPP30 | PRE | 20 | 4 | 20 | 5.7 | 43.7 | 9 | 4.1 | 19.6 |
| | | PI(D30) | 24 | 24 | 100 | 85.8 | 100 | 1132.3 | 750.8 | 1707.6 |
| | | PII(D90) | 21 | 21 | 100 | 83.9 | 100 | 1830 | 1220.6 | 2743.7 |
| | 23PPV | PRE | 18 | 4 | 22.2 | 6.4 | 47.6 | 9.3 | 4.1 | 20.8 |
| | | PI(D30) | 24 | 23 | 95.8 | 78.9 | 99.9 | 454.3 | 217.1 | 950.7 |
| | | PII(D90) | 22 | 20 | 90.9 | 70.8 | 98.9 | 311.6 | 134.9 | 719.5 |

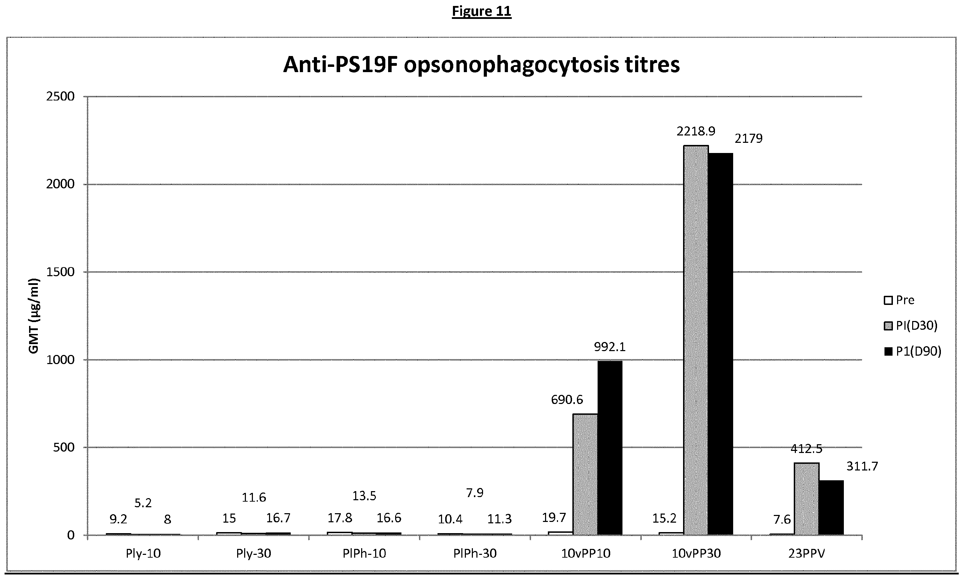

Seropositivity rates and OPA GMTs for serotype 19F antibodies are described in Table 11 below, these data are illustrated in Figure 11:

| **Table 11: Seropositivity rates and GMTs for anti-PS19F antibodies (ATP cohort for immunogenicity)** | | | | | | | | | | |
|---|---|---|---|---|---|---|---|---|---|---|
| | | | | **≥ 8** | | | | **GMT** | | |
| | | | | | | **95% CI** | | | **95% CI** | |
| **Antibody** | **Group** | **Timing** | **N** | **n** | **%** | **LL** | **UL** | **value** | **LL** | **UL** |
| OPSONO-19F | Ply-10 | PRE | 10 | 4 | 40 | 12.2 | 73.8 | 9.2 | 4 | 21.4 |
| | | PI(D30) | 8 | 1 | 12.5 | 0.3 | 52.7 | 5.2 | 2.8 | 9.8 |
| | | PII(D90) | 10 | 5 | 50 | 18.7 | 81.3 | 8 | 4.7 | 13.7 |
| | Ply-30 | PRE | 20 | 10 | 50 | 27.2 | 72.8 | 15 | 7.2 | 31.4 |
| | | PI(D30) | 23 | 10 | 43.5 | 23.2 | 65.5 | 11.6 | 6.1 | 21.9 |
| | | PII(D90) | 21 | 11 | 52.4 | 29.8 | 74.3 | 16.7 | 7.9 | 35.5 |
| | PlPh-10 | PRE | 19 | 13 | 68.4 | 43.4 | 87.4 | 17.8 | 9.8 | 32.3 |
| | | PI(D30) | 23 | 12 | 52.2 | 30.6 | 73.2 | 13.5 | 7 | 25.7 |
| | | PII(D90) | 23 | 14 | 60.9 | 38.5 | 80.3 | 16.6 | 8.4 | 32.8 |
| | PlPh-30 | PRE | 21 | 8 | 38.1 | 18.1 | 61.6 | 10.4 | 5.7 | 19.2 |
| | | PI(D30) | 23 | 8 | 34.8 | 16.4 | 57.3 | 7.9 | 4.9 | 12.6 |
| | | PII(D90) | 24 | 10 | 41.7 | 22.1 | 63.4 | 11.3 | 5.9 | 21.7 |
| | 10vPP10 | PRE | 21 | 10 | 47.6 | 25.7 | 70.2 | 19.7 | 7.8 | 49.8 |
| | | PI(D30) | 21 | 21 | 100 | 83.9 | 100 | 690.6 | 374.4 | 1273.7 |
| | | PII(D90) | 24 | 24 | 100 | 85.8 | 100 | 992.1 | 532.9 | 1847.2 |
| | 10vPP30 | PRE | 20 | 11 | 55 | 31.5 | 76.9 | 15.2 | 7.7 | 30.1 |
| | | PI(D30) | 23 | 23 | 100 | 85.2 | 100 | 2218.9 | 1351.2 | 3643.6 |
| | | PII(D90) | 21 | 21 | 100 | 83.9 | 100 | 2179 | 1404.4 | 3380.9 |
| | 23PPV | PRE | 20 | 7 | 35 | 15.4 | 59.2 | 7.6 | 4.9 | 11.7 |
| | | PI(D30) | 20 | 20 | 100 | 83.2 | 100 | 412.5 | 189.4 | 898.4 |
| | | PII(D90) | 23 | 22 | 95.7 | 78.1 | 99.9 | 311.7 | 141.7 | 685.6 |

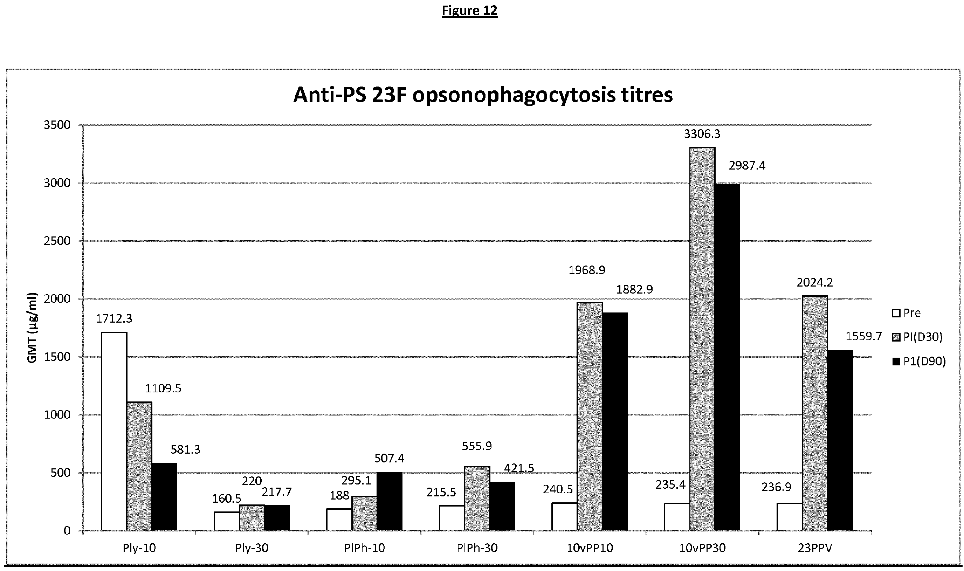

Seropositivity rates and OPA GMTs for serotype 23F antibodies are described in Table 12 below, these data are illustrated in Figure 12:

| **Table 12: Seropositivity rates and GMTs for anti-PS23F antibodies (ATP cohort for immunogenicity)** | | | | | | | | | | |
|---|---|---|---|---|---|---|---|---|---|---|
| | | | | **≥ 8** | | | | **GMT** | | |
| | | | | | | **95% CI** | | | **95% CI** | |
| **Antibody** | **Group** | **Timing** | **N** | **n** | **%** | **LL** | **UL** | **value** | **LL** | **UL** |
| OPSONO-23F | Ply-10 | PRE | 6 | 6 | 100 | 54.1 | 100 | 1712.3 | 705.1 | 4158.6 |
| | | PI(D30) | 9 | 9 | 100 | 66.4 | 100 | 1109.5 | 425.5 | 2893 |
| | | PII(D90) | 10 | 9 | 90 | 55.5 | 99.7 | 581.3 | 126.3 | 2674.8 |
| | Ply-30 | PRE | 16 | 11 | 68.8 | 41.3 | 89 | 160.5 | 40 | 644.8 |
| | | PI(D30) | 19 | 14 | 73.7 | 48.8 | 90.9 | 220 | 66 | 733.8 |
| | | PII(D90) | 18 | 14 | 77.8 | 52.4 | 93.6 | 217.7 | 70.9 | 668.3 |
| | PlPh-10 | PRE | 12 | 8 | 66.7 | 34.9 | 90.1 | 188 | 29.2 | 1211 |
| | | PI(D30) | 21 | 16 | 76.2 | 52.8 | 91.8 | 295.1 | 92.9 | 937.5 |
| | | PII(D90) | 19 | 16 | 84.2 | 60.4 | 96.6 | 507.4 | 170.4 | 1510.5 |
| | PlPh-30 | PRE | 9 | 6 | 66.7 | 29.9 | 92.5 | 215.5 | 17.6 | 2635.2 |
| | | PI(D30) | 21 | 19 | 90.5 | 69.6 | 98.8 | 555.9 | 230.5 | 1340.8 |
| | | PII(D90) | 22 | 18 | 81.8 | 59.7 | 94.8 | 421.5 | 140.9 | 1261 |
| | 10vPP10 | PRE | 19 | 13 | 68.4 | 43.4 | 87.4 | 240.5 | 58.6 | 987.6 |
| | | PI(D30) | 24 | 24 | 100 | 85.8 | 100 | 1968.9 | 1256.5 | 3085.1 |
| | | PII(D90) | 24 | 24 | 100 | 85.8 | 100 | 1882.9 | 1176.2 | 3014.3 |
| | 10vPP30 | PRE | 17 | 12 | 70.6 | 44 | 89.7 | 235.4 | 55.6 | 996.7 |
| | | PI(D30) | 24 | 24 | 100 | 85.8 | 100 | 3306.3 | 2280.7 | 4793.1 |
| | | PII(D90) | 21 | 21 | 100 | 83.9 | 100 | 2987.4 | 1977.1 | 4513.9 |
| | 23PPV | PRE | 15 | 11 | 73.3 | 44.9 | 92.2 | 236.9 | 56.8 | 988.4 |
| | | PI(D30) | 24 | 24 | 100 | 85.8 | 100 | 2024.2 | 1237.8 | 3310.1 |
| | | PII(D90) | 23 | 22 | 95.7 | 78.1 | 99.9 | 1559.7 | 778.7 | 3123.8 |

### Summary of results

All candidate vaccine formulations were safe and well tolerated. No clinically significant changes in the haematology, biochemistry and urine parameters were observed over time.

During the 7-day post-vaccination period after dose 1, maximum 4.2% to 12.5% of doses of the candidate vaccine formulations were followed by grade 3 solicited local and general AEs, respectively. These incidences were within the same ranges as those observed in the 23PPV comparator group (4.2 % of doses). No grade 3 fever (oral temperature > 39.5°C) was reported.

No trend towards higher incidences of grade 3 solicited local and/or general AEs was generally observed in all groups with consecutive dose of candidate vaccine formulations.

Grade 3 unsolicited AEs were reported following 4.5% to 13.3% of doses for the candidate vaccine formulations. Unsolicited AEs considered by the investigator to be causally related to vaccination were reported following 10.4% to 33.3% of doses for the candidate vaccine formulations and were mainly local reactions.

No SAEs were reported in the subjects receiving the candidate vaccine formulations.

The formulations containing the pneumococcal protein dPly with or without PhtD were immunogenic. Increases in the anti-Ply antibody GMCs were observed following each vaccination in the groups vaccinated with dPly containing formulations; increase in anti-PhtD antibody GMCs were observed following each vaccination in groups vaccinated with PhtD containing formulations. The highest anti-Ply and anti-PhtD antibody GMCs were observed for the formulations containing 30µg of dPly and PhtD proteins (i.e in the group PIPh-30 followed by 10vPP30 group).

Free protein formulations (alum adsorbed) do not induce an OPA responses One month post-dose 1 and one month post-dose 2, for each of the vaccine pneumococcal serotypes, at least 95.7% of subjects receiving the formulation combining pneumococcal proteins (dPly and PhtD) and 10Pn-PD-DiT had OPA titres ≥ 8. An increase in OPA GMTs was observed from post-dose 1 to post-dose 2 timepoint for some of the pneumococcal serotypes. For a majority of the tested serotypes (6B, 7F, 9V, 18C, 19F, 23F) the calculated OPA GMT point estimates post dose 1 or post dose 2 were higher in the 10vPP30 groups as compared to the 10vPP10 group.

### Example 3 - Clinical trial to study efficacy of vaccines comprising high and low doses of dPly and PhtD carried out in infants

A phase II, randomized, controlled, observer-blind study to assess the safety, reactogenicity and immunogenicity of two formulations of GlaxoSmithKline (GSK) Biologicals' Streptococcus pneumoniae protein containing vaccine given as a 3-dose primary vaccination course co-administered with DTPa-HBV-IPV/Hib vaccine during the first 6 months (Epoch 1) of life and as a booster dose at 12-15 months of age (Epoch 2).

This clinical trial was carried out using 4 parallel groups;
**1. 10PCV/dPly/PhtD-10-AIPO₄ group** ('10vPP10' in result tables and figures): subjects who received GSK Biologicals' 10PCV/dPly/PhtD-10-AIPO₄ vaccine co-administered with DTPa-HBV-IPV/Hib vaccine.
**2. 10PCV/dPly/PhtD-30-AIPO₄group** ('10vPP30' in result tables and figures): subjects who received GSK Biologicals' 10PCV/dPly/PhtD-30-AIPO₄ vaccine co-administered with DTPa-HBV-IPV/Hib vaccine.
**3. 10PCV group** ('10Pn' in result tables and figures): subjects who received GSK Biologicals' 10PCV vaccine co-administered with DTPa-HBV-IPV/Hib vaccine.
**4. Prev13 group** ('13Pn-DTPa' in result tables and figures): subjects who received Pfizer's commercially available 13-valent pneumococcal conjugate vaccine (Prevenar 13) co-administered with DTPa-HBV-IPV/Hib vaccine.

The vaccines were given to healthy infants according to a 3-dose primary vaccination schedule at 2, 3, 4 months of age (Epoch 1, completed) to be followed by a booster dose at 12-15 months of age (Epoch 2, study ongoing).

### Safety/reactogenicity methods

### Within group assessment (descriptive analysis):

The percentage of subjects reporting each individual solicited local and general AE (adverse event) during the 7-day (Day 0-Day 6) solicited follow-up period were tabulated for each group, after each vaccine dose and overall primary doses, with exact 95% Cl (confidence interval). The percentage of doses followed by each individual solicited local and general AE was tabulated for each group, over the full primary vaccination course, with exact 95% Cl. The same tabulations were performed for grade 3 solicited AEs and for solicited AEs with causal relationship to vaccination. For redness and swelling, grade 2 or 3 AEs were also tabulated. Occurrence of fever was reported per 0.5°C cumulative increments. All the above tabulations for each individual solicited adverse event were also performed for the 4-day follow-up period after each vaccination (Day 0-Day 3).

The proportion of subjects/doses with at least one report of unsolicited AE classified by the Medical Dictionary for Regulatory Activities (MedDRA) and reported up to 30 days after primary vaccination was tabulated with exact 95% Cl for each group. The same tabulation was performed for grade 3 unsolicited AEs and for unsolicited AEs with a relationship to vaccination.

The proportion of AEs that resulted in a medically attended visit was also tabulated.

SAEs (serious adverse events) and withdrawal(s) due to SAE(s) were described in detail.

### Laboratory assays and time points for immunogenicity evaluation

Blood samples were collected at Visit 1 and Visit 4 during the Epoch 001. After blood centrifugation and serum separation, samples were stored at -20°C until collection by the sponsor. The aliquots of serum (approximately 2.5 mL) were sent to GSK Biologicals for the serological tests described in the following paragraphs.
Pneumococcal serotype specific total IgG antibodies (antibodies to 1, 3, 4, 5, 6A, 6B, 7F, 9V, 14, 18C, 19A, 19F and 23F) were each measured by 22F-inhibition ELISA (enzyme-linked immunosorbent assay) as described for example 2. The antibody concentration was determined by logistic log comparison of the ELISA curves with a standard reference serum 89-SF available from the US Food and Drug Administration (FDA) for which concentration of IgG and IgM to the 10 serotypes are known in µg/mL. The cut-off of the assay was 0.05 µg/mL.
Opsonophagocytic activity (OPA) for antibodies against each of the pneumococcal serotypes 1, 3, 4, 5, 6A, 6B, 7F, 9V, 14, 18C, 19A, 19F and 23F was measured by an OPA assay (similar to that described in example 2). The results were presented as the dilution of serum (opsonic titre) able to sustain 50% killing of live pneumococci under the assay conditions. The cut-off of the assay was an opsonic titre of 8.
Anti-Ply and anti-PhtD antibodies were quantified using individual ELISA. Concentration of specific anti-Ply and anti-PhtD antibodies was determined using a standard reference serum. The cut-off of the assay was 100 EL.U/mL for PD, 12 EL.U/mL for dPly and 17 EL.U/mL for PhtD.

### Safety and reactogenicity results (primary outcome of the study)

The safety analysis was performed on the Total vaccinated cohort. Solicited adverse events During the 7-day post-vaccination period:
Redness was the most frequently reported solicited local AE in each group (after 43.6%, 44.7%, 40.1% and 41.1% of doses in the 10PCV/dPly/PhtD -10-AIPO₄, 10PCV/dPly/PhtD-30-AIPO₄, 10PCV and Prev13 groups, respectively). No more than 3.3% of doses were followed by grade 3 solicited local AEs of given category, in each group.
Irritability was the most frequently reported solicited general AE in each group (after 55.5%, 55.5%, 55.0% and 56.6% of doses in the 10PCV/dPly/PhtD -10-AIPO₄, 10PCV/dPly/PhtD - 30-AIPO₄, 10PCV and Prev13 groups, respectively). No more than 4.8% of doses were followed by grade 3 general solicited AEs of given category, in each group and most of them were considered by the investigator to be causally related to vaccination.
No increase in the incidence of solicited local and general AEs was observed with consecutive doses of either 10PCV/dPly/PhtD -10-AIPO₄ or 10PCV/dPly/PhtD -30-AIPO₄, vaccines, during the 3-dose primary vaccination course.

### Unsolicited adverse events

During the 31-day post-primary vaccination period:
16.9%, 21.1%, 20.1% and 19.7% of doses in the 10PCV/dPly/PhtD -10-AIPO₄, 10PCV/dPly/PhtD -30-AIPO₄, 10PCV and Prev13 group, respectively were followed by at least one unsolicited AE.
Grade 3 unsolicited AEs were reported after 0.0%, 0.2%, 0.5% and 0.2% of doses in the 10PCV/dPly/PhtD -10-AIPO₄, 10PCV/dPly/PhtD -30-AIPO₄, 10PCV and Prev13 group, respectively. Of these, one grade 3 AE (hypotonic hyporesponsive episode) reported in the 10PCV group was considered by the investigator to be causally related to vaccination.
Unsolicited AEs considered by the investigator to be causally related to vaccination were reported for 0.2%, 0.5%, 0.9% and 0.0% of doses in the 10PCV/dPly/PhtD -10-AIPO₄, 10PCV/dPly/PhtD -30-AIPO₄, 10PCV and Prev13 group, respectively.

### Serious adverse events:

During the primary epoch (from dose 1 up to visit 4), at least one SAE was reported for 20 subjects: 5 out of 146 (3.4%) vaccinated subjects in the 10PCV/dPly/PhtD -10-AIPO₄ group, 2 out of 142 (1.4%) vaccinated subjects in the 10PCV/dPly/PhtD -30-AIPO>₄group, 10 out of 145 (6.9%) vaccinated subjects in the 10PCV group and 3 out of 142 (2.1%) vaccinated subjects in the Prev13 group. Of these, one SAE (hypotonic hyporesponsive episode) reported as the grade 3 AE during the 31-day follow-up period in the 10Pn group was considered by the investigator to be causally related to vaccination. All SAEs resolved without sequelae, except one SAE (psychomotor retardation) in the 10Pn group which was not resolved at the time of visit 4. No fatal SAEs were reported.

### Withdrawals due to adverse events /serious adverse events:

One subject in the 10PCV group (subject number 607) was withdrawn from the study due to a SAE (hypotonic-hyporesponsive episode) which was considered by the investigator to be causally related to vaccination. This event resolved without sequelae after 6 days.

### Immunogenicity

The results suggested that the 10PCV/dPly/PhtD -10-AIPO4 and 10PCV/dPly/PhtD -30-AIPO4 vaccines induced immune responses to all vaccine antigens (i.e. dPly and PhtD pneumococcal proteins, pneumococcal serotype-specific capsular polysaccharides and NTHi protein D).

### Example 4: Formulation of multivalent vaccines comprising a PE-PilA fusion protein

3 vaccines were designed:
**10V:** A ten valent (10V) vaccine containing the following ten S. *pneumoniae* capsular saccharide conjugates: capsular saccharide from serotype 1 conjugated to protein D (1-PD), capsular saccharide from serotype 4 conjugated to protein D (4-PD), capsular saccharide from serotype 5 conjugated to protein D (5-PD), capsular saccharide from serotype 6B conjugated to protein D (6B-PD), capsular saccharide from serotype 7F conjugated to protein D (7F-PD), capsular saccharide from serotype 9V conjugated to protein D (9V-PD), capsular saccharide from serotype 14 conjugated to protein D (14-PD), capsular saccharide from serotype 23F conjugated to protein D (23F-PD), capsular saccharide from serotype 18C conjugated to tetanus toxoid (18C-TT) and capsular saccharide from serotype 19F conjugated to Diphtheria Toxin (19F-DT).
**12V:** A twelve valent (12V) vaccine containing the same ten S. *pneumoniae* capsular saccharide conjugates as 10V with an additional two S. *pneumoniae* saccharide conjugates, 19A conjugated to CRM197 (19ACRM) and 6A conjugated to CRM197 (6ACRM).
**12V+ proteins (12V+prot):** A vaccine containing the same twelve S. *pneumoniae* capsular saccharide conjugates as 12V with the addition of PhtD, dPly and PE-PilA fusion protein.

### Preparation of the antigens

The antigens were prepared as described in example 1. The PEPilA antigen was prepared as described in WO2012/139225.

### Formulation of the vaccines

The 10V vaccine contains S. *pneumoniae* capsular saccharide serotype 1, 4, 5, 6B, 7F, 9V, 14, 18C, 19F and 23F conjugates adsorbed onto aluminium phosphate together at a human dose of 1, 3, 1, 1, 1, 1, 1, 3, 3, 1 µg (the saccharides were individually adsorbed to aluminium phosphate, they were then mixed together and the level of aluminium phosphate adjusted to 500µg).

The 12V vaccine was made in the same way as the 10V vaccine with additional serotypes 19A and 6A conjugates at doses of 2µg adsorbed onto aluminium phosphate added.

The 12V + proteins vaccine was made in the same way as the 12V vaccine except proteins PhtD, dPly and PE-PilA were added. The 12 conjugates and the proteins were mixed together using the dosages described for 12V above and the proteins at dosages of 30µg each (note that this refers to 30µg of PE-PilA, not 30µg of PE and 30µg of PilA).

### Example 5: Comparison of the immunogenicity of 10V. 12V and 12V+proteins vaccines in mice

### Description of the anti-pneumococcal polysaccharide PS (polysaccharide) ELISA

Microplates were coated for 2 hours at 37°C with capsular polysaccharide (CPS) (100 µl per well of 2.5 µg/ml of PS1 and PS3, 5 µg/ml of PS4, 5, 6A, 6B, 7F, 9V or 14; 10 µg/ml of PS19A and 23F or 40 µg/ml of PS18C and PS19F in PBS). The plates were washed three times with NaCl 150 mM (0.9%) -Polysorbate 20 0.05%. Sera was diluted (1/2 for 6A and 6B and 1/10 for the other serotypes) in PBS-Polysorbate20 0.05% containing CPS (1 mg CPS/ml of non diluted serum except or 6A and 6B which was at 2.5mg/ml) V/V and incubated for 1 hour at 37°C in order to neutralize antibodies directed to the CPS. The sera from mice immunised as described in the section entitled 'immunogenicity of three vaccine formulations in mice' or a reference serum (an internal reference calibrated with Chrompure mouse IgG) was added to the microwells and serially diluted 100 µl (two-fold dilution step) in PBS-Polysorbate20 0.05%. The plates were incubated under agitation for 30 minutes at room temperature. The plates were washed as above and anti-mouse IgG antibodies conjugated to peroxidase (100 µl per well) was added, the plates were incubated for 30 minutes at room temperature with shaking. After washing, the substrate (4 mg of OPDA (ortho phenylen-diamine) in 10 ml of citrate 0.1M pH 4.5-4.6 and 5 µl of H₂O₂) was added to each well (100 µl) and the plates incubated for 15 minutes in the dark. The reaction was stopped by addition of HCI 1N (50µl). The absorbance was read at 490nm or 620nm for the reference using a spectrophotometer. The color developed is directly proportional to the amount of antibody present in the serum.

### Description of the ELISA to measure PD, PE and PilA antibodies

Plates were coated overnight at 4°C with 100 µl per well of 2 µg/ml of PD (1 mg/ml), 2 µg/ml of PE (1500 µg/ml), 2 µg/ml of PilA (3660 µg/ml) in carbonate buffer pH 9.6. The plates were washed four times with NaCl 0.9% Polysorbate 20 0.05%. For PE and PilA ELISA, the plates were saturated for 30 min at room temperature (with shaking) with PBS-BSA1 %. After washing, sera from mice immunised as described in the section entitled 'immunogenicity of three vaccine formulations in mice' or a reference serum (an internal reference calibrated with Chrompure mouse IgG) was added to microwells and serially diluted 100 µl (two-fold dilution step) in PBS Polysorbate 20 0.05% (for the PD assay) and PBS Polysorbate 20 0.05% BSA 0.1% (for the PE and PilA assay). The plates were then incubated for 30 min at room temperature with agitation. After washing, the plates were incubated with an anti-mouse IgG antibody conjugated to peroxidase (100 µl per well) at room temperature for 30 minutes with shaking. The plates were then washed as above and the substrate conjugate (4 mg of OPDA (ortho phenylen-diamine) in 10 ml of citrate 0.1M pH 4.5-4.6 and 5 µl of H₂O₂) as added to each well (100 µl) for 15 min in darkness. The reaction was stopped by addition of HCl 1N 50 µl and the absorbance was read at 490 nm (620 nm for the reference).

### Description of the ELISAto measure PhtD and dPly antibodies

Plates were coated for 2 hours at 37°C with 100 µl per well of 1 µg/ml of PhtD (1021 µg/ml) or 4µg/ml of Ply (367µg/ml). The plates were then washed three times with NaCl 0.09% Polysorbate 0.05%. After washing, sera from mice immunised as described in the section entitled 'immunogenicity of three vaccine formulations in mice' or a reference (an internal reference calibrated with Chrompure mouse IgG) (was added to microwells and serially diluted 100 µl (two-fold dilution step) in PBS Polysorbate 20 0.05%. The plates were incubated for 30 min at room temperature with agitation. After washing, the plates were incubated with an anti-mouse IgG antibody conjugated to peroxidase (100 µl per well) at room temperature for 30 minutes with shaking. The plates were washed as above and the substrate conjugate (4 mg of OPDA in 10 ml of citrate 0.1M ph 4.5 and 5 µl of H₂O₂) was added to each well (100 µl) for 15 min in darkness. The reaction was stopped by addition of HCI 1N 50 µl and the absorbance was read at 490 nm (620 nm for the reference filter).

### Description of opsonophaqocytosis assay (OPA)

Serum samples were heated for 45 min at 56 °C to inactivate any remaining endogenous complement. Twenty-five microlitres aliquots of each 1:2 diluted serum sample were two-fold serially diluted in 25 µl OPA buffer (HBSS (Hank's Balanced Salt Solution)-14.4% inactivated FCS (Foetal Calf Serum)) per well of a 96-well round bottom microtitre plate. Subsequently, 25 µl of a mixture of activated HL-60 cells (1 × 107 cells/ml), freshly thawed pneumococcal working seed and freshly thawed baby rabbit complement in an e.g. 4/2/1 ratio (v/v/v) (except for serotypes 1, 6B and 6A for which the ratio was 4/2/2) were added to the diluted sera to yield a final volume of 50 µl. The assay plate was incubated for 2 h at 37 °C with orbital shaking (210 rpm) to promote the phagocytic process. The reaction was stopped by laying the microplate on ice for at least 1 min, the plate is kept on ice until use. A 20 µl aliquot of each well of the plate was then transferred into the corresponding well of a 96-well flat bottom microplate and 50 µl of Todd-Hewitt Broth-0.9% agar was added to each well. After overnight incubation at 37 °C and 5% CO₂, pneumococcal colonies which appeared in the agar were counted using an automated image analysis system (KS 400, Zeiss, Oberkochen, Germany). Eight wells without serum sample were used as bacterial controls to determine the number of pneumococci per well. The mean number of CFU of the control wells was determined and used for the calculation of the killing activity for each serum sample. The OPA titre for the serum samples was determined by the reciprocal dilution of serum able to facilitate 50% killing of the pneumococci. The opsonophagocytic titre was calculated by using a 4-parameter curve fit analysis.

### Immunogenicity of three vaccine formulations in mice.

2 groups of 27 female Balb/c mice were immunized by intramuscular (IM) injections on days 0, 14 and 28 with 1/10 human dose of different formulation including Proteins alone (PhtD, dPly and PEPilA - results not presented), Prevnar 13 (_{™} a commercially available Streptococcal vaccine - results not presented) 10V, 12V (DSP2A017) and 12V+ proteins (DSP2A012) GMP lots. Mice received in a different leg (mimicking co-administration at different sites in infants in clinical trials) 1/10^{th} human dose of *Infanrix Hexa* (_{™} a vaccine comprising diphtheria toxoid, tetanus toxoid, pertussis toxoid, filamentous haemmagglutinin, pertactin, hepatitis B surface antigen, inactivated polio virus types 1, 2 and 3 and *Haemophilus influenzae* b saccharide (PRP)).

Anti-IgG levels and opsonophagocytosis titers were determined respectively in individual and pooled sera collected at day 42.

The potential of the 12V +proteins vaccine to induce IgG antibody titers and opsonic activity was evaluated and compared to that of the 12V and 10V vaccines.

Sera from mice injected with the different formulations were tested in ELISA (as described above) against the polysaccharide serotypes and proteins and in OPA against the 12 polysaccharide serotypes.

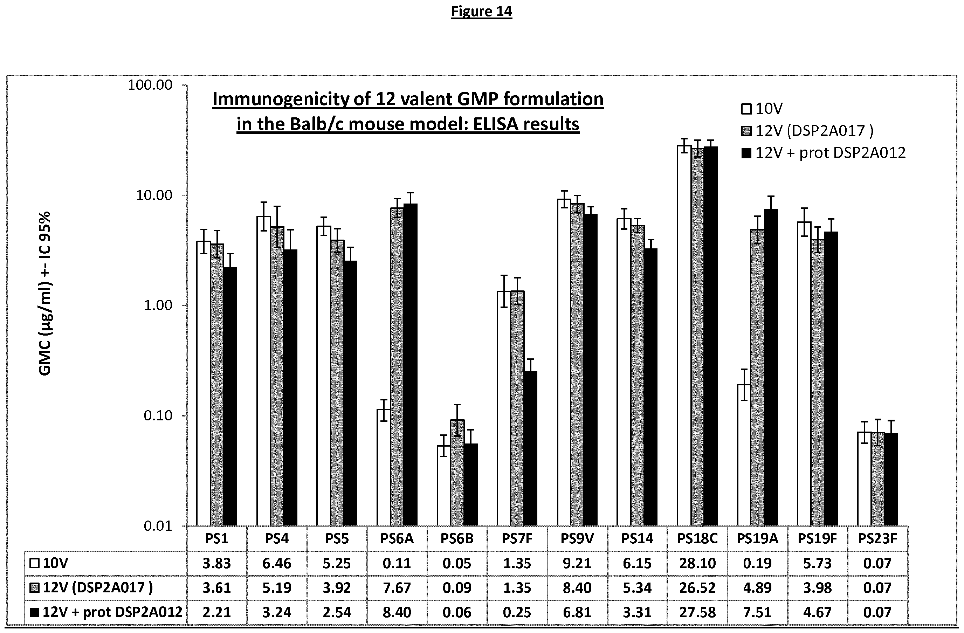

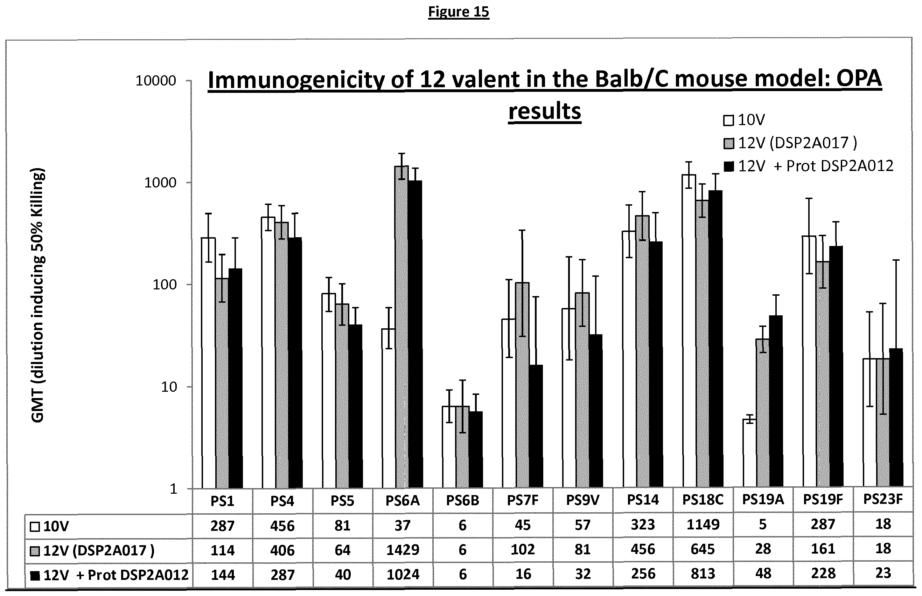

The 12V + proteins vaccine induced similar response to the 12V for most serotypes (Figures 14 and 15).

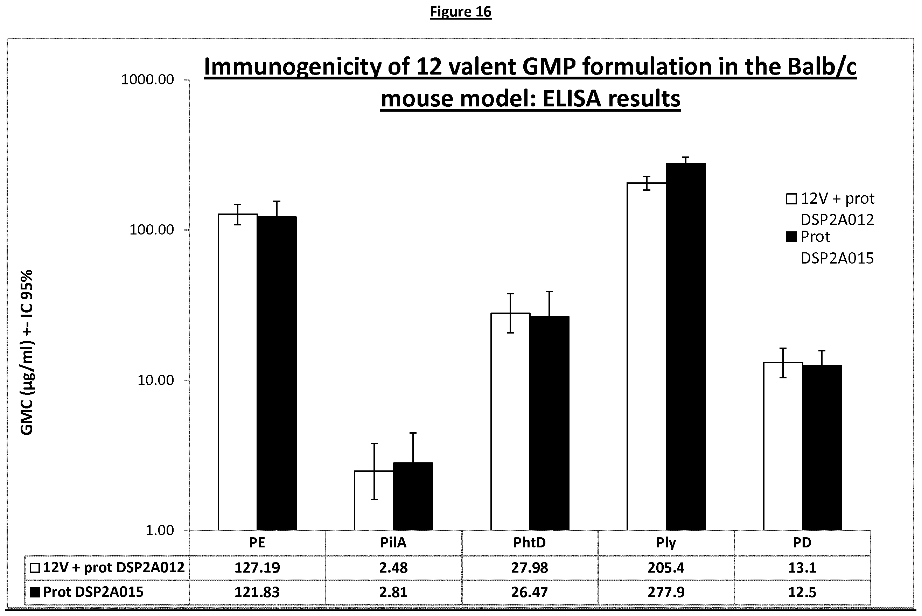

The results in Figure 16 demonstrated that there was no statistical difference between the PE, PiIA, PhtD, Ply and PD antibody responses measured for the 12V+ formulation and a formulation that did not comprises the S. *pneumoniae* saccharide conjugates.

### Example 6: Comparison of the immunogenicity of 10V, 12V and 12V+protein vaccines in guinea pigs

### Description of ELISA anti-pneumococcal polysacharide PS

Microplates were coated for 2 hours at 37°C with 100 µl per well of 2.5 µg/ml of PS1, 5 µg/ml of PS4, 5, 6A, 6B, 7F, 9V or 14; 10 µg/ml of PS19A and 23F, 40 µg/ml of PS18C and PS19F or Affinipure Goat anti-guinea pig IgG(2.4mg/ml) diluted to 2µg/ml for the reference wells in PBS. The plates were washed three times with NaCl 150 mM (0.9%)-Polysorbate20 0.05%. Pooled serum from each group was diluted (1/2 for PS 6A and 6B and 1/10 for all the other serotypes) in PBS-Polysorbate20 0.05% containing CPS (1 mg CPS/ml of non diluted serum except for 6A and 6B at 2.5mg/ml) V/V and incubated for 1 hour at 37°C in order to neutralize antibodies directed to CPS. The serum from guinea pigs immunised as described in the section entitled 'immunogenicity of three vaccine formulations in guinea pigs' was added to the microwells and serially diluted 100 µl (two-fold dilution step) in PBS-Polysorbate20 0.05% or a reference serum (Chrompure guinea pig IgG (11mg/ml) diluted to 0.25µg/ml in PBS-polysorbate20 0.05%) was added . The plates were incubated under agitation for 30 minutes at room temperature. The plates were washed as above and anti-guinea pig IgG antibodies conjugated to peroxidase (100 µl per well) were added and the plates incubated for 30 minutes at room temperature with shaking. After washing, the substrate (4 mg of OPDA in 10 ml of citrate 0.1M pH 4.5-4.6 and 5 µl of H₂O₂) was added to each well (100 µl) and incubated for 15 minutes in the dark. The reaction was stopped by addition of HCI 1N. Absorbance was read at 490nm (620nm for the reference) using a spectrophotometer. The color developed is directly proportional to the amount of antibody present in the serum.

### Description of the ELISA to measure anti PD, PE, and PilA, antibodies

Plates were coated for 2 hours at 37°C with 100 µl per well of 2 µg/ml of PD (1 mg/ml), 2 µg/ml of PE (1500 µg/ml), or 2 µg/ml of PilA (3660 µg/ml) in carbonate buffer pH 9.6 in PBS or Affinipure Goat anti-guinea pig IgG (2.4mg/ml) diluted to 2µg/m for the reference wells in PBS. The plates were washed 4 times with NaCl 0.9% Polysorbate 20 0.05%. For PE and PilA ELISAs (this step was not carried out for the PD and Ply ELISAs), the plates were saturated 30 min at room temperature with PBS-BSA1 %. After washing, sera from guinea pigs immunised as described in the section entitled 'immunogenicity of three formulations in guinea pigs' or a reference serum sample (an internal reference calibrated with Chrompure guinea pig IgG) was added to microwells and serially diluted 100 µl (two-fold dilution step) in PBS Polysorbate 20 0.05% (for the PD ELISA) and PBS Polysorbate 20 0.05% BSA 0.1% for the PE and PilA ELISAs. The plates were incubated for 30 min at room temperature. After washing, the plates were incubated with an anti-guinea pig IgG antibody conjugated to peroxydase (100 µl per well) at room temperature for 30 minutes with shaking. Plates were washed as above and the substrate conjugate (4 mg of OPDA in 10 ml of citrate 0.1M pH 4.5-4.6 and 5 µl of H₂O₂) was added to each well (100 µl) for 15 min in darkness. The reaction was stopped by addition of HCl 1N 50 µl and the absorbance is read at 490 nm (620 nm for the reference filter).

### Description of the ELISAto measure PhtD and dPly antibodies

Plates were coated for 2 hours at 37°C with 100 µl per well of 1 µg/ml of PhtD (1021 µg/ml) or 2µg/ml Ply (376µg/ml) in PBS. The plates were then washed 4 times with NaCl 0.9% Polysorbate 20 0.05%. After washing, sera from guinea pigs immunised as described in the section entitled 'immunogenicity of three vaccine formulations in guinea pigs' or a reference (an internal reference calibrated with Chrompure guinea pig IgG) was added to microwells and serially diluted 100 µl (two-fold dilution step) in PBS Polysorbate 20 0.05%. The plates were incubated for 30 min at room temperature with agitation. After washing, the plates were incubated with an anti-guinea pig IgG antibody conjugated to peroxydase (100 µl per well) at room temperature for 30 minutes with shaking. Plates were washed as above and the substrate conjugate (4 mg of OPDA in 10 ml of citrate 0.1M pH 4.5-4.6 and 5 µl of H₂O₂) was added to each well (100 µl) for 15 min in darkness. The reaction was stopped by addition of HCI 1N 50 µl and the absorbance was read at 490 nm (620 nm for the reference filter).

### Opsonophagocytosis assay

Serum samples were heated for 45 min at 56 °C to inactivate any remaining endogenous complement. Twenty-five microlitres aliquots of each 1:2 diluted serum sample was two-fold serially diluted in 25 µl OPA buffer (HBSS (Hank's Balanced Salt Solution)-14.4% inactivated FCS (foetal calf serum)) per well of a 96-well round bottom microtitre plate. Subsequently, 25 µl of a mixture of activated HL-60 cells (1 × 107 cells/ml), freshly thawed pneumococcal working seed and freshly thawed baby rabbit complement in an e.g. 4/2/1 ratio (v/v/v) (except for serotypes 1,6B and 6A for which the ratio was 4/2/2) was added to the diluted sera to yield a final volume of 50 µl. The assay plate was incubated for 2 h at 37 °C with orbital shaking (210 rpm) to promote the phagocytic process. The reaction was stopped by laying the microplate on ice for at least 1 min (the plate should be kept on ice until further use. A 20 µl aliquot of each well of the plate was then transferred into the corresponding well of a 96-well flat bottom microplate and 50 µl of Todd-Hewitt Broth-0.9% agar was added to each well. After overnight incubation at 37 °C and 5% CO2, pneumococcal colonies appearing in the agar were counted using an automated image analysis system (KS 400, Zeiss, Oberkochen, Germany). Eight wells without serum sample were used as bacterial controls to determine the number of pneumococci per well. The mean number of CFU of the control wells was determined and used for the calculation of the killing activity for each serum sample. The OPA titre for the serum samples was determined by the reciprocal dilution of serum able to facilitate 50% killing of the pneumococci. The opsonophagocytic titre was calculated by using a 4-parameter curve fit analysis.

### Immunogenicity of three formulations in guinea pigs

2 groups of 17 guinea pigs were immunized by intramuscular (IM) injections on days 0, 14 and 28 with ¼ human dose of different formulation including Proteins alone (PhtD, dPly and PEPiIA - results not presented), Prevnar 13 (_{™} a commercially available Streptococcal vaccine - results not presented) 10V, 12V (DSP2A017) and 12V+ proteins (DSP2A012) GMP lots. Guinea pigs received in a different leg (mimicking co-administration at different sites in infants in clinical trials) ¼ of human dose of *Infanrix Hexa* ((_{™} a vaccine comprising diphtheria toxoid, tetanus toxoid, pertussis toxoid, filamentous haemmagglutinin, pertactin, hepatitis B surface antigen, inactivated polio virus types 1, 2 and 3 and *Haemophilus influenzae* b saccharide (PRP).

Anti-IgG levels and opsonophagocytosis titers were determined respectively in individual and pooled sera collected at days 42.

The IgG antibody titers and opsonic activity was evaluated and compared between the 12V+proteins, 12V and 10V vaccines.

Sera from guinea pigs injected with the different formulations were tested in ELISA against the polysaccharide serotypes and proteins and in OPA against the 12 serotypes in the formulation.

The 12V + proteins induced similar responses to the 12V formulation.

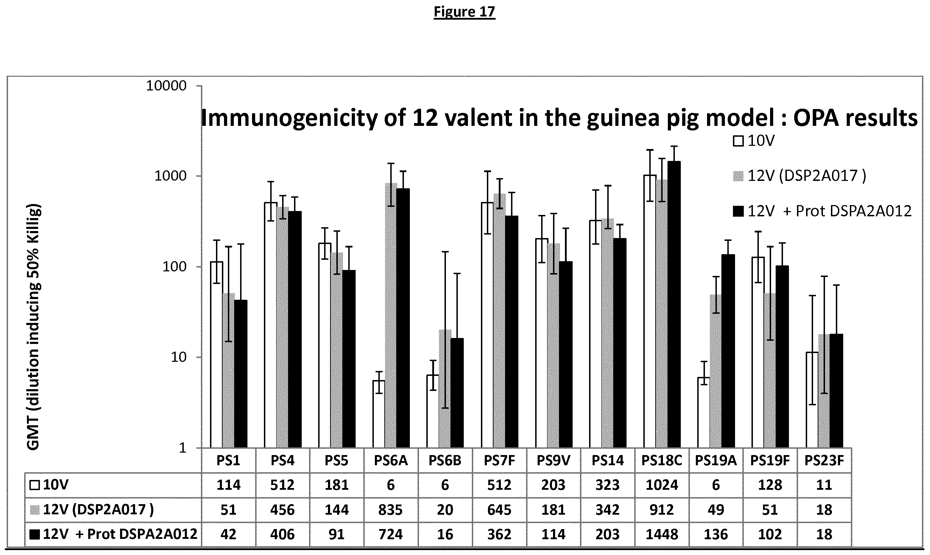

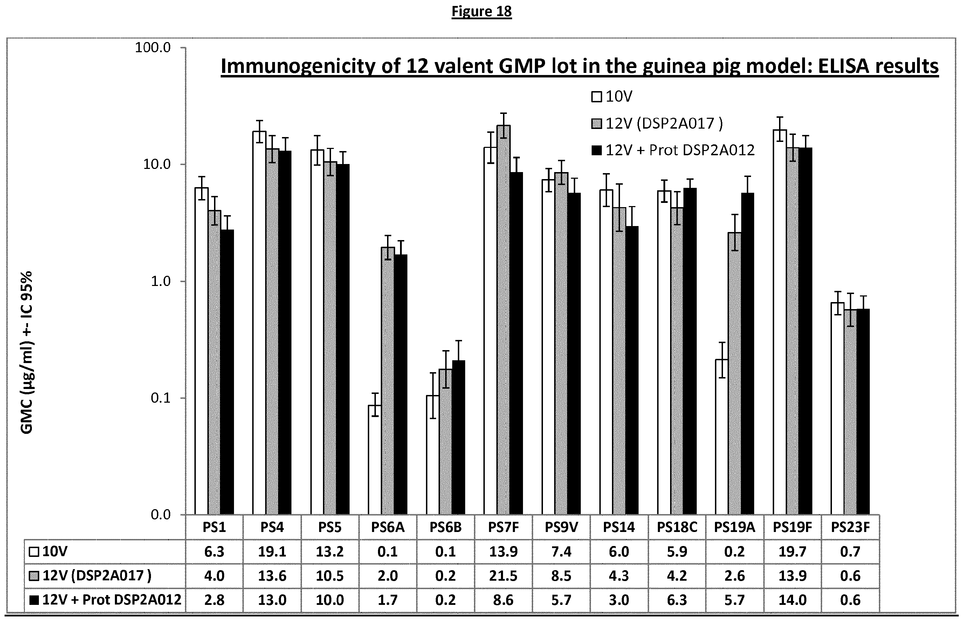

The results in Figures 17 and 18 demonstrated that there is no negative impact on the immunogenicity of the 12 valent conjugates when they are combined with PEPilA.

The results obtained with the 12V+proteins GMP formulation demonstrated immunogenicity of the 10V polysaccharides as well as the proteins and support the clinical evaluation of this formulation.

## Claims

1. An immunogenic composition comprising 26µg-45µg (for example 26µg-40µg, 28µg-35µg or around 30µg) of PhtD per human dose and 26µg-45µg (for example 26µg-40µg, 28µg-35µg or around 30µg) of detoxified pneumolysin (dPly) per human dose, for use in the treatment or prevention of disease caused by *Streptococcus pneumoniae* infection wherein the immunogenic composition further comprises *Streptococcus pneumoniae* capsular saccharide conjugates comprising a conjugated serotype 4 saccharide, a conjugated serotype 6B saccharide, a conjugated serotype 9V saccharide, a conjugated serotype 14 saccharide, a conjugated serotype 18C saccharide, a conjugated serotype 19F saccharide and a conjugated serotype 23F saccharide.

2. The immunogenic composition for the use according to claim 1 wherein the PhtD (poly histidine triad family D) comprises an amino acid sequence at least 85%, 90%, 95%, 98%, 99% or 100% identical to the sequence at amino acids 21-838 of Sequence ID No.4 of WO00/37105.

3. The immunogenic composition for the use according to any preceding claim wherein the immunogenic composition has a dose volume of 0.5ml.

4. The immunogenic composition for the use according to any preceding claim which comprises at least one further unconjugated or conjugated *Streptococcus pneumoniae* protein selected from the group consisting of Poly Histidine Triad family (PhtX), Choline Binding Protein Family (CbpX), CbpX truncates, LytX (autolytic enzyme) family, LytX truncates, CbpX truncate-LytX truncate chimeric proteins, PcpA (pneumococcal choline binding protein A), PspA (Pneumococcal surface protein A), PsaA (pneumococcal surface adhesion A), Sp128 *(Streptococcus pneumoniae* 128), Sp101 *(Streptococcus pneumoniae* 101), Sp130 *(Streptococcus pneumoniae* 130), SP125 *(Streptococcus pneumoniae* 125) and SP133 *(Streptococcus pneumoniae* 133).

5. The immunogenic composition for the use according to any preceding claim wherein the immunogenic composition does not comprise an oil in water adjuvant.

6. The immunogenic composition for the use according to any one of the preceding claims wherein the *Streptococcus pneumoniae* capsular saccharide conjugates further comprise a conjugated serotype 1 saccharide, a conjugated serotype 5 saccharide and a conjugated serotype 7F saccharide.

7. The immunogenic composition for the use according to any one of the preceding claims wherein the *Streptococcus pneumoniae* capsular saccharide conjugates further comprise a conjugated serotype 6A saccharide and a conjugated serotype 19A saccharide.

8. The immunogenic composition for the use according to any one of the preceding claims wherein the *Streptococcus pneumoniae* capsular saccharide conjugates further comprise a conjugated serotype 33F saccharide, and a conjugated serotype 22F saccharide.

9. The immunogenic composition for the use according to any one of the preceding claims wherein the *Streptococcus pneumoniae* capsular saccharide conjugates are conjugated to a carrier protein independently selected from the group consisting of tetanus toxoid (TT), fragment C of TT, diphtheria toxoid, CRM197 (cross reacting material 197), detoxified pneumolysin, protein D (from *H.influenzae),* PhtD (Poly Histidine Triad D), PhtDE (fusion protein between Poly Histidine Triad D and Poly Histidine Triad E) and N19.

10. The immunogenic composition for the use according to any one of claims 1-9 wherein the *Streptococcus pneumoniae* capsular saccharide conjugates comprise a serotype 1 saccharide conjugated to protein D, a serotype 4 saccharide conjugated to protein D, a serotype 5 saccharide conjugated to protein D, a serotype 6B saccharide conjugated to protein D, a serotype 7F saccharide conjugated to protein D, a serotype 9V saccharide conjugated to protein D, a serotype 14 saccharide conjugated to protein D, a serotype 23F saccharide conjugated to protein D, a serotype 18C saccharide conjugated to tetanus toxoid and a serotype 19F saccharide conjugated to diphtheria toxoid.

11. The immunogenic composition for the use according to any preceding claim which further comprises an adjuvant.

12. The immunogenic composition for use according to any one of the preceding claims wherein the use comprises administration of the immunogenic composition to an adult human host, an elderly human host, or to an infant or toddler human host and wherein the diseases comprise at least one disease selected from the group consisting of pneumonia, invasive pneumococcal disease (IPD), exacerbations of chronic obstructive pulmonary disease (COPD), otitis media, recurrent otitis media, meningitis, bacteraemia, and conjunctivitis.

13. A vaccine comprising an immunogenic composition comprising 26µg-45µg (for example 26µg-40µg, 28µg-35µg or around 30 µg) of PhtD per human dose and 26µg-45µg (for example 26µg-40µg, 28µg-35µg or around 30 µg) of detoxified pneumolysin (dPly) per human dose, for use in the treatment or prevention of disease caused by *Streptococcus pneumoniae* infection wherein the immunogenic composition further comprises *Streptococcus pneumoniae* capsular saccharide conjugates comprising a conjugated serotype 4 saccharide, a conjugated serotype 6B saccharide, a conjugated serotype 9V saccharide, a conjugated serotype 14 saccharide, a conjugated serotype 18C saccharide, a conjugated serotype 19F saccharide and a conjugated serotype 23F saccharide and a pharmaceutically acceptable excipient.

## Patentansprüche

1. Immunogene Zusammensetzung, umfassend 26 µg-45 µg (zum Beispiel 26 µg-40 µg, 28 µg-35 µg oder ungefähr 30 µg) PhtD pro humaner Dosis und 26 µg-45 µg (zum Beispiel 26 µg-40 µg, 28 µg-35 µg oder ungefähr 30 µg) entgiftetes Pneumolysin (dPly) pro humaner Dosis, zur Verwendung bei der Behandlung oder Prävention von durch *Streptococcus* pneumoniae-Infektion verursachter Erkrankung, wobei die immunogene Zusammensetzung weiterhin *Streptococcus* pneumoniae-Kapselsaccharidkonjugate umfasst, umfassend ein konjugiertes Serotyp 4-Saccharid, ein konjugiertes Serotyp 6B-Saccharid, ein konjugiertes Serotyp 9V-Saccharid, ein konjugiertes Serotyp 14-Saccharid, ein konjugiertes Serotyp 18C-Saccharid, ein konjugiertes Serotyp 19F-Saccharid und ein konjugiertes Serotyp 23F-Saccharid.

2. Immunogene Zusammensetzung zur Verwendung gemäß Anspruch 1, wobei das PhtD (Polyhistidin-Triaden-Familie D) eine Aminosäuresequenz umfasst, die zu wenigstens 85%, 90%, 95%, 98%, 99% oder 100% identisch zu der Sequenz an Aminosäuren 21-838 von Sequenz-ID-Nr. 4 aus WO 00/37105 ist.

3. Immunogene Zusammensetzung zur Verwendung gemäß irgendeinem vorangegangenen Anspruch, wobei die immunogene Zusammensetzung ein Dosisvolumen von 0,5 ml hat.

4. Immunogene Zusammensetzung zur Verwendung gemäß irgendeinem vorangegangenen Anspruch, welche wenigstens ein weiteres unkonjugiertes oder konjugiertes *Streptococcus pneumoniae-Protein* umfasst, ausgewählt aus der Gruppe, bestehend aus Polyhistidin-Triaden-Familie (PhtX), Cholin-Bindungsprotein-Familie (CbpX), CbpX-Trunkierungen, LytX- (autolytisches Enzym) Familie, LytX-Trunkierungen, chimären CbpX-Trunkierung-LytX-Trunkierung-Proteinen, PcpA (Pneumokokken-Cholin-Bindungsprotein A), PspA (Pneumokokken-Oberflächenprotein A), PsaA (Pneumokokken-Oberflächenadhäsion A), Sp128 *(Streptococcus pneumoniae* 128), Sp101 (*Streptococcus pneumoniae* 101), Sp130 (*Streptococcus pneumoniae* 130), SP125 (*Streptococcus pneumoniae* 125) und SP133 (*Streptococcus pneumoniae* 133) .

5. Immunogene Zusammensetzung zur Verwendung gemäß irgendeinem vorangegangenen Anspruch, wobei die immunogene Zusammensetzung kein Öl-in-Wasser-Adjuvans umfasst.

6. Immunogene Zusammensetzung zur Verwendung gemäß irgendeinem der vorangegangenen Ansprüche, wobei die *Streptococcus* pneumoniae-Kapselsaccharidkonjugate weiterhin ein konjugiertes Serotyp 1-Saccharid, ein konjugiertes Serotyp 5-Saccharid und ein konjugiertes Serotyp 7F-Saccharid umfassen.

7. Immunogene Zusammensetzung zur Verwendung gemäß irgendeinem der vorangegangenen Ansprüche, wobei die *Streptococcus* pneumoniae-Kapselsaccharidkonjugate weiterhin ein konjugiertes Serotyp 6A-Saccharid und ein konjugiertes Serotyp 19A-Saccharid umfassen.

8. Immunogene Zusammensetzung zur Verwendung gemäß irgendeinem der vorangegangenen Ansprüche, wobei die *Streptococcus* pneumoniae-Kapselsaccharidkonjugate weiterhin ein konjugiertes Serotyp 33F-Saccharid und ein konjugiertes Serotyp 22F-Saccharid umfassen.

9. Immunogene Zusammensetzung zur Verwendung gemäß irgendeinem der vorangegangenen Ansprüche, wobei die *Streptococcus* pneumoniae-Kapselsaccharidkonjugate an ein Trägerprotein konjugiert sind, das unabhängig aus der Gruppe ausgewählt ist, bestehend aus Tetanustoxoid (TT), Fragment C von TT, Diphtherietoxoid, CRM197 (kreuzreagierendes Material 197), entgiftetem Pneumolysin, Protein D (von *H. influenzae*)*,* PhtD (Polyhistidin-Triade D), PhtDE (Fusionsprotein zwischen Polyhistidin-Triade D und Polyhistidin-Triade E) und N19.

10. Immunogene Zusammensetzung zur Verwendung gemäß irgendeinem der Ansprüche 1-9, wobei die *Streptococcus* pneumoniae-Kapselsaccharidkonjugate ein Serotyp 1-Saccharid, konjugiert an Protein D, ein Serotyp 4-Saccharid, konjugiert an Protein D, ein Serotyp 5-Saccharid, konjugiert an Protein D, ein Serotyp 6B-Saccharid, konjugiert an Protein D, ein Serotyp 7F-Saccharid, konjugiert an Protein D, ein Serotyp 9V-Saccharid, konjugiert an Protein D, ein Serotyp 14-Saccharid, konjugiert an Protein D, ein Serotyp 23F-Saccharid, konjugiert an Protein D, ein Serotyp 18C-Saccharid, konjugiert an Tetanustoxoid, und ein Serotyp 19F-Saccharid, konjugiert an Diphtherietoxoid, umfassen.

11. Immunogene Zusammensetzung zur Verwendung gemäß irgendeinem vorangegangenen Anspruch, welche weiterhin ein Adjuvans umfasst.

12. Immunogene Zusammensetzung zur Verwendung gemäß irgendeinem der vorangegangenen Ansprüche, wobei die Verwendung das Verabreichen der immunogenen Zusammensetzung an einen erwachsenen menschlichen Wirt, einen älteren menschlichen Wirt oder einen menschlichen Säuglings- oder Kleinkindwirt umfasst und wobei die Erkrankungen wenigstens eine Erkrankung umfassen, die aus der Gruppe ausgewählt ist, bestehend aus Pneumonie, invasiver Pneumokokkenerkrankung (invasive pneumococcal disease; IPD), Exazerbationen von chronisch-obstruktiver Lungenerkrankung (COPD), Otitis media, wiederkehrender Otitis media, Meningitis, Bakteriämie und Konjunktivitis.

13. Impfstoff, welcher eine immunogene Zusammensetzung umfasst, umfassend 26 µg-45 µg (zum Beispiel 26 µg-40 µg, 28 µg-35 µg oder ungefähr 30 µg) PhtD pro humaner Dosis und 26 µg-45 µg (zum Beispiel 26 µg-40 µg, 28 µg-35 µg oder ungefähr 30 µg) entgiftetes Pneumolysin (dPly) pro humaner Dosis, zur Verwendung bei der Behandlung oder Prävention von durch *Streptococcus* pneumoniae-Infektion verursachter Erkrankung, wobei die immunogene Zusammensetzung weiterhin *Streptococcus* pneumoniae-Kapselsaccharidkonjugate umfasst, umfassend ein konjugiertes Serotyp 4-Saccharid, ein konjugiertes Serotyp 6B-Saccharid, ein konjugiertes Serotyp 9V-Saccharid, ein konjugiertes Serotyp 14-Saccharid, ein konjugiertes Serotyp 18C-Saccharid, ein konjugiertes Serotyp 19F-Saccharid und ein konjugiertes Serotyp 23F-Saccharid und einen pharmazeutisch akzeptablen Hilfsstoff.

## Revendications

1. Composition immunogène comprenant 26 µg à 45 µg (par exemple 26 µg à 40 µg, 28 µg à 35 µg ou environ 30 µg) de PhtD par dose humaine et 26 µg à 45 µg (par exemple 26 µg à 40 µg, 28 µg à 35 µg ou environ 30 µg) de pneumolysine détoxifiée (dPly) par dose humaine, pour une utilisation dans le traitement ou la prévention d'une maladie provoquée par une infection par *Streptococcus pneumoniae,* dans laquelle la composition immunogène comprend en outre des conjugués de saccharide capsulaire de *Streptococcus pneumoniae* comprenant un saccharide de sérotype 4 conjugué, un saccharide de sérotype 6B conjugué, un saccharide de sérotype 9V conjugué, un saccharide de sérotype 14 conjugué, un saccharide de sérotype 18C, un saccharide de sérotype 19F et un saccharide de sérotype 23F.

2. Composition immunogène pour l'utilisation selon la revendication 1, dans laquelle la PhtD (famille de triade de poly-histidine D) comprend une séquence d'acides aminés identique au moins à 85 %, 90 %, 95 %, 98 %, 99 % ou 100 % à la séquence de SEQ ID n° 4 de WO00/37105 au niveau des acides aminés 21 à 838.

3. Composition immunogène pour l'utilisation selon l'une quelconque des revendications précédentes, dans laquelle la composition immunogène a un volume de dose de 0,5 ml.

4. Composition immunogène pour l'utilisation selon l'une quelconque des revendications précédentes , qui comprend au moins une protéine de *Streptococcus pneumoniae* supplémentaire non conjuguée ou conjuguée sélectionnée dans le groupe constitué par la famille de Triade de Poly-Histidine (PhtX), la Famille Protéique de Liaison à la Choline (CbpX), des tronçons de CbpX, la famille LytX (enzyme autolytique), des tronçons de LytX, des protéines chimériques de tronçons de CbpX-tronçons de LytX, PcpA (protéine A de liaison à la choline pneumococcique), PspA (protéine A superficielle Pneumococcique), PsaA (adhérence superficielle pneumococcique A), Sp128 (*Streptococcus pneumoniae* 128), Sp101 *(Streptococcus pneumoniae* 101), Sp130 (*Streptococcus pneumoniae* 130), SP125 *(Streptococcus pneumoniae* 125) et SP133 *(Streptococcus pneumoniae* 133).

5. Composition immunogène pour l'utilisation selon l'une quelconque des revendications précédentes, dans laquelle la composition immunogène ne comprend pas d'huile dans un adjuvant aqueux.

6. Composition immunogène pour l'utilisation selon l'une quelconque des revendications précédentes, dans laquelle les conjugués de saccharide capsulaire de *Streptococcus pneumoniae* comprennent en outre un saccharide de sérotype 1 conjugué, un saccharide de sérotype 5 conjugué et un saccharide de sérotype 7F conjugué.

7. Composition immunogène pour l'utilisation selon l'une quelconque des revendications précédentes, dans laquelle les conjugués de saccharide capsulaire de *Streptococcus pneumoniae* comprennent en outre un saccharide de sérotype 6A conjugué et un saccharide de sérotype 19A conjugué.

8. Composition immunogène pour l'utilisation selon l'une quelconque des revendications précédentes, dans laquelle les conjugués de saccharide capsulaire de *Streptococcus pneumoniae* comprennent en outre un saccharide de sérotype 33F conjugué et un saccharide de sérotype 22F conjugué.

9. Composition immunogène pour l'utilisation selon l'une quelconque des revendications précédentes, dans lequel les conjugués de saccharide capsulaire de *Streptococcus pneumoniae* sont conjugués à une protéine porteuse indépendamment sélectionnée dans le groupe constitué par l'anatoxine tétanique (TT), un fragment C de TT, l'anatoxine diphtérique, la CRM197 (matière de réaction croisée 197), la pneumolysine détoxifiée, la protéine D (provenant de *H.influenzae*)*,* la PhtD (Triade de Poly-Histidine D), la PhtDE (protéine de fusion entre la Triade de Poly-Histidine D et la Triade de Poly-Histidine E) et N19.

10. Composition immunogène pour l'utilisation selon l'une quelconque des revendications 1 à 9, dans laquelle les conjugués de saccharide capsulaire de *Streptococcus pneumoniae* comprennent un saccharide de 1 sérotype conjugué à la protéine D, un saccharide de sérotype 4 conjugué à la protéine D, un saccharide de sérotype 5 conjugué à la protéine D, un saccharide de sérotype 6B conjugué à la protéine D, un saccharide de sérotype 7F conjugué à la protéine D, un saccharide de sérotype 9V conjugué à la protéine D, un saccharide de sérotype 14 conjugué à la protéine D, un saccharide de sérotype 23F conjugué à la protéine D, un saccharide de sérotype 18C conjugué à l'anatoxine tétanique et un saccharide de sérotype 19F conjugué à l'anatoxine diphtérique.

11. Composition immunogène pour l'utilisation selon l'une quelconque des revendications précédentes, qui comprend en outre un adjuvant.

12. Composition immunogène pour l'utilisation selon l'une quelconque des revendications précédentes, dans laquelle l'utilisation comprend l'administration de la composition immunogène à un hôte humain adulte, un hôte humain âgé, ou à un enfant en bas âge ou un hôte d'humain nourrisson, et dans laquelle les maladies comprennent au moins une maladie sélectionnée dans le groupe constitué par la pneumonie, la maladie pneumococcique invasive (IPD), des exacerbations de maladie pulmonaire obstructive chronique (COPD), l'otite moyenne, l'otite moyenne récurrente, la méningite, la bactériémie et la conjonctivite.

13. Vaccin comprenant une composition immunogène comprenant 26 µg à 45 µg (par exemple 26 µg à 40 µg, 28 µg à 35 µg ou environ 30 µg) de PhtD par dose humaine et 26 µg à 45 µg (par exemple 26 µg à 40 µg, 28 µg à 35 µg ou environ 30 µg) de pneumolysine détoxifiée (dPly) par dose humaine, pour une utilisation dans le traitement ou la prévention d'une maladie provoquée par une infection par *Streptococcus pneumoniae,* dans lequel la composition immunogène comprend en outre des conjugués de saccharide capsulaire de *Streptococcus pneumoniae* comprenant un saccharide de sérotype 4 conjugué, un saccharide de sérotype 6B conjugué, un saccharide de sérotype 9V conjugué, un saccharide de sérotype 14 conjugué, un saccharide de sérotype 18C conjugué, un saccharide de sérotype 19F conjugué et un saccharide de sérotype 23F conjugué et un excipient acceptable d'un point de vue pharmaceutique.
